# EUROPEAN PATENT APPLICATION

(11) **EP 4 714 970 A1**
(43) Date of publication of application: **25.03.2026**
(21) Application number: 24778254.3
(22) Date of filing: 29.03.2024
(51) Int. Cl.: C07K 16/28, A61K 39/00, A61P 35/00

(54) **BISPECIFIC ANTIBODY, PHARMACEUTICAL COMPOSITION, AND USE**

(30) Priority: 29.03.2023 CN 202310327266
(71) Applicant: Akeso Biopharma Co., Ltd., Zhongshan, Guangdong 528437 (CN)
(72) Inventor: LI, Baiyong, Zhongshan, Guangdong 528437 (CN); XIA, Yu, Zhongshan, Guangdong 528437 (CN)
(74) Representative: Santarelli
(86) International application number: PCT/CN2024/084904
(87) International publication number: WO 2024/199468

(57) **Abstract**

A bispecific antibody, a pharmaceutical composition, and a use. Specifically, the bispecific antibody comprises a first protein functional region and a second protein functional region, wherein: the first protein functional region targets LAG3, and the second protein functional region targets a target other than LAG3.

## Description

### CROSS-REFERENCE TO RELATED APPLICATIONS

The present application is based on and claims priority to Chinese Patent Application No. CN202310327266.6 filed on Mar. 29, 2023, which is incorporated herein by reference in its entirety.

### TECHNICAL FIELD

The present disclosure pertains to the field of biomedicine and relates to a bispecific antibody, a pharmaceutical composition comprising the bispecific antibody, and use.

### BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor therapeutic effect, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

Lymphocyte-activation gene 3 (LAG3), namely CD223, is a type I transmembrane protein composed of 498 amino acids and is a member of the immunoglobulin superfamily (IgSF). LAG3 is mainly expressed in activated CD4⁺ T cells and CD8⁺ T cells. Additionally, in cells such as natural killer (NK) cells, B cells, regulatory T cells (Tregs), and plasmacytoid dendritic cells (pDCs), LAG3 is also expressed. (Ruffo Elisa,Wu Richard C,Bruno Tullia C et al. Lymphocyte-activation gene 3 (LAG3): The next immune checkpoint receptor.[J]. Semin Immunol, 2019, 42: 101305).

The LAG3 molecule gene is located on human chromosome 12 (12p13.3), adjacent to the CD4 molecule gene, and both have the same exons and introns. The LAG3 molecule and the CD4 molecule have a high degree of structural similarity, although the amino acid sequence homology between the two is only about 20%. Major histocompatibility complex class II (MHC-II) molecules, liver sinusoidal endothelial cell lectin (LSECtin) molecules, and galectin-3 molecules are related ligands for the LAG3 molecule. The MHC-II molecules are the main ligands for LAG3. The affinity (Kd: 60 nmol·L⁻¹) of LAG3 molecules for the MHC-II molecules is 100 times that of CD4 molecules, indicating that the LAG3 molecules can effectively compete with the CD4 molecules for binding to the MHC-II molecules and inhibit T cell activation.

In the tumor microenvironment, the expression of the immunosuppressive molecule LAG3 can be detected 24 hours after T cell activation, which then leads to T cell dysfunction or apoptosis. The LAG3 molecule, through its D1 domain (which contains one proline-rich loop structure), forms a dimer molecule to specifically bind to the MHC-II molecule in the first signaling axis of CD4⁺ T cell activation, "CD3-TCR-MHC-II", so that in one aspect, a signal transduction pathway for T cell activation is blocked, and in another aspect, an intracellular segment of the LAG3 molecule (KIEELE motif) generates an immunosuppressive signal to down-regulate the activity of CD4⁺ T cells. The LAG3 molecule can promote the differentiation of Treg cells, participate in downstream signaling of signal transducer and activator of transcription 5, and thus enhance the inhibitory effect of Treg cells. This is one of the mechanisms by which tumors escape from killing by the immune system (Andrews Lawrence P, Marciscano Ariel E, Drake Charles G, et al., LAG3 (CD223) as a cancer immunotherapy target. [J]. Immunol Rev, 2017, 276: 80-96). Multiple studies have shown that LAG3 is overexpressed in tumor-infiltrating CD8⁺ T cells of various malignant tumors. For example, in ovarian cancer, tumor-infiltrating New York esophageal squamous cell carcinoma 1 (NY-ESO-1) antigen-specific CD8⁺ T cells express high levels of PD-1 and LAG3, and have a reduced ability to produce IFN-y and TNF-α, thereby leading to lymphocyte inactivation. Galectin-3 and LSECtin interact primarily with LAG3 to regulate the activation and function of CD8⁺ T cells. In addition, melanoma antigen-specific T cells isolated from patients with metastatic melanoma exhibit a significant up-regulation in the expression of LAG3 and other immune checkpoint molecules CTLA-4 and TIM-3. (Liu Hao, Li Xinying, Luo Longlong, et al., Research advances in biological function of lymphocyte activation gene-3 (LAG-3) molecule and clinical application of antibody drugs targeting LAG-3 [J]. Chinese Journal of Pharmacology and Toxicology, 2019, 33(01): 70-78).

Currently, several LAG3 antibody drugs have entered the clinical research stages, among which Bristol Myers Squibb's Relatlimab has progressed the fastest, with 10 clinical studies underway. The vast majority of these studies involve the combination therapy of Relatlimab with nivolumab for the treatment of tumors such as hematological malignancies, melanoma, glioblastoma, renal cell carcinoma, non-small cell lung cancer, and the like.

Ecto-5'-nucleotidase, namely CD73 protein, is a multifunctional glycoprotein encoded by an NT5E gene and having a protein molecular weight of 70 KD. It is anchored on a cell membrane by glyocsyl phosphatidy linositol (GPI) (Zimmermann H. Biochem J. 1992; 285:345-365).

CD73 is widely distributed on the surface of human tissue cells, and it has been found in research that CD73 is highly expressed in various solid tumors, specifically in cancer cells, dendritic cells, regulatory T cells (Tregs), natural killer cells (NK cells), myeloid-derived suppressor cells (MDSCs), tumor-associated macrophages (TAMs) and the like in a tumor micro environment. An important feature of the tumor micro environment is hypoxia. Hypoxia induces the up-regulation of molecules such as hypoxia-inducible factor-1 (HIF-1), thereby leading to the widespread expression of CD73 in the tumor micro environment (Synnestvedt K, et al. J Clin Invest. 2002; 110:993-1002). Analysis of clinical tumor samples has shown that high expression of CD73 is a potential biomarker and is closely related to poor prognosis of various types of tumors, including breast cancer, lung cancer, ovarian cancer, kidney cancer, gastric cancer, head and neck cancer, and the like.

CD73 has both hydrolase activity and non-hydrolase activity. The enzyme and non-enzyme functions of CD73 simultaneously work in the related process in tumors, and mutually promote and maintain the progression of tumors. More and more studies have found that CD73 is a key regulatory molecule for tumor cell proliferation, metastasis, and invasion *in vitro* and tumor angiogenesis and tumor immune escape mechanism *in vivo,* wherein an important immunosuppression mechanism is mediated by CD73-adenosine metabolic signaling pathway. CD39 at the upstream of CD73 can catalyze ATP to generate adenosine monophosphate (AMP), the generated AMP is converted into adenosine by CD73, and adenosine binds to a downstream adenosine receptor (A2AR). A2AR inhibits a series of signaling pathways related to immune activation, such as LCK, MAPK, PKC, and inhibits the immune killing effect of T cells by activating protein kinase A (PKA) and Csk kinase, thereby playing an immunosuppression role to enable tumor to achieve immune escape (Antonioli L, et al. Nat Rev Cancer. 2013; 13:842-857). Preclinical animal model studies have shown that CD73 expressed in immune cells and non-immune cells can promote the immune escape, progression, and metastasis of tumors, wherein the inhibition of cytotoxic T cell (CTL) and NK cell functions by Treg cell-related CD73-adenosine signals is the most significant.

For the treatment of solid tumors, one important aspect of overcoming drug resistance and improving the therapeutic effect is to relieve the inhibition effect of the tumor micro environment (TME) on immune effector cells. TME is a very complex system composed of various cells, intercellular matrix, enzymes, cytokines, metabolites, etc. It has the characteristics of significant low hydrogen, low pH, and high pressure and is thus very different from normal tissues. Hypoxia or ATP enrichment caused by chemoradiotherapy for killing tumor cells promotes the cascade reaction of CD39-CD73 adenosine signals, which is beneficial to the proliferation and function of various cancer-promoting cells but not to cancer-inhibiting cells (Regateiro, F. S., Cobbold, S. P. & Waldmann, H. Clin. Exp. Immunol. 2013; 171:1-7).

The use of antibodies targeting CD73 or gene knock-out of CD73 in animal models can effectively block the growth and metastasis of tumors. Recently, the use of CD73 monoclonal antibody, small interfering RNA technology, specific inhibitor APCP, and the like has achieved remarkable therapeutic effect in anti-tumor treatment of animal experiments, providing a new way for anti-tumor treatment. Evidence from *in vivo* studies has shown that targeted blockade of CD73 will be an effective treatment means for tumor patients.

The relation between CD73 overexpression and tumor subtype, prognosis, and response in patients has shown that CD73 can be an important marker for future tumor treatment and detection of individuals. Therefore, the study of the CD73 target is indispensable.

Currently, there is a need to develop a novel anti-LAG3 antibody drug and a bispecific antibody drug that targets both CD73 and LAG3.

### SUMMARY

Through intensive studies and creative efforts, the inventors have obtained an anti-LAG3-anti-CD73 bispecific antibody. The inventors have surprisingly found that the anti-LAG3-anti-CD73 bispecific antibody of the present disclosure (also referred to as the antibody or the antibody of the present disclosure for short) has good anti-tumor activity.

The present disclosure is detailed below:
One aspect of the present disclosure relates to a bispecific antibody comprising a first protein functional region and a second protein functional region, wherein:
the first protein functional region targets LAG3, and
the second protein functional region targets a target other than LAG3 (e.g., CD73 or PD-1),
wherein the first protein functional region is an anti-LAG3 antibody or an antigen-binding fragment thereof, and the anti-LAG3 antibody comprises a heavy chain variable region and a light chain variable region;
the heavy chain variable region comprises HCDR1, HCDR2, and HCDR3, and the light chain variable region comprises LCDR1, LCDR2, and LCDR3, wherein
HCDR1 has the amino acid sequence set forth in SEQ ID NO: 9,
HCDR2 has the amino acid sequence set forth in SEQ ID NO: 10, and
HCDR3 has the amino acid sequence set forth in SEQ ID NO: 11;
LCDR1 has the amino acid sequence set forth in SEQ ID NO: 12 or SEQ ID NO: 17,
LCDR2 has the amino acid sequence set forth in SEQ ID NO: 13 or SEQ ID NO: 15, and
LCDR3 has the amino acid sequence set forth in SEQ ID NO: 14 or SEQ ID NO: 16.

In some embodiments of the present disclosure, provided is the bispecific antibody, wherein
LCDR1 has the amino acid sequence set forth in SEQ ID NO: 12,
LCDR2 has the amino acid sequence set forth in SEQ ID NO: 13, and
LCDR3 has the amino acid sequence set forth in SEQ ID NO: 14;
   or
LCDR1 has the amino acid sequence set forth in SEQ ID NO: 12,
LCDR2 has the amino acid sequence set forth in SEQ ID NO: 15, and
LCDR3 has the amino acid sequence set forth in SEQ ID NO: 16;
   or
LCDR1 has the amino acid sequence set forth in SEQ ID NO: 17,
LCDR2 has the amino acid sequence set forth in SEQ ID NO: 15, and
LCDR3 has the amino acid sequence set forth in SEQ ID NO: 14;
   or
LCDR1 has the amino acid sequence set forth in SEQ ID NO: 12,
LCDR2 has the amino acid sequence set forth in SEQ ID NO: 13, and
LCDR3 has the amino acid sequence set forth in SEQ ID NO: 16;
   or
LCDR1 has the amino acid sequence set forth in SEQ ID NO: 12,
LCDR2 has the amino acid sequence set forth in SEQ ID NO: 15, and
LCDR3 has the amino acid sequence set forth in SEQ ID NO: 14;
   or
LCDR1 has the amino acid sequence set forth in SEQ ID NO: 17,
LCDR2 has the amino acid sequence set forth in SEQ ID NO: 13, and
LCDR3 has the amino acid sequence set forth in SEQ ID NO: 14;
   or
LCDR1 has the amino acid sequence set forth in SEQ ID NO: 17,
LCDR2 has the amino acid sequence set forth in SEQ ID NO: 13, and
LCDR3 has the amino acid sequence set forth in SEQ ID NO: 16;
   or
LCDR1 has the amino acid sequence set forth in SEQ ID NO: 17,
LCDR2 has the amino acid sequence set forth in SEQ ID NO: 15, and
LCDR3 has the amino acid sequence set forth in SEQ ID NO: 16.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10, and
HCDR3 has the amino acid sequence set forth in SEQ ID NO: 11;
LCDR1 has the amino acid sequence set forth in SEQ ID NO: 12,
LCDR2 has the amino acid sequence set forth in SEQ ID NO: 13, and
LCDR3 has the amino acid sequence set forth in SEQ ID NO: 14.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 12,
LCDR2 has the amino acid sequence set forth in SEQ ID NO: 15, and
LCDR3 has the amino acid sequence set forth in SEQ ID NO: 16.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 17,
LCDR2 has the amino acid sequence set forth in SEQ ID NO: 15, and
LCDR3 has the amino acid sequence set forth in SEQ ID NO: 14.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 12,
LCDR2 has the amino acid sequence set forth in SEQ ID NO: 13, and
LCDR3 has the amino acid sequence set forth in SEQ ID NO: 16.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 12,
LCDR2 has the amino acid sequence set forth in SEQ ID NO: 15, and
LCDR3 has the amino acid sequence set forth in SEQ ID NO: 14.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 17,
LCDR2 has the amino acid sequence set forth in SEQ ID NO: 13, and
LCDR3 has the amino acid sequence set forth in SEQ ID NO: 14.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 17,
LCDR2 has the amino acid sequence set forth in SEQ ID NO: 13, and
LCDR3 has the amino acid sequence set forth in SEQ ID NO: 16.

In some embodiments of the present disclosure, provided is the bispecific antibody, wherein
HCDR1 has the amino acid sequence set forth in SEQ ID NO: 9,
HCDR2 has the amino acid sequence set forth in SEQ ID NO: 10,
HCDR3 has the amino acid sequence set forth in SEQ ID NO: 11;
LCDR1 has the amino acid sequence set forth in SEQ ID NO: 17,
LCDR2 has the amino acid sequence set forth in SEQ ID NO: 15, and
LCDR3 has the amino acid sequence set forth in SEQ ID NO: 16.

In some embodiments of the present disclosure, provided is the bispecific antibody, wherein
the heavy chain variable region of the anti-LAG3 antibody is selected from amino acid sequences set forth in SEQ ID NO: 2 and SEQ ID NO: 44, and the light chain variable region of the anti-LAG3 antibody is selected from amino acid sequences set forth in SEQ ID NO: 4, SEQ ID NO: 6, SEQ ID NO: 8, and SEQ ID NO: 45;
preferably,
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 4;
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 6;
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 8;
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 45;
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 4;
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 6;
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 8; or
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 45.

In some embodiments of the present disclosure, provided is the bispecific antibody, wherein the anti-LAG3 antibody or the antigen-binding fragment thereof is selected from Fab, Fab', F(ab')₂, Fd, Fv, dAb, a complementarity determining region fragment, a single chain antibody, a humanized antibody, and a chimeric antibody.

In some embodiments of the present disclosure, provided is the bispecific antibody, wherein
the anti-LAG3 antibody comprises a non-CDR region derived from a human antibody.

In some embodiments of the present disclosure, provided is the bispecific antibody, which is an anti-LAG3-anti-CD73 bispecific antibody.

In some embodiments of the present disclosure, provided is the bispecific antibody, wherein
the second protein functional region is an anti-CD73 antibody or an antigen-binding fragment thereof, and the anti-CD73 antibody comprises a heavy chain variable region and a light chain variable region; the heavy chain variable region comprises HCDR1 having the amino acid sequence set forth in SEQ ID NO: 25, HCDR2 having the amino acid sequence set forth in SEQ ID NO: 26, and HCDR3 having the amino acid sequence set forth in SEQ ID NO: 27; and
the light chain variable region comprises LCDR1 having the amino acid sequence set forth in SEQ ID NO: 28, LCDR2 having the amino acid sequence set forth in SEQ ID NO: 29, and LCDR3 having the amino acid sequence set forth in SEQ ID NO: 30.

In some embodiments of the present disclosure, provided is the bispecific antibody, wherein the heavy chain variable region of the anti-CD73 antibody is selected from amino acid sequences set forth in SEQ ID NO: 31, SEQ ID NO: 33, SEQ ID NO: 35, and SEQ ID NO: 46; the light chain variable region of the anti-CD73 antibody is selected from amino acid sequences set forth in SEQ ID NO: 32, SEQ ID NO: 34, SEQ ID NO: 36, SEQ ID NO: 37, SEQ ID NO: 47, and SEQ ID NO: 62;
preferably,
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 47;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 62;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 47;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 62;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 47;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 62;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 47; or
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 62.

In some embodiments of the present disclosure, provided is the bispecific antibody, wherein
the anti-LAG3 antibody or the anti-CD73 antibody further comprises a constant region derived from a human antibody;
preferably, the constant region of the anti-LAG3 antibody or the anti-CD73 antibody is selected from a constant region of human IgG1, IgG2, IgG3, or IgG4;
preferably, the anti-LAG3 antibody comprises a heavy chain constant region and a light chain constant region, wherein the heavy chain constant region is Ig gamma-1 chain C region (e.g., as set forth in SEQ ID NO: 18) or Ig gamma-4 chain C region (e.g., as set forth in SEQ ID NO: 20), and the light chain constant region is Ig kappa chain C region (e.g., as set forth in SEQ ID NO: 19);
preferably, the anti-CD73 antibody comprises a heavy chain constant region and a light chain constant region, wherein the heavy chain constant region is Ig gamma-1 chain C region (e.g., as set forth in SEQ ID NO: 18) or Ig gamma-4 chain C region (e.g., as set forth in SEQ ID NO: 20), and the light chain constant region is Ig kappa chain C region (e.g., as set forth in SEQ ID NO: 19).

Amino acid sequence of IgG1 heavy chain constant region








 (SEQ ID NO: 18)

In some embodiments of the present disclosure, provided is the bispecific antibody, which is characterized by any one of the following items (1) to (4):
(1)
   the anti-LAG3 antibody is of human IgG1 subtype,
   wherein according to the EU numbering system, the heavy chain constant region of the antibody has the following mutations:
      L234A and L235A;
      L234A and G237A;
      L235A and G237A; or
      L234A, L235A, and G237A;
(2)
   the anti-LAG3 antibody is of human IgG4 subtype,
   wherein according to the EU numbering system, the heavy chain constant region of the antibody has the following mutations:
      F234A and L235A;
      F234A and G237A;
      L235A and G237A; or
      F234A, L235A, and G237A;
(3)
   the anti-CD73 antibody is of human IgG1 subtype,
   wherein according to the EU numbering system, the heavy chain constant region of the antibody has the following mutations:
      L234A and L235A;
      L234A and G237A;
      L235A and G237A; or
      L234A, L235A, and G237A;
         or
(4)
   the anti-CD73 antibody is of human IgG4 subtype,
   wherein according to the EU numbering system, the heavy chain constant region of the antibody has the following mutations:
      F234A and L235A;
      F234A and G237A;
      L235A and G237A; or
      F234A, L235A, and G237A.

In some embodiments of the present disclosure, provided is the bispecific antibody, wherein
the bispecific antibody is in an IgG-scFv form;
preferably, the first protein functional region is an immunoglobulin, and the second protein functional region is a single chain antibody; or
preferably, the first protein functional region is a single chain antibody, and the second protein functional region is an immunoglobulin targeting a target other than LAG3 (e.g., CD73 or PD-1).

In some embodiments of the present disclosure, provided is the bispecific antibody, which comprises:
a first protein functional region targeting LAG3, and
a second protein functional region targeting CD73,
wherein
the first protein functional region is an anti-LAG3 antibody, the anti-LAG3 antibody is an immunoglobulin, and the second protein functional region is an anti-CD73 single chain antibody; or
the first protein functional region is an anti-LAG3 single chain antibody, the second protein functional region is an anti-CD73 antibody, and the anti-CD73 antibody is an immunoglobulin.

In some embodiments of the present disclosure, provided is the bispecific antibody, wherein
a heavy chain variable region of the immunoglobulin comprises HCDR1-HCDR3 having amino acid sequences set forth in SEQ ID NOs: 9-11, respectively, and a light chain variable region of the immunoglobulin comprises LCDR1-LCDR3 having amino acid sequences set forth in SEQ ID NOs: 12-14, respectively; a heavy chain variable region of the single chain antibody comprises HCDR1-HCDR3 having amino acid sequences set forth in SEQ ID NOs: 25-27, respectively, and a light chain variable region of the single chain antibody comprises LCDR1-LCDR3 having amino acid sequences set forth in SEQ ID NOs: 28-30 respectively; or
a heavy chain variable region of the single chain antibody comprises HCDR1-HCDR3 having amino acid sequences set forth in SEQ ID NOs: 9-11, respectively, and a light chain variable region of the single chain antibody comprises LCDR1-LCDR3 having amino acid sequences set forth in SEQ ID NOs: 12-14, respectively; a heavy chain variable region of the immunoglobulin comprises HCDR1-HCDR3 having amino acid sequences set forth in SEQ ID NOs: 25-27, respectively, and a light chain variable region of the immunoglobulin comprises LCDR1-LCDR3 having amino acid sequences set forth in SEQ ID NOs: 28-30, respectively.

In some embodiments of the present disclosure, provided is the bispecific antibody, wherein
the heavy chain variable region of the immunoglobulin is selected from amino acid sequences set forth in SEQ ID NO: 2 and SEQ ID NO: 44, and the light chain variable region of the immunoglobulin is selected from amino acid sequences set forth in SEQ ID NO: 4, SEQ ID NO: 6, SEQ ID NO: 8, and SEQ ID NO: 45; the heavy chain variable region of the single chain antibody is selected from amino acid sequences set forth in SEQ ID NO: 31, SEQ ID NO: 33, SEQ ID NO: 35, and SEQ ID NO: 46, and the light chain variable region of the single chain antibody is selected from amino acid sequences set forth in SEQ ID NO: 32, SEQ ID NO: 34, SEQ ID NO: 36, SEQ ID NO: 37, SEQ ID NO: 47, and SEQ ID NO: 62; or
the heavy chain variable region of the single chain antibody is selected from amino acid sequences set forth in SEQ ID NO: 2 and SEQ ID NO: 44, and the light chain variable region of the single chain antibody is selected from amino acid sequences set forth in SEQ ID NO: 4, SEQ ID NO: 6, SEQ ID NO: 8, and SEQ ID NO: 45; the heavy chain variable region of the immunoglobulin is selected from amino acid sequences set forth in SEQ ID NO: 31, SEQ ID NO: 33, SEQ ID NO: 35, and SEQ ID NO: 46, and the light chain variable region of the immunoglobulin is selected from amino acid sequences set forth in SEQ ID NO: 32, SEQ ID NO: 34, SEQ ID NO: 36, SEQ ID NO: 37, SEQ ID NO: 47, and SEQ ID NO: 62.

In some embodiments of the present disclosure, provided is the bispecific antibody, which is selected from any one of the following (1)-(24):
(1)
   the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 31, and the light chain variable region of the single chain antibody has the amino acid sequence set forth in SEQ ID NO: 32;
(2)
   the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 33, and the light chain variable region of the single chain antibody has the amino acid sequence set forth in SEQ ID NO: 34;
(3)
   the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 35, and the light chain variable region of the single chain antibody has the amino acid sequence set forth in SEQ ID NO: 36;
(4)
   the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 35, and the light chain variable region of the single chain antibody has the amino acid sequence set forth in SEQ ID NO: 37;
(5)
   the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 46, and the light chain variable region of the single chain antibody has the amino acid sequence set forth in SEQ ID NO: 47;
(6)
   the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 35, and the light chain variable region of the single chain antibody has the amino acid sequence set forth in SEQ ID NO: 62;
(7)
   the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 31, and the light chain variable region of the single chain antibody has the amino acid sequence set forth in SEQ ID NO: 32;
(8)
   the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 33, and the light chain variable region of the single chain antibody has the amino acid sequence set forth in SEQ ID NO: 34;
(9)
   the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 35, and the light chain variable region of the single chain antibody has the amino acid sequence set forth in SEQ ID NO: 36;
(10)
   the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 35, and the light chain variable region of the single chain antibody has the amino acid sequence set forth in SEQ ID NO: 37;
(11)
   the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 46, and the light chain variable region of the single chain antibody has the amino acid sequence set forth in SEQ ID NO: 47;
(12)
   the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 35, and the light chain variable region of the single chain antibody has the amino acid sequence set forth in SEQ ID NO: 62;
(13)
   the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 31, and the light chain variable region of the immunoglobulin has the amino acid sequence set forth in SEQ ID NO: 32;
(14)
   the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 33, and the light chain variable region of the immunoglobulin has the amino acid sequence set forth in SEQ ID NO: 34;
(15)
   the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 35, and the light chain variable region of the immunoglobulin has the amino acid sequence set forth in SEQ ID NO: 36;
(16)
   the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 35, and the light chain variable region of the immunoglobulin has the amino acid sequence set forth in SEQ ID NO: 37;
(17)
   the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 46, and the light chain variable region of the immunoglobulin has the amino acid sequence set forth in SEQ ID NO: 47;
(18)
   the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 35, and the light chain variable region of the immunoglobulin has the amino acid sequence set forth in SEQ ID NO: 62;
(19)
   the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 31, and the light chain variable region of the immunoglobulin has the amino acid sequence set forth in SEQ ID NO: 32;
(20)
   the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 33, and the light chain variable region of the immunoglobulin has the amino acid sequence set forth in SEQ ID NO: 34;
(21)
   the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 35, and the light chain variable region of the immunoglobulin has the amino acid sequence set forth in SEQ ID NO: 36;
(22)
   the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 35, and the light chain variable region of the immunoglobulin has the amino acid sequence set forth in SEQ ID NO: 37;
(23)
   the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 46, and the light chain variable region of the immunoglobulin has the amino acid sequence set forth in SEQ ID NO: 47; and
(24)
   the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 35, and the light chain variable region of the immunoglobulin has the amino acid sequence set forth in SEQ ID NO: 62.

In some embodiments of the present disclosure, provided is the bispecific antibody, wherein the immunoglobulin is of human IgG1 subtype, and according to the EU numbering system, the heavy chain constant region of the immunoglobulin has the following mutations:
L234A and L235A;
L234A and G237A;
L235A and G237A;
   or
L234A, L235A, and G237A.

In the present disclosure, letters before the position number represent amino acids before mutation, and letters after the position number represent amino acids after mutation, unless otherwise specified.

In some embodiments of the present disclosure, according to the EU numbering system, the heavy chain constant region of the immunoglobulin further has one or more mutations selected from:
N297A, D265A, D270A, P238D, L328E, E233D, H268D, P271G, A330R, C226S, C229S, E233P, P331S, S267E, L328F, A330L, M252Y, S254T, T256E, N297Q, P238S, P238A, A327Q, A327G, P329A, K322A, T394D, G236R, G236A, L328R, A330S, P331S, H268A, E318A, and K320A.

In some embodiments of the present disclosure, provided is the bispecific antibody, wherein the immunoglobulin is of human IgG4 subtype, and according to the EU numbering system, the heavy chain constant region of the immunoglobulin has the following mutations:
F234A and L235A;
F234A and G237A;
L235A and G237A; or
F234A, L235A, and G237A.

In some embodiments of the present disclosure, provided is the bispecific antibody, wherein the first protein functional region and the second protein functional region are each independently 1, 2, or more in number; preferably, the first protein functional region and the second protein functional region are linked directly or via a linker;
preferably, the heavy chain variable region and the light chain variable region of the anti-LAG3 single chain antibody are linked directly or via a linker;
preferably, the heavy chain variable region and the light chain variable region of the anti-CD73 single chain antibody are linked directly or via a linker;
preferably, the linker is independently the polypeptide set forth in SEQ ID NO: 48 (GGGGS) or a polypeptide formed by concatenating a plurality of (e.g., 2, 3, 4, 5, or 6) polypeptides set forth in SEQ ID NO: 48;
preferably, the linker is independently a polypeptide having one or more glycines linked to the C-terminus of the polypeptide set forth in SEQ ID NO: 48 or a polypeptide formed by concatenating a plurality of (e.g., 2, 3, 4, 5, or 6) polypeptides set forth in SEQ ID NO: 48.

In some embodiments of the present disclosure, provided is the bispecific antibody, wherein each single chain antibody is linked to the C-terminus or the N-terminus of one of the two heavy chains of the immunoglobulin. In some embodiments of the present disclosure, provided is the bispecific antibody, which comprises:
a first protein functional region targeting LAG3, and
a second protein functional region targeting CD73,
the first protein functional region is 1 in number, and the second protein functional region is 2 in number;
wherein the first protein functional region is an immunoglobulin, and the second protein functional region is a single chain antibody;
a heavy chain of the immunoglobulin has the amino acid sequence set forth in SEQ ID NO: 38, and a light chain of the immunoglobulin has the amino acid sequence set forth in SEQ ID NO: 39;
a heavy chain variable region of the single chain antibody has the amino acid sequence set forth in SEQ ID NO: 35, and a light chain variable region of the single chain antibody has the amino acid sequence set forth in SEQ ID NO: 62; or a heavy chain variable region of the single chain antibody has the amino acid sequence set forth in SEQ ID NO: 46, and a light chain variable region of the single chain antibody has the amino acid sequence set forth in SEQ ID NO: 47;
each single chain antibody is linked to the C-terminus of one of the two heavy chains of the immunoglobulin;
the first protein functional region and the second protein functional region are linked via a first linker; the heavy chain variable region of the single chain antibody and the light chain variable region of the single chain antibody are linked via a second linker; the first linker and the second linker are identical or different;
preferably, the first linker and the second linker each have an amino acid sequence independently selected from SEQ ID NO: 42 and SEQ ID NO: 43;
preferably, the first linker and the second linker both have the amino acid sequence set forth in SEQ ID NO: 43;
preferably, according to the EU numbering system, the constant region of the heavy chain (SEQ ID NO: 38) of the immunoglobulin comprises the following mutations:
   L234A and L235A;
   L234A and G237A;
   L235A and G237A; or
   L234A, L235A, and G237A.

In some embodiments of the present disclosure, provided is the bispecific antibody, which comprises:
a first protein functional region targeting LAG3, and
a second protein functional region targeting CD73,
the first protein functional region is 2 in number, and the second protein functional region is 1 in number;
wherein the first protein functional region is a single chain antibody, and the second protein functional region is an immunoglobulin;
a heavy chain of the immunoglobulin has the amino acid sequence set forth in SEQ ID NO: 40, and a light chain of the immunoglobulin has the amino acid sequence set forth in SEQ ID NO: 41;
a heavy chain variable region of the single chain antibody has the amino acid sequence set forth in SEQ ID NO: 2, and a light chain variable region of the single chain antibody has the amino acid sequence set forth in SEQ ID NO: 4; or a heavy chain variable region of the single chain antibody has the amino acid sequence set forth in SEQ ID NO: 44, and a light chain variable region of the single chain antibody has the amino acid sequence set forth in SEQ ID NO: 45;
each single chain antibody is linked to the C-terminus of one of the two heavy chains of the immunoglobulin;
the first protein functional region and the second protein functional region are linked via a first linker; the heavy chain variable region of the single chain antibody and the light chain variable region of the single chain antibody are linked via a second linker; the first linker and the second linker are identical or different;
preferably, the first linker and the second linker each have an amino acid sequence independently selected from SEQ ID NO: 42 and SEQ ID NO: 43;
preferably, the first linker and the second linker both have the amino acid sequence set forth in SEQ ID NO: 43;
preferably, according to the EU numbering system, the constant region of the heavy chain (SEQ ID NO: 40) of the immunoglobulin comprises the following mutations:
   L234A and L235A;
   L234A and G237A;
   L235A and G237A; or
   L234A, L235A, and G237A.

Provided is the bispecific antibody according to any one of the embodiments of the present disclosure, which is for use in treating or preventing a tumor, wherein
preferably, the tumor is selected from one or more of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

Another aspect of the present disclosure relates to an isolated nucleic acid molecule encoding the bispecific antibody according to any one of the embodiments of the present disclosure.

Yet another aspect of the present disclosure relates to a recombinant vector comprising the isolated nucleic acid molecule of the present disclosure.

Yet another aspect of the present disclosure relates to a host cell comprising the isolated nucleic acid molecule of the present disclosure or the recombinant vector of the present disclosure.

Yet another aspect of the present disclosure relates to a method for preparing the bispecific antibody according to any one of the embodiments of the present disclosure, which comprises culturing the host cell of the present disclosure under suitable conditions and isolating the bispecific antibody from the cell cultures.

Yet another aspect of the present disclosure relates to a pharmaceutical composition comprising the bispecific antibody according to any one of the embodiments of the present disclosure; optionally, the pharmaceutical composition further comprises a pharmaceutically acceptable auxiliary material.

Yet another aspect of the present disclosure relates to use of the bispecific antibody according to any one of the embodiments of the present disclosure in the preparation of a medicament for treating or preventing a tumor;
preferably, the tumor is selected from one or more of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

Yet another aspect of the present disclosure relates to a method for treating or preventing a tumor, which comprises a step of administering to a subject in need an effective amount of the bispecific antibody according to any one of the embodiments of the present disclosure;
preferably, the tumor is selected from one or more of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

In one or more embodiments of the present disclosure, provided is the method for treating or preventing a tumor, wherein
the anti-LAG3-anti-CD73 bispecific antibody is administered at a single dose of 0.1-100 mg per kg body weight, preferably 1-15 mg, 1-12 mg, 1-10 mg (e.g., 1 mg, 2 mg, 3 mg, 4 mg, 5 mg, 6 mg, 7 mg, 8 mg, 9 mg, or 10 mg), or 6-10 mg per kg body weight; or the anti-LAG3-anti-CD73 bispecific antibody is administered to each subject at a single dose of 10-1000 mg (e.g., about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, or about 1000 mg), preferably 50-500 mg, 100-400 mg, 150-300 mg, 150-250 mg, or 200 mg;
preferably, administration is performed once every 3 days, 4 days, 5 days, 6 days, 10 days, 1 week, 2 weeks, or 3 weeks;
preferably, a route of administration is intravenous drip infusion or intravenous injection.

In some embodiments, the administration of the anti-LAG3-anti-CD73 bispecific antibody is performed in cycles of 2 weeks (14 days) or 3 weeks (21 days), and preferably, the anti-LAG3-anti-CD73 bispecific antibody is administered intravenously on the first day (D1) of each cycle. For example, the anti-LAG3-anti-CD73 bispecific antibody is administered once every two weeks (q2w) or three weeks (q3w).

However, it will be appreciated that the total daily dose of the drug (e.g., the pharmaceutical composition) or the pharmaceutically active ingredient (e.g., the anti-LAG3-anti-CD73 bispecific antibody) of the present disclosure must be determined by an attending physician within the scope of reliable medical judgment. For any specific patient, the specific therapeutically effective dose level is determined based on a variety of factors including the specific type and severity of the tumor being treated, the form of the specific drug formulation employed, the age, body weight, general health condition, gender, and diet of the patient, the time of administration, the route of administration, excretion rate, the duration of the treatment, other drugs used concurrently, and similar factors well known in the medical field. For example, the practice in the art involves initiating administration at dose levels below those required to achieve the desired therapeutic effect and gradually increasing the dose until the desired effect is attained.

In the present disclosure, unless otherwise defined, the scientific and technical terms used herein have the meanings generally understood by those skilled in the art. In addition, the laboratory operations of cell culture, molecular genetics, nucleic acid chemistry, and immunology used herein are the routine procedures widely used in the corresponding fields. Meanwhile, in order to better understand the present disclosure, the definitions and explanations of the related terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol., 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously.

The monoclonal antibody is highly specific for a single epitope on an antigen. The polyclonal antibody, relative to the monoclonal antibody, generally comprises at least two or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or an antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction, or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as **λ** phages or M13 phages; and animal viruses. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site.

As used herein, the term "host cell" refers to cells to which vectors can be introduced, including, but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, GS cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M, or 10⁻¹⁰ M or less.

As used herein, the term "K_{D}" refers to a dissociation equilibrium constant for a specific antibody-antigen interaction and is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, such as less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio molecular interaction instrument.

As used herein, the terms "monoclonal antibody" and "mAb" have the same meaning and are used interchangeably; the terms "polyclonal antibody" and "pAb" have the same meaning and are used interchangeably. Besides, in the present disclosure, amino acids are generally represented by single-letter and three-letter abbreviations known in the art. For example, alanine can be represented by A or Ala. As used herein, the term "pharmaceutically acceptable auxiliary material" refers to a carrier and/or excipient that is pharmacologically and/or physiologically compatible with the subject and the active ingredient, which is well known in the art (see, e.g., Remington's Pharmaceutical Sciences. Edited by Gennaro AR, 19th ed. Pennsylvania: Mack Publishing Company, 1995), and includes, but is not limited to, pH regulators, surfactants, adjuvants, and ionic strength enhancers. For example, the pH regulators include, but are not limited to, phosphate buffer; the surfactants include, but are not limited to, cationic, anionic, or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the development of the disease (e.g., a tumor); a therapeutically effective amount refers to an amount sufficient to cure or at least partially stop the disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

As used herein, when referring to the amino acid sequence of lymphocyte-activation gene 3 (LAG3), it includes the full length of the LAG3 protein, or the extracellular fragment LAG3 ECD of LAG3, or a fragment comprising LAG3 ECD, and it also includes a fusion protein of the full length of the LAG3 protein or a fusion protein of LAG3 ECD, such as a fragment fused to an Fc protein fragment of mouse or human IgG (mFc or hFc). However, those skilled in the art will appreciate that in the amino acid sequence of the LAG3 protein, mutations or variations (including but not limited to, substitutions, deletions, and/or additions) can be naturally produced or artificially introduced without affecting biological functions thereof. Therefore, in the present disclosure, the term "LAG3" or "LAG3 protein" shall include all such sequences, including their natural or artificial variants. In addition, when describing a sequence fragment of the LAG3 protein, it also includes the corresponding sequence fragments in their natural or artificial variants.

As used herein, when referring to the amino acid sequence of CD73, it includes the full length of the CD73 protein, or the extracellular fragment CD73 ECD of CD73, or a fragment comprising CD73 ECD, and it also includes a fusion protein of the full length of the CD73 protein or a fusion protein of CD73 ECD, such as a fragment fused to an Fc protein fragment of mouse or human IgG (mFc or hFc). However, those skilled in the art will appreciate that in the amino acid sequence of the CD73 protein, mutations or variations (including but not limited to, substitutions, deletions, and/or additions) can be naturally produced or artificially introduced without affecting biological functions thereof. Therefore, in the present disclosure, the term "CD73" or "CD73 protein" shall include all such sequences, including their natural or artificial variants. In addition, when describing a sequence fragment of the CD73 protein, it also includes the corresponding sequence fragments in their natural or artificial variants.

In the present disclosure, the terms "first" (e.g., first protein functional region or first linker) and "second" (e.g., second protein functional region or second linker) are used for distinguishing or clarity in expression and do not carry typical sequential meanings, unless otherwise specified.

### Beneficial Effects of the Present Disclosure

The present disclosure achieves one or more of the following technical effects:
(1) the bispecific antibody of the present disclosure can specifically bind to LAG3 well and effectively block the binding of LAG3 to MHC-II, thereby specifically relieving the immunosuppression of LAG3 on an organism;
(2) the bispecific antibody of the present disclosure can effectively inhibit the enzyme activity reaction of CD73;
(3) the bispecific antibody of the present disclosure can promote INF-y secretion by T cells;
(4) the first protein functional region and the second protein functional region in the bispecific antibody of the present disclosure have a synergistic effect;
(5) the bispecific antibody of the present disclosure can effectively inhibit tumor growth in a mouse xenograft tumor model;
(6) the antibody of the present disclosure can effectively treat or prevent tumors.

### BRIEF DESCRIPTION OF THE DRAWINGS

FIG. 1: Results of assays for the binding activity of H9L8(hG4WT), H9L9(hG4WT), and H9L10(hG4WT) for antigen human LAG3-mG1Fc by ELISA.
FIG. 2: Results of assays for the binding activity of H9L8(hG4WT), H9L9(hG4WT), and H9L10(hG4WT) to antigen human LAG3 on 293T-LAG3 cell surface by FACS.
FIG. 3: Results of assays for H9L8(hG4WT), H9L9(hG4WT), and H9L10(hG4WT) competing with antigen MHC II on Raji cell membrane surface for binding to human LAG3-mG1Fc by competitive flow cytometry.
FIG. 4: Results of assays for the biological activity of anti-LAG3 antibodies in promoting IFN-y secretion by mixed lymphocyte reaction (MLR).
FIG. 5: Results of assays for the biological activity of anti-LAG3 antibodies in promoting IL-2 secretion by mixed lymphocyte reaction (MLR).
FIG. 6: Results of assays for the biological activity of anti-LAG3 antibodies in blocking the interaction between LAG3 and MHC-II.
FIG. 7: Results of assays for the binding of LA5EV1 and LA5EV2 to antigen NT5E-His by ELISA.
FIG. 8: Results of assays for the binding of NTLAV2 and NTLAV7 to antigen NT5E-His by ELISA.
FIG. 9: Results of assay for the binding of NTLAV8 to antigen NT5E-His by ELISA.
FIG. 10: Results of assays for the binding of LA5EV1 and LA5EV2 to antigen huLAG3-mG1Fc by ELISA.
FIG. 11: Results of assays for the binding of NTLAV2 and NTLAV7 to antigen huLAG3-mG1Fc by ELISA.
FIG. 12: Results of assay for the binding of NTLAV8 to antigen huLAG3-mG1Fc by ELISA.
FIG. 13: Affinity constant of antibody LA5EV1 for hNT5E(1-552)-His.
FIG. 14: Affinity constant of antibody LA5EV2 for hNT5E(1-552)-His.
FIG. 15: Affinity constant of antibody NTLAV2 for hNT5E(1-552)-His.
FIG. 16: Affinity constant of antibody NTLAV7 for hNT5E(1-552)-His.
FIG. 17: Affinity constant of antibody NTLAV8 for hNT5E(1-552)-His.
FIG. 18: Affinity constant of antibody 19F3H2L3(hG1DM) for hNT5E(1-552)-His.
FIG. 19: Affinity constant of antibody LA5EV1 for huLAG3-mG1Fc.
FIG. 20: Affinity constant of antibody LA5EV2 for huLAG3-mG1Fc.
FIG. 21: Affinity constant of antibody NTLAV2 for huLAG3-mG1Fc.
FIG. 22: Affinity constant of antibody NTLAV7 for huLAG3-mG1Fc.
FIG. 23: Affinity constant of antibody NTLAV8 for huLAG3-mG1Fc.
FIG. 24: Affinity constant of antibody H9L8(hG4WT) for huLAG3-mG1Fc.
FIG. 25: Affinity constant of antibody Relatlimab for huLAG3-mG1Fc.
FIG. 26: Results of assays for the binding activity of NTLAV8, LA5EV2, H9L8(hG4WT), and Relatlimab for LAG3 on 293T-LAG3 cell surface by FACS.
FIG. 27: Results of assays for the binding activity of NTLAV8, LA5EV2, 19F3H2L3(hG1DM), and CPI-006 for CD73 on U87-MG cell surface by FACS.
FIG. 28: Results of assays for NTLAV8, LA5EV2, Relatlimab, and H9L8 competing with MHC II on Raji cell surface for binding to the human LAG3-mG1Fc antigen by FACS.
FIG. 29: Results for the blocking of the interaction between LAG3 and MHCII by antibodies NTLAV8 and H9L8(hG4WT).
FIG. 30: Results of assays for the inhibition of the enzyme activity of CD73 on cell membrane surface by anti-CD73-anti-LAG3 bispecific antibodies.
FIG. 31: Results of assays for the biological activity of anti-CD73-anti-LAG3 bispecific antibodies in promoting IFN-y secretion by mixed lymphocyte reaction.
FIG. 32: Results of assays for the potential cell phagocytic activity mediated by anti-CD73-anti-LAG3 bispecific antibodies on CHO-K1-LAG3-CD73 target cells.
FIG. 33: Results of pharmacodynamic evaluation of anti-CD73-anti-LAG3 bispecific antibody in a mouse model subcutaneously grafted with tumor cells.
FIG. 34: Results for the effects of anti-CD73-anti-LAG3 bispecific antibody on body weight in a mouse model subcutaneously grafted with tumor cells.

### DETAILED DESCRIPTION

The embodiments of the present disclosure will be described in detail below with reference to the examples. Those skilled in the art will appreciate that the following examples are only for illustrating the present disclosure, and should not be construed as limitations to the scope of the present disclosure. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

The positive control antibody, Relatlimab, has sequences referenced to the U.S. Patent Publication No. US20160326248A1, wherein for the heavy chain amino acid sequence, see SEQ ID NO: 1 of this patent publication, and for the light chain amino acid sequence, see SEQ ID NO: 2 of this patent publication. Relatlimab is an anti-LAG3 antibody.
Heavy chain amino acid sequence of Relatlimab: 
QVQLQQWGAGLLKPSETLSLTCAVYGGSFSDYYWNWIRQPPGK
GLEWIGEINHRGSTNSNPSLKSRVTLSLDTSKNQFSLKLRSVTAA
DTAVYYCAFGYSDYEYNWFDPWGQGTLVTVSSASTKGPSVFPLA
PCSRSTSESTAALGCLVKDYFPEPVTVSWNSGALTSGVHTFPAVL
QSSGLYSLSSVVTVPSSSLGTKTYTCNVDHKPSNTKVDKRVESKY
GPPCPPCPAPEFLGGPSVFLFPPKPKDTLMISRTPEVTCVVVDVSQ
EDPEVQFNWYVDGVEVHNAKTKPREEQFNSTYRVVSVLTVLHQ
DWLNGKEYKCKVSNKGLPSSIEKTISKAKGQPREPQVYTLPPSQE
EMTKNQVSLTCLVKGFYPSDIAVEWESNGQPENNYKTTPPVLDS
DGSFFLYSRLTVDKSRWQEGNVFSCSVMHEALHNHYTQKSLSLS
LGK (SEQ ID NO: 23)
Light chain amino acid sequence of Relatlimab:

EIVLTQSPATLSLSPGERATLSCRASQSISSYLAWYQQKPGQAPRL
LIYDASNRATGIPARFSGSGSGTDFTLTISSLEPEDFAVYYCQQRS
NWPLTFGQGTNLEIKRTVAAPSVFIFPPSDEQLKSGTASVVCLLN
NFYPREAKVQWKVDNALQSGNSQESVTEQDSKDSTYSLSSTLTLS
KADYEKHKVYACEVTHQGLSSPVTKSFNRGEC (SEQ ID NO: 24)

Antibody 14C12H1L1(hG1TM) is an anti-PD-1 antibody prepared by Akeso Biopharma Inc.
Heavy chain amino acid sequence of 14C12H1L1(hG1TM): 
EVQLVESGGGLVQPGGSLRLSCAASGFAFSSYDMSWVRQAPGKG
LDWVATISGGGRYTYYPDSVKGRFTISRDNSKNNLYLQMNSLRA
EDTALYYCANRYGEAWFAYWGQGTLVTVSSASTKGPSVFPLAPS
SKSTSGGTAALGCLVKDYFPEPVTVSWNSGALTSGVHTFPAVLQS
SGLYSLSSVVTVPSSSLGTQTYICNVNHKPSNTKVDKKVEPKSCD
KTHTCPPCPAPEAAGAPSVFLFPPKPKDTLMISRTPEVTCVVVDV
SHEDPEVKFNWYVDGVEVHNAKTKPREEQYNSTYRVVSVLTVL
 
HQDWLNGKEYKCKVSNKALPAPIEKTISKAKGQPREPQVYTLPP
SRDELTKNQVSLTCLVKGFYPSDIAVEWESNGQPENNYKTTPPVL
DSDGSFFLYSKLTVDKSRWQQGNVFSCSVMHEALHNHYTQKSLS
LSPGK (SEQ ID NO: 21)
Light chain amino acid sequence of 14C12H1L1(hG1TM): 
DIQMTQSPSSMSASVGDRVTFTCRASQDINTYLSWFQQKPGKSPK
TLIYRANRLVSGVPSRFSGSGSGQDYTLTISSLQPEDMATYYCLQ
YDEFPLTFGAGTKLELKRTVAAPSVFIFPPSDEQLKSGTASVVCLL
NNFYPREAKVQWKVDNALQSGNSQESVTEQDSKDSTYSLSSTLTL
SKADYEKHKVYACEVTHQGLSSPVTKSFNRGEC (SEQ ID NO: 22)
Amino acid sequence of human LAG3-mG1Fc: 
LQPGAEVPVVWAQEGAPAQLPCSPTIPLQDLSLLRRAGVTWQHQ
PDSGPPAAAPGHPLAPGPHPAAPSSWGPRPRRYTVLSVGPGGLRS
GRLPLQPRVQLDERGRQRGDFSLWLRPARRADAGEYRAAVHLR
DRALSCRLRLRLGQASMTASPPGSLRASDWVILNCSFSRPDRPAS
VHWFRNRGQGRVPVRESPHHHLAESFLFLPQVSPMDSGPWGCIL
TYRDGFNVSIMYNLTVLGLEPPTPLTVYAGAGSRVGLPCRLPAG
VGTRSFLTAKWTPPGGGPDLLVTGDNGDFTLRLEDVSQAQAGTY
TCHIHLQEQQLNATVTLAIITVTPKSFGSPGSLGKLLCEVTPVSGQ
ERFVWSSLDTPSQRSFSGPWLEAQEAQLLSQPWQCQLYQGERLL
GAAVYFTELSSPGAQRSGRAPGALPAGHLKLENLYFQGPRGPTIK
PCPPCKCPAPNLLGGPSVFIFPPKIKDVLMISLSPIVTCVVVDVSED
DPDVQISWFVNNVEVHTAQTQTHREDYNSTLRVVSALPIQHQDW
MSGKEFKCKVNNKDLPAPIERTISKPKGSVRAPQVYVLPPPEEEM
TKKQVTLTCMVTDFMPEDIYVEWTNNGKTELNYKNTEPVLDSD
GSYFMYSKLRVEKKNWVERNSYSCSVVHEGLHNHHTTKSFSRTP
GK (SEQ ID NO: 60)
Amino acid sequence of hNT5E(1-552)-His:

WELTILHTNDVHSRLEQTSEDSSKCVNASRCMGGVARLFTKVQQ
IRRAEPNVLLLDAGDQYQGTIWFTVYKGAEVAHFMNALRYDAM
ALGNHEFDNGVEGLIEPLLKEAKFPILSANIKAKGPLASQISGLYL
PYKVLPVGDEVVGIVGYTSKETPFLSNPGTNLVFEDEITALQPEV
DKLKTLNVNKIIALGHSGFEMDKLIAQKVRGVDVVVGGHSNTFL
YTGNPPSKEVPAGKYPFIVTSDDGRKVPVVQAYAFGKYLGYLKIE
FDERGNVISSHGNPILLNSSIPEDPSIKADINKWRIKLDNYSTQELG
KTIVYLDGSSQSCRFRECNMGNLICDAMINNNLRHTDEMFWNHV
SMCILNGGGIRSPIDERNNGTITWENLAAVLPFGGTFDLVQLKGS
TLKKAFEHSVHRYGQSTGEFLQVGGIHVVYDLSRKPGDRVVKLD
VLCTKCRVPSYDPLKMDEVYKVILPNFLANGGDGFQMIKDELLR
HDSGDQDINVVSTYISKMKVIYPAVEGRIKFSTGSHHHHHH (SEQ
ID NO: 61)

The cell line 293T-LAG3 was constructed by Akeso Biopharma Inc. The cell line 293T-LAG3 was produced by viral infection of HEK293T cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-huLAG3FL-BSD (LAG3, Genebank ID: NP_002277.4; vector plenti6.3/V5-BSD, purchased from Invitrogen, Cat. No. K5315-20). The cell line Raji-PDL1 was constructed by Akeso Biopharma Inc. The cell line Raji-PDL1 was produced by viral infection of Raji cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998.72(11): 8463-8471), wherein the lentivirus expression vector used was plenti6.3/V5-PDL1 (PDL1, Genebank ID: NP_054862.1; vector plenti6.3/V5, purchased from Invitrogen, Cat. No. K5315-20).

The cell line Jurkat-NFAT-PD1-LAG3 was constructed by Akeso Biopharma Inc. The cell line Jurkat-NFAT-PD1-LAG3 was produced by viral infection of PD-1 effector cells (CPM, manufacturer: Promega, Cat. No. J112A) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was pCDH-huLAG3FL-RFP-NEO (LAG3, Genebank ID: NP_002277.4; vector pCDH-CMV-MCS-EF1-RFP+Neo, purchased from Youbio, Cat. No. VT9005).

The cell line CHO-K1-LAG3-CD73 was constructed by Akeso Biopharma Inc. The cell line CHO-K1-LAG3-CD73 was produced by viral infection of CHO-K1 cells (manufacturer: Institute of Basic Medical Sciences of the Chinese Academy of Medical Sciences, Cat. No. 3111C0001CCC000004) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vectors used were plenti6.3/V5-huLAG3FL-BSD (LAG3, Genebank ID: NP_002277.4; vector plenti6.3/V5-BSD, purchased from Invitrogen, Cat. No. K5315-20) and **pCDH-GFP-NT5EFL-puro** (NT5E, Genebank ID: NP_002517.1; vector pCDH-puro, purchased from Youbio).

Sequence information of positive control antibody MEDI9447 is as follows. MEDI9447 is an anti-CD73 antibody.
Heavy chain amino acid sequence of MEDI9447 
EVQLLESGGGLVQPGGSLRLSCAASGFTFSSYAYSWVRQAPGKG
LEWVSAISGSGGRTYYADSVKGRFTISRDNSKNTLYLQMNSLRAE
DTAVYYCARLGYGRVDEWGRGTLVTVSSASTKGPSVFPLAPSSK
STSGGTAALGCLVKDYFPEPVTVSWNSGALTSGVHTFPAVLQSSG
LYSLSSVVTVPSSSLGTQTYICNVNHKPSNTKVDKRVEPKSCDKT
HTCPPCPAPEFEGGPSVFLFPPKPKDTLMISRTPEVTCVVVDVSHE
DPEVKFNWYVDGVEVHNAKTKPREEQYNSTYRVVSVLTVLHQD
WLNGKEYKCKVSNKALPASIEKTISKAKGQPREPQVYTLPPSREE
MTKNQVSLTCLVKGFYPSDIAVEWESNGQPENNYKTTPPVLDSD
GSFFLYSKLTVDKSRWQQGNVFSCSVMHEALHNHYTQKSLSLSP
GK (SEQ ID NO: 56)
Light chain amino acid sequence of MEDI9447

QSVLTQPPSASGTPGQRVTISCSGSLSNIGRNPVNWYQQLPGTAP
KLLIYLDNLRLSGVPDRFSGSKSGTSASLAISGLQSEDEADYYCAT
WDDSHPGWTFGGGTKLTVLGQPKAAPSVTLFPPSSEELQANKAT
LVCLISDFYPGAVTVAWKADSSPVKAGVETTTPSKQSNNKYAASS
YLSLTPEQWKSHRSYSCQVTHEGSTVEKTVAPTECS (SEQ ID NO:
57)

Sequence information of positive control antibody CPI-006 is as follows. CPI-006 is an anti-CD73 antibody.
Heavy chain amino acid sequence of CPI-006 
QVQLVQSGAEVEKPGASVKVSCKASGYTFTSYWITWVRQAPGQ
GLEWMGDIYPGSGNTNYNEKFKTRVTITADKSTSTAYMELSSLRS
EDTAVYYCAKEGGLTTEDYALDYWGQGTLVTVSSASTKGPSVFP
LAPSSKSTSGGTAALGCLVKDYFPEPVTVSWNSGALTSGVHTFPA
VLQSSGLYSLSSVVTVPSSSLGTQTYICNVNHKPSNTKVDKKVEP
KSCDKTHTCPPCPAPELLGGPSVFLFPPKPKDTLMISRTPEVTCV
 
VVDVSHEDPEVKFNWYVDGVEVHNAKTKPREEQYQSTYRVVSV
LTVLHQDWLNGKEYKCKVSNKALPAPIEKTISKAKGQPREPQVY
TLPPSRDELTKNQVSLTCLVKGFYPSDIAVEWESNGQPENNYKTT
PPVLDSDGSFFLYSKLTVDKSRWQQGNVFSCSVMHEALHNHYTQ
KSLSLSPGK (SEQ ID NO: 58)
Light chain amino acid sequence of CPI-006

EIVLTQSPATLSLSPGERATLSCRASKNVSTSGYSYMHWYQQKPG
QAPRLLIYLASNLESGIPPRFSGSGYGTDFTLTINNIESEDAAYYFC
QHSRELPFTFGQGTKVEIKRTVAAPSVFIFPPSDEQLKSGTASVVC
LLNNFYPREAKVQWKVDNALQSGNSQESVTEQDSKDSTYSLSSTL
TLSKADYEKHKVYACEVTHQGLSSPVTKSFNRGEC (SEQ ID NO:
59)

### Preparation Example 1: Design and Preparation of Anti-LAG3 Antibodies

### 1. Design of antibodies

The inventors creatively designed a series of antibody sequences based on the known LAG3 protein sequence (NCBI Reference Sequence: NP_002277.4), the three-dimensional crystal structure thereof, etc. Through extensive screening and testing, humanized monoclonal antibodies specifically binding to LAG3 were finally obtained, named H9L8, H9L9, and H9L10, respectively. The amino acid sequences of the heavy and light chain variable regions of the monoclonal antibodies and the encoding sequences thereof are as follows.
Nucleotide sequence of the heavy chain variable region H9v of H9L8 (360 bp): 
CAGGTGCAGCTGCAGCAGTGGGGAGCTGGACTGCTGAAACCT
AGCGAGACACTGAGCCTGACCTGTGCTGTGTACGGCGGATCT
 
ATCAGCGATTACTACTGGAACTGGATCAGGCAGCCCCCTGGA
AAGGGACTGGAATGGATCGGAGAGATCAACTACAGGGGCACC
ACCAACTCCAATCCCTCTCTGAAGAGCAGGGTGACACTGAGC
CTCGACACAAGCAAGAATCAGTTCAGCCTGAAGCTGAGGTCC
GTGACCGCTGCTGATACAGCTGTGTACTACTGTGCCTTCGGCT
ACAGCGATTACGAGTACGATTGGTTCGACCCTTGGGGCCAGG
GAACACTGGTTACAGTGAGCTCC (SEQ ID NO: 1)
Amino acid sequence of the heavy chain variable region H9v of H9L8 (120 aa): 
QVQLQQWGAGLLKPSETLSLTCAVYGGSISDYYWNWIRQPPGK
GLEWIGEINYRGTTNSNPSLKSRVTLSLDTSKNQFSLKLRSVTAA
DTAVYYCAFGYSDYEYDWFDPWGQGTLVTVSS (SEQ ID NO: 2)
Nucleotide sequence of the light chain variable region L8v of H9L8 (321 bp): 
GAGATCGTTCTGACCCAGAGCCCAGCTACACTGAGCCTGTCT
CCTGGAGAGAGGGCTACACTGTCCTGCAGAGCTAGCCAGACC
ATCAGCAGCTACCTGGCTTGGTACCAGCAGAAGCCTGGCCAA
GCTCCAAGGCTGCTGATCTACGACGCCTCTAATAGGGCCACC
GGCATCCCTGCTAGATTCTCTGGAAGCGGCAGCGGAACCGAC
TTTACACTGACAATCAGCTCCCTGGAGCCCGAGGATTTCGCTG
TTTACTACTGTCAGCAGCGCAGCAACTGGCCCATCACATTCGG
ACAGGGCACAAATCTGGAGATCAAG (SEQ ID NO: 3)
Amino acid sequence of the light chain variable region L8v of H9L8 (107 aa):

EIVLTQSPATLSLSPGERATLSCRASQTISSYLAWYQQKPGQAPRL
LIYDASNRATGIPARFSGSGSGTDFTLTISSLEPEDFAVYYCQQRS
NWPITFGQGTNLEIK (SEQID NO: 4)

The nucleotide sequence of the heavy chain variable region H9v of H9L9 is identical to the nucleotide sequence of the heavy chain variable region H9v of H9L8, as set forth in SEQ ID NO: 1.

The amino acid sequence of the heavy chain variable region H9v of H9L9 is identical to the amino acid sequence of the heavy chain variable region H9v of H9L8, as set forth in SEQ ID NO: 2.
Nucleotide sequence of the light chain variable region L9v of H9L9 (321 bp): 
GAGATCGTTCTGACCCAGAGCCCAGCTACACTGAGCCTGTCT
CCTGGAGAGAGGGCTACACTGTCCTGCAGAGCTAGCCAGACC
ATCAGCAGCTACCTGGCTTGGTACCAGCAGAAGCCTGGCCAA
GCTCCAAGGCTGCTGATCTACGACGGCTCTAATAGGGCCACC
GGCATCCCTGCTAGATTCTCTGGAAGCGGCAGCGGAACCGAC
TTTACACTGACAATCAGCTCCCTGGAGCCCGAGGATTTCGCTG
TTTACTACTGTCAGCAGCGCAGCAACTGGCCCCTCACATTCGG
ACAGGGCACAAATCTGGAGATCAAG (SEQ ID NO: 5)
Amino acid sequence of the light chain variable region L9v of H9L9 (107 bp):

EIVLTQSPATLSLSPGERATLSCRASQTISSYLAWYQQKPGQAPRL
LIYDGSNRATGIPARFSGSGSGTDFTLTISSLEPEDFAVYYCQQRS
NWPLTFGQGTNLEIK (SEQ ID NO: 6)

The nucleotide sequence of the heavy chain variable region H9v of H9L10 is identical to the nucleotide sequence of the heavy chain variable region H9v of H9L8, as set forth in SEQ ID NO: 1.

The amino acid sequence of the heavy chain variable region H9v of H9L10 is identical to the amino acid sequence of the heavy chain variable region H9v of H9L8, as set forth in SEQ ID NO: 2.
Nucleotide sequence of the light chain variable region L10v of H9L10 (321 bp): 
GAGATCGTTCTGACCCAGAGCCCAGCTACACTGAGCCTGTCT
CCTGGAGAGAGGGCTACACTGTCCTGCAGAGCTAGCCAGTCC
ATCAGCAGCTACCTGGCTTGGTACCAGCAGAAGCCTGGCCAA
GCTCCAAGGCTGCTGATCTACGACGGCTCTAATAGGGCCACC
GGCATCCCTGCTAGATTCTCTGGAAGCGGCAGCGGAACCGAC
TTTACACTGACAATCAGCTCCCTGGAGCCCGAGGATTTCGCTG
TTTACTACTGTCAGCAGCGCAGCAACTGGCCCATCACATTCGG
ACAGGGCACAAATCTGGAGATCAAG (SEQ ID NO: 7)
Amino acid sequence of the light chain variable region L10v of H9L10 (107 bp):

EIVLTQSPATLSLSPGERATLSCRASQSISSYLAWYQQKPGQAPRL
LIYDGSNRATGIPARFSGSGSGTDFTLTISSLEPEDFAVYYCQQRS
NWPITFGQGTNLEIK (SEQ ID NO: 8)

The amino acid sequences of the CDRs of the antibody H9L8 are as follows (according to the IMGT numbering system):
HCDR1: GGSISDYY (SEQ ID NO: 9);
HCDR2: INYRGTT (SEQ ID NO: 10);
HCDR3: AFGYSDYEYDWFDP (SEQ ID NO: 11);
LCDR1: QTISSY (SEQ ID NO: 12);
LCDR2: DAS (SEQ ID NO: 13);
LCDR3: QQRSNWPIT (SEQ ID NO: 14).

The amino acid sequences of the CDRs of the antibody H9L9 are as follows (according to the IMGT numbering system):
HCDR1: GGSISDYY (SEQ ID NO: 9);
HCDR2: INYRGTT (SEQ ID NO: 10);
HCDR3: AFGYSDYEYDWFDP (SEQ ID NO: 11);
LCDR1: QTISSY (SEQ ID NO: 12);
LCDR2: DGS (SEQ ID NO: 15);
LCDR3: QQRSNWPLT (SEQ ID NO: 16).

The amino acid sequences of the CDRs of the antibody H9L10 are as follows (according to the IMGT numbering system):
HCDR1: GGSISDYY (SEQ ID NO: 9);
HCDR2: INYRGTT (SEQ ID NO: 10);
HCDR3: AFGYSDYEYDWFDP (SEQ ID NO: 11);
LCDR1: QSISSY (SEQ ID NO: 17);
LCDR2: DGS (SEQ ID NO: 15);
LCDR3: QQRSNWPIT (SEQ ID NO: 14).

### 2. Expression and purification of humanized antibodies H9L8(hG4WT), H9L9(hG4WT), and H9L10(hG4WT)

The heavy chain cDNA sequences (the encoding sequences of the variable regions are set forth in SEQ ID NO: 1; the constant regions were Ig gamma-4 chain C regions) of H9L8(hG4WT), H9L9(hG4WT), and H9L10(hG4WT), the light chain cDNA sequence (the encoding sequence of the variable region is set forth in SEQ ID NO: 3; the constant region was human Ig kappa chain C region) of H9L8(hG4WT), the light chain cDNA sequence (the encoding sequence of the variable region is set forth in SEQ ID NO: 5; the constant region was human Ig kappa chain C region) of H9L9(hG4WT), and the light chain cDNA sequence (the encoding sequence of the variable region is set forth in SEQ ID NO: 7; the constant region was human Ig kappa chain C region) of H9L10(hG4WT) were separately cloned into pUC57simple vectors (supplied by GenScript), and plasmids pUC57simple-H9, pUC57simple-L8, pUC57simple-L9, and pUC57simple-L10 were obtained, respectively. The plasmids pUC57simple-H9, pUC57simple-L8, pUC57simple-L9, and pUC57simple-L10 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture solution was separated by high-speed centrifugation, and the supernatant was concentrated and loaded onto a HiTrap MabSelect SuRe column. The protein was eluted in one step with an elution buffer. The target sample was isolated and buffer-exchanged into PBS.
Amino acid sequence of the heavy chain constant region of H9L8(hG4WT), H9L9(hG4WT), or H9L10(hG4WT): 
ASTKGPSVFPLAPCSRSTSESTAALGCLVKDYFPEPVTVSWNSGA
LTSGVHTFPAVLQSSGLYSLSSVVTVPSSSLGTKTYTCNVDHKPSN
TKVDKRVESKYGPPCPPCPAPEFLGGPSVFLFPPKPKDTLMISRTP
EVTCVVVDVSQEDPEVQFNWYVDGVEVHNAKTKPREEQFNSTY
RVVSVLTVLHQDWLNGKEYKCKVSNKGLPSSIEKTISKAKGQPR
EPQVYTLPPSQEEMTKNQVSLTCLVKGFYPSDIAVEWESNGQPE
NNYKTTPPVLDSDGSFFLYSRLTVDKSRWQEGNVFSCSVMHEAL
HNHYTQKSLSLSLGK (SEQ ID NO: 20)
Amino acid sequence of the light chain constant region of H9L8(hG4WT), H9L9(hG4WT), or H9L10(hG4WT):



 (SEQ ID NO: 19)

### 3. Expression and purification of humanized antibody H9L8(hG1WT)

On the basis of the variable region of the antibody H9L8(hG4WT) described above, the antibody H9L8(hG1WT)was obtained using Ig gamma-1 chain C region as the heavy chain constant region.
Heavy chain amino acid sequence of H9L8(hG1WT) 
QVQLQQWGAGLLKPSETLSLTCAVYGGSISDYYWNWIRQPPGK
GLEWIGEINYRGTTNSNPSLKSRVTLSLDTSKNQFSLKLRSVTAA
DTAVYYCAFGYSDYEYDWFDPWGQGTLVTVSSASTKGPSVFPLA
PSSKSTSGGTAALGCLVKDYFPEPVTVSWNSGALTSGVHTFPAVL
QSSGLYSLSSVVTVPSSSLGTQTYICNVNHKPSNTKVDKKVEPKSC
DKTHTCPPCPAPELLGGPSVFLFPPKPKDTLMISRTPEVTCVVVD
VSHEDPEVKFNWYVDGVEVHNAKTKPREEQYNSTYRVVSVLTVL
HQDWLNGKEYKCKVSNKALPAPIEKTISKAKGQPREPQVYTLPP
SRDELTKNQVSLTCLVKGFYPSDIAVEWESNGQPENNYKTTPPVL
DSDGSFFLYSKLTVDKSRWQQGNVFSCSVMHEALHNHYTQKSLS
LSPGK (SEQ ID NO: 38)
Light chain amino acid sequence of H9L8(hG1WT)

EIVLTQSPATLSLSPGERATLSCRASQTISSYLAWYQQKPGQAPRL
LIYDASNRATGIPARFSGSGSGTDFTLTISSLEPEDFAVYYCQQRS
NWPITFGQGTNLEIKRTVAAPSVFIFPPSDEQLKSGTASVVCLLNN
FYPREAKVQWKVDNALQSGNSQESVTEQDSKDSTYSLSSTLTLSK
ADYEKHKVYACEVTHQGLSSPVTKSFNRGEC (SEQ ID NO: 39)

### Preparation Example 2: Preparation of Human Anti-Hen Egg Lysozyme Antibody

The sequence of the human anti-hen egg lysozyme IgG (anti-HEL, or human IgG, abbreviated as hIgG) antibody was derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1): 130-46). The preparation method was as follows: Nanjing GenScript Biotech was entrusted to carry out codon optimization of amino acids and gene synthesis on heavy and light chain (complete sequence or variable region) genes of the human IgG antibody, and by referring to the standard technologies introduced in the Molecular Cloning: A Laboratory Manual (Third Edition) and using standard molecular cloning techniques such as PCR, enzyme digestion, DNA gel extraction, ligation transformation, colony PCR or enzyme digestion identification, the heavy and light chain genes were subcloned into the antibody heavy chain expression vector and antibody light chain expression vector of the mammalian expression system, respectively. The heavy and light chain genes of the recombinant expression vectors were further sequenced and analyzed. After the sequences were verified to be correct, a medium or large amount of endotoxin-free expression plasmids were prepared, and the heavy and light chain expression plasmids were transiently co-transfected into HEK293 cells for recombinant antibody expression. After 7 days of culture, the cell culture solutions were collected and subjected to affinity purification on a rProtein A column (GE), and the quality of the resulting antibody sample was determined using SDS-PAGE and SEC-HPLC standard analysis techniques.

Unless otherwise specified, hIgG1, hIgG1DM, and hIgG4WT used in the present disclosure were anti-HEL isotype control antibodies with hIgG1, hG1DM, and hG4WT constant region sequences, respectively, prepared in the laboratory of Akeso Biopharma Inc.

### Preparation Example 3: Design and Preparation of Anti-CD73 Antibodies

1. For the sequences and preparation methods of anti-CD73 antibodies 19F3, 19F3H1L1, 19F3H2L2, and 19F3H2L3,reference was made to Chinese Patent Application CN113527489A, and part of the sequences are cited as follows.

19F3, 19F3H1L1, 19F3H2L2, and 19F3H2L3 have identical 3 heavy chain CDRs and 3 light chain CDRs (according to the IMGT numbering system):
HCDR1: GYSFTGYT (SEQ ID NO: 25)
HCDR2: INPYNAGT (SEQ ID NO: 26)
HCDR3: ARSEYRYGGDYFDY (SEQ ID NO: 27)
LCDR1: QSLLNSSNQKNY (SEQ ID NO: 28)
LCDR2: FAS (SEQ ID NO: 29)
LCDR3: QQHYDTPYT (SEQ ID NO: 30)
Amino acid sequence of the heavy chain variable region of 19F3, with CDR sequences underlined: 
EVQLQQSGPELVKPGASMRMSCKASGYSFTGYTMNWVKQSHG
KNLEWIGLINPYNAGTSYNQKFKGKATLTVDKSSSTAYMELLSLT
SEDSAVYYCARSEYRYGGDYFDYWGQGTTLTVSS (SEQ ID NO: 31)
Amino acid sequence of the light chain variable region of 19F3, with CDR sequences underlined: 
DIVMTQSPSSLAMSVGQKVTMSCKSSQSLLNSSNQKNYLAWYQQ
KPGQSPKLLVYFASTRESGVPDRFIGSGSGTDFTLTISSVQAEDLA
DYFCQQHYDTPYTFGGGTKLEIK (SEQ ID NO: 32)
Amino acid sequence of the heavy chain variable region of 19F3H1L1, with CDR sequences underlined: 
QVQLQQSGAEVVKPGASMKMSCKASGYSFTGYTMNWVKQAHG
QNLEWIGLINPYNAGTSYNQKFQGKATLTVDKSTSTAYMELSSL
RSEDTAVYYCARSEYRYGGDYFDYWGQGTTLTVSS (SEQ ID NO:
33)
Amino acid sequence of the light chain variable region of 19F3H1L1, with CDR sequences underlined: 
DIVMTQSPSSLAMSVGERVTMSCKSSQSLLNSSNQKNYLAWYQQ
KPGQAPKLLVYFASTRESGVPDRFSGSGSGTDFTLTISSVQAEDLA
DYFCQQHYDTPYTFGGGTKLEIK (SEQ ID NO: 34)
Amino acid sequence of the heavy chain variable region of 19F3H2L2, with CDR sequences underlined: 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSS (SEQ ID NO: 35)
Amino acid sequence of the light chain variable region of 19F3H2L2, with CDR sequences underlined: 
DIVMTQSPSSLAVSVGERVTISCKSSQSLLNSSNQKNYLAWYQQK
PGQAPKLLIYFASTRESGVPDRFSGSGSGTDFTLTISSVQAEDVAD
YYCQQHYDTPYTFGGGTKLEIK (SEQ ID NO: 36)
Amino acid sequence of the heavy chain variable region of 19F3H2L3, with CDR sequences underlined: 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSS (SEQ ID NO: 35)
Amino acid sequence of the light chain variable region of 19F3H2L3, with **CDR** sequences underlined:

DIVMTQSPSSLAVSVGERVTISCKSSQSLLNSSNQKNYLAWYQQK
PGQAPKLLIYFASTRESGVPDRFSGSGSGTDFTLTISSLQAEDVAV
YYCQQHYDTPYTFGGGTKLEIK (SEQ ID NO: 37)

### 2. Preparation of humanized antibody 19F3H2L3

The heavy chain constant regions were all the Ig gamma-1 chain C region, and the light chain constant regions were all the Ig kappa chain C region. The heavy chain cDNA and the light chain cDNA of 19F3H2L3 were separately cloned into pUC57simple vectors (provided by GenScript) to obtain pUC57simple-19F3H2 and pUC57simple-19F3L3, respectively. Referring to the standard techniques described in Molecular Cloning: A Laboratory Manual (Third Edition), the heavy and light chain full-length genes synthesized by EcoRI&HindIII digestion were separately subcloned into expression vectors pcDNA3.1 through digestion by a restriction enzyme (EcoRI&HindIII) to obtain expression plasmids pcDNA3.1-19F3H2 and pcDNA3.1-19F3L3, and the heavy/light chain genes of the recombinant expression plasmids were further subjected to sequencing analysis. Then, the designed gene combination comprising the corresponding light and heavy chain recombinant plasmids (pcDNA3.1-19F3H2/pcDNA3.1-19F3L3) was co-transfected into 293F cells, and the culture solution was collected and purified. After the sequences were verified to be correct by sequencing, endotoxin-free expression plasmids were prepared and transiently transfected into HEK293 cells for antibody expression. After 7 days, the cell culture solution was collected and subjected to affinity purification on a Protein A column to obtain the humanized antibody.
Heavy chain amino acid sequence of 19F3H2L3 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSSASTKGPSVFPL
APSSKSTSGGTAALGCLVKDYFPEPVTVSWNSGALTSGVHTFPAV
LQSSGLYSLSSVVTVPSSSLGTQTYICNVNHKPSNTKVDKKVEPKS
CDKTHTCPPCPAPELLGGPSVFLFPPKPKDTLMISRTPEVTCVVV
DVSHEDPEVKFNWYVDGVEVHNAKTKPREEQYNSTYRVVSVLT
VLHQDWLNGKEYKCKVSNKALPAPIEKTISKAKGQPREPQVYTL
PPSRDELTKNQVSLTCLVKGFYPSDIAVEWESNGQPENNYKTTPP
VLDSDGSFFLYSKLTVDKSRWQQGNVFSCSVMHEALHNHYTQKS
LSLSPGK (SEQ ID NO: 40)
Light chain amino acid sequence of 19F3H2L3

DIVMTQSPSSLAVSVGERVTISCKSSQSLLNSSNQKNYLAWYQQK
PGQAPKLLIYFASTRESGVPDRFSGSGSGTDFTLTISSLQAEDVAV
YYCQQHYDTPYTFGGGTKLEIKRTVAAPSVFIFPPSDEQLKSGTA
SVVCLLNNFYPREAKVQWKVDNALQSGNSQESVTEQDSKDSTYS
LSSTLTLSKADYEKHKVYACEVTHQGLSSPVTKSFNRGEC (SEQ
ID NO: 41)

### 3. Preparation of humanized antibody 19F3H2L3(hG1DM)

The light chain constant region of the antibody 19F3H2L3(hG1DM) was the Ig kappa chain C region, as set forth in SEQ ID NO: 19.

On the basis of the heavy chain constant region Ig gamma-1 chain C region (SEQ ID NO: 18), a humanized antibody was obtained by introducing a leucine-to-alanine point mutation at position 234 (L234A) and a leucine-to-alanine point mutation at position 235 (L235A) and was designated as 19F3H2L3(hG1DM).

The heavy chain cDNA and the light chain cDNA of 19F3H2L3(hG1DM) were separately cloned into pUC57simple vectors (provided by GenScript) to obtain pUC57simple-19F3H2(hG1DM) and pUC57simple-19F3L3, respectively. Referring to the standard techniques described in Molecular Cloning: A Laboratory Manual (Third Edition), the heavy and light chain full-length genes synthesized by EcoRI&HindIII digestion were separately subcloned into expression vectors pcDNA3.1 through digestion by a restriction enzyme (EcoRI&HindIII) to obtain expression plasmids pcDNA3.1-19F3H2(hG1DM) and pcDNA3.1-19F3L3, and the heavy/light chain genes of the recombinant expression plasmids were further subjected to sequencing analysis. Then, the designed gene combination comprising the corresponding light and heavy chain recombinant plasmids (pcDNA3.1-19F3H2(hG1DM)/pcDNA3.1-19F3L3) was co-transfected into 293F cells, and the culture solution was collected and purified. After the sequences were verified to be correct by sequencing, endotoxin-free expression plasmids were prepared and transiently transfected into HEK293 cells for antibody expression. After 7 days, the cell culture solution was collected and subjected to affinity purification on a Protein A column to obtain the humanized antibody.
Full-length heavy chain of 19F3H2L3(hG1DM) 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSSASTKGPSVFPL
APSSKSTSGGTAALGCLVKDYFPEPVTVSWNSGALTSGVHTFPAV
LQSSGLYSLSSVVTVPSSSLGTQTYICNVNHKPSNTKVDKKVEPKS
CDKTHTCPPCPAPEAAGGPSVFLFPPKPKDTLMISRTPEVTCVVV
DVSHEDPEVKFNWYVDGVEVHNAKTKPREEQYNSTYRVVSVLT
 
VLHQDWLNGKEYKCKVSNKALPAPIEKTISKAKGQPREPQVYTL
PPSRDELTKNQVSLTCLVKGFYPSDIAVEWESNGQPENNYKTTPP
VLDSDGSFFLYSKLTVDKSRWQQGNVFSCSVMHEALHNHYTQKS
LSLSPGK (SEQ ID NO: 49)
Full-length light chain of 19F3H2L3(hG1DM)

DIVMTQSPSSLAVSVGERVTISCKSSQSLLNSSNQKNYLAWYQQK
PGQAPKLLIYFASTRESGVPDRFSGSGSGTDFTLTISSLQAEDVAV
YYCQQHYDTPYTFGGGTKLEIKRTVAAPSVFIFPPSDEQLKSGTA
SVVCLLNNFYPREAKVQWKVDNALQSGNSQESVTEQDSKDSTYS
LSSTLTLSKADYEKHKVYACEVTHQGLSSPVTKSFNRGEC (SEQ
ID NO: 50)

### Preparation Example 4: Design and Preparation of Anti-CD73-Anti-LAG3 Bispecific Antibodies

### 1. Sequence design

The structural formats of the bispecific antibodies NTLAV2, NTLAV7, NTLAV8, LASEV1, and LASEV2 of the present disclosure are in the Morrison format (IgG-scFv), i.e., C-termini of two heavy chains of one IgG antibody are each linked to the scFv fragment of another antibody, and the main composition design of the heavy and light chains is as shown in Table 1 below.

**Table 1. Composition design of the heavy and light chains of bispecific antibodies**

| **Bispecific antibody No.** | **Immunoglobulin portion** | | **Linker** | **scFv portion** | | |
|---|---|---|---|---|---|---|
| | **Heavy chain** | **Light chain** | | **Heavy chain variable region** | **Linker** | **Light chain variable region** |
| **NTLAV2** | **19FH2 (SEQ ID NO: 40)** | **19L3 (SEQ ID NO: 41)** | **(GGGGS)4 (SEQ ID NO: 43)** | **H9v(M) (SEQ ID NO: 44)** | **(GGGGS)4 (SEQ ID NO: 43)** | **L8v(M) (SEQ ID NO: 45)** |
| **NTLAV7** | **19FH2 (SEQ ID NO: 40)** | **19L3 (SEQ ID NO: 41)** | **(GGGGS)3 (SEQ ID NO: 42)** | **H9v (SEQ ID NO: 2)** | **(GGGGS)4 (SEQ ID NO: 43)** | **L8v (SEQ ID NO: 4)** |
| **NTLAV8** | **19FH2 (SEQ ID NO: 40)** | **19L3 (SEQ ID NO: 41)** | **(GGGGS)3 (SEQ ID NO: 42)** | **H9v(M) (SEQ ID NO: 44)** | **(GGGGS)4 (SEQ ID NO: 43)** | **L8v(M) (SEQ ID NO: 45)** |
| **LA5EV1** | **H9 (SEQ ID NO: 38)** | **L8 (SEQ ID NO: 39)** | **(GGGGS)4 (SEQ ID NO: 43)** | **19FH2v (SEQ ID NO: 35)** | **(GGGGS)4 (SEQ ID NO: 43)** | **19L3v(M2) (SEQ ID NO: 62)** |
| **LA5EV2** | **H9 (SEQ ID NO: 38)** | **L8 (SEQ ID NO: 39)** | **(GGGGS)4 (SEQ ID NO: 43)** | **19FH2v(M) (SEQ ID NO: 46)** | **(GGGGS)4 (SEQ ID NO: 43)** | **19L3v(M1) (SEQ ID NO: 47)** |

In Table 1 above:
(1) Amino acid sequence of linker (GGGGS)3:
   GGGGSGGGGSGGGGS (SEQ ID NO: 42)
(2) Amino acid sequence of linker (GGGGS)4:
   GGGGSGGGGSGGGGSGGGGS (SEQ ID NO: 43)

Those with "v" label at lower right corner refer to the variable region of corresponding heavy chain or the variable region of corresponding light chain. For those without "v" label, the corresponding heavy or light chain is the full length comprising the constant region. Unless otherwise specified, for all the amino acid sequences of these variable regions or the full lengths and the nucleotide sequences encoding them, reference was made to the corresponding sequences described in the above preparation examples.

In Table 1 described above, H9ᵥ(M), L8ᵥ(M), 19FH2ᵥ(M), 19L3ᵥ(M1), and 19L3ᵥ(M2) in the scFv fragments of the antibodies were generated by introducing specific amino acid mutations into the corresponding framework regions of H9ᵥ, L8ᵥ, 19FH2ᵥ, and 19L3ᵥ, and thus the structures of the antibodies were effectively optimized and the effectiveness of the antibodies was enhanced.
Amino acid sequence of H9ᵥ(M) 
QVQLQQWGAGLLKPSETLSLTCAVYGGSISDYYWNWIRQPPGKCLE
WIGEINYRGTTNSNPSLKSRVTLSLDTSKNQFSLKLRSVTAADTAVYY
CAFGYSDYEYDWFDPWGQGTLVTVSS (SEQ ID NO: 44)
Amino acid sequence of L8ᵥ(M) 
EIVLTQSPATLSLSPGERATLSCRASQTISSYLAWYQQKPGQAPRLLIY
DASNRATGIPARFSGSGSGTDFTLTISSLEPEDFAVYYCQQRSNWPITF
GCGTNLEIK (SEQ ID NO: 45)
Amino acid sequence of 19FH2ᵥ(M) 
QVQLVQSGAEVVKPGASVKVSCKASGYSFTGYTMNWVRQAPGQCLE
WIGLINPYNAGTSYNQKFQGKVTLTVDKSTSTAYMELSSLRSEDTAVY
YCARSEYRYGGDYFDYWGQGTTLTVSS (SEQ ID NO: 46)
Amino acid sequence of 19L3ᵥ(M1) 
DIVMTQSPSSLAVSVGERVTISCKSSQSLLNSSNQKNYLAWYQQK
PGQAPKLLIYFASTRESGVPDRFSGSGSGTDFTLTISSLQAEDVAV
YYCQQHYDTPYTFGCGTKLEIKR (SEQ ID NO: 47)
Amino acid sequence of 19L3ᵥ(M2)

DIVMTQSPSSLAVSVGERVTISCKSSQSLLNSSNQKNYLAWYQQK
PGQAPKLLIYFASTRESGVPDRFSGSGSGTDFTLTISSLQAEDVAV
YYCQQHYDTPYTFGGGTKLEIKR (SEQ ID NO: 62)

On the basis of the bispecific antibodies designed in Table 1 described above, by introducing a leucine-to-alanine point mutation at position 234 (L234A), a leucine-to-alanine point mutation at position 235 (L235A), and a glycine-to-alanine point mutation at position 237 (G237A) in the heavy chains, bispecific antibodies comprising the above mutation sites were obtained and were designated as NTLAV2(hG1TM), NTLAV7(hG1TM), NTLAV8(hG1TM), LASEV1(hG1TM), and LASEV2(hG1TM).
Heavy chain amino acid sequence of NTLAV2(hG1TM) 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSSASTKGPSVFPL
 
APSSKSTSGGTAALGCLVKDYFPEPVTVSWNSGALTSGVHTFPAV
LQSSGLYSLSSVVTVPSSSLGTQTYICNVNHKPSNTKVDKKVEPKS
CDKTHTCPPCPAPEAAGAPSVFLFPPKPKDTLMISRTPEVTCVVV
DVSHEDPEVKFNWYVDGVEVHNAKTKPREEQYNSTYRVVSVLT
VLHQDWLNGKEYKCKVSNKALPAPIEKTISKAKGQPREPQVYTL
PPSRDELTKNQVSLTCLVKGFYPSDIAVEWESNGQPENNYKTTPP
VLDSDGSFFLYSKLTVDKSRWQQGNVFSCSVMHEALHNHYTQKS
LSLSPGKGGGGSGGGGSGGGGSGGGGSQVQLQQWGAGLLKPSE
TLSLTCAVYGGSISDYYWNWIRQPPGKCLEWIGEINYRGTTNSNP
SLKSRVTLSLDTSKNQFSLKLRSVTAADTAVYYCAFGYSDYEYD
WFDPWGQGTLVTVSSGGGGSGGGGSGGGGSGGGGSEIVLTQSP
ATLSLSPGERATLSCRASQTISSYLAWYQQKPGQAPRLLIYDASN
RATGIPARFSGSGSGTDFTLTISSLEPEDFAVYYCQQRSNWPITFG
CGTNLEIK (SEQ ID NO: 51)
Heavy chain amino acid sequence of NTLAV7(hG1TM) 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSSASTKGPSVFPL
APSSKSTSGGTAALGCLVKDYFPEPVTVSWNSGALTSGVHTFPAV
LQSSGLYSLSSVVTVPSSSLGTQTYICNVNHKPSNTKVDKKVEPKS
CDKTHTCPPCPAPEAAGAPSVFLFPPKPKDTLMISRTPEVTCVVV
DVSHEDPEVKFNWYVDGVEVHNAKTKPREEQYNSTYRVVSVLT
VLHQDWLNGKEYKCKVSNKALPAPIEKTISKAKGQPREPQVYTL
PPSRDELTKNQVSLTCLVKGFYPSDIAVEWESNGQPENNYKTTPP
VLDSDGSFFLYSKLTVDKSRWQQGNVFSCSVMHEALHNHYTQKS
LSLSPGKGGGGSGGGGSGGGGSQVQLQQWGAGLLKPSETLSLT
CAVYGGSISDYYWNWIRQPPGKGLEWIGEINYRGTTNSNPSLKSR
 
VTLSLDTSKNQFSLKLRSVTAADTAVYYCAFGYSDYEYDWFDPW
GQGTLVTVSSGGGGSGGGGSGGGGSGGGGSEIVLTQSPATLSLSP
GERATLSCRASQTISSYLAWYQQKPGQAPRLLIYDASNRATGIPA
RFSGSGSGTDFTLTISSLEPEDFAVYYCQQRSNWPITFGQGTNLEI
K (SEQ ID NO: 52)
Heavy chain amino acid sequence of NTLAV8(hG1TM) 
QVQLVQSGAEVVKPGASVKVSCKASGYSFTGYTMNWVRQAPGQ
NLEWIGLINPYNAGTSYNQKFQGKVTLTVDKSTSTAYMELSSLRS
EDTAVYYCARSEYRYGGDYFDYWGQGTTLTVSSASTKGPSVFPL
APSSKSTSGGTAALGCLVKDYFPEPVTVSWNSGALTSGVHTFPAV
LQSSGLYSLSSVVTVPSSSLGTQTYICNVNHKPSNTKVDKKVEPKS
CDKTHTCPPCPAPEAAGAPSVFLFPPKPKDTLMISRTPEVTCVVV
DVSHEDPEVKFNWYVDGVEVHNAKTKPREEQYNSTYRVVSVLT
VLHQDWLNGKEYKCKVSNKALPAPIEKTISKAKGQPREPQVYTL
PPSRDELTKNQVSLTCLVKGFYPSDIAVEWESNGQPENNYKTTPP
VLDSDGSFFLYSKLTVDKSRWQQGNVFSCSVMHEALHNHYTQKS
LSLSPGKGGGGSGGGGSGGGGSQVQLQQWGAGLLKPSETLSLT
CAVYGGSISDYYWNWIRQPPGKCLEWIGEINYRGTTNSNPSLKSR
VTLSLDTSKNQFSLKLRSVTAADTAVYYCAFGYSDYEYDWFDPW
GQGTLVTVSSGGGGSGGGGSGGGGSGGGGSEIVLTQSPATLSLSP
GERATLSCRASQTISSYLAWYQQKPGQAPRLLIYDASNRATGIPA
RFSGSGSGTDFTLTISSLEPEDFAVYYCQQRSNWPITFGCGTNLEI
K (SEQ ID NO: 53)
Heavy chain amino acid sequence of LASEV1(hG1TM) 
QVQLQQWGAGLLKPSETLSLTCAVYGGSISDYYWNWIRQPPGK
GLEWIGEINYRGTTNSNPSLKSRVTLSLDTSKNQFSLKLRSVTAA
 
DTAVYYCAFGYSDYEYDWFDPWGQGTLVTVSSASTKGPSVFPLA
PSSKSTSGGTAALGCLVKDYFPEPVTVSWNSGALTSGVHTFPAVL
QSSGLYSLSSVVTVPSSSLGTQTYICNVNHKPSNTKVDKKVEPKSC
DKTHTCPPCPAPEAAGAPSVFLFPPKPKDTLMISRTPEVTCVVVD
VSHEDPEVKFNWYVDGVEVHNAKTKPREEQYNSTYRVVSVLTVL
HQDWLNGKEYKCKVSNKALPAPIEKTISKAKGQPREPQVYTLPP
SRDELTKNQVSLTCLVKGFYPSDIAVEWESNGQPENNYKTTPPVL
DSDGSFFLYSKLTVDKSRWQQGNVFSCSVMHEALHNHYTQKSLS
LSPGKGGGGSGGGGSGGGGSGGGGSQVQLVQSGAEVVKPGASV
KVSCKASGYSFTGYTMNWVRQAPGQNLEWIGLINPYNAGTSYN
QKFQGKVTLTVDKSTSTAYMELSSLRSEDTAVYYCARSEYRYGG
DYFDYWGQGTTLTVSSGGGGSGGGGSGGGGSGGGGSDIVMTQS
PSSLAVSVGERVTISCKSSQSLLNSSNQKNYLAWYQQKPGQAPKL
LIYFASTRESGVPDRFSGSGSGTDFTLTISSLQAEDVAVYYCQQHY
DTPYTFGGGTKLEIKR (SEQ ID NO: 54)
Heavy chain amino acid sequence of LASEV2(hG1TM)

QVQLQQWGAGLLKPSETLSLTCAVYGGSISDYYWNWIRQPPGK
GLEWIGEINYRGTTNSNPSLKSRVTLSLDTSKNQFSLKLRSVTAA
DTAVYYCAFGYSDYEYDWFDPWGQGTLVTVSSASTKGPSVFPLA
PSSKSTSGGTAALGCLVKDYFPEPVTVSWNSGALTSGVHTFPAVL
QSSGLYSLSSVVTVPSSSLGTQTYICNVNHKPSNTKVDKKVEPKSC
DKTHTCPPCPAPEAAGAPSVFLFPPKPKDTLMISRTPEVTCVVVD
VSHEDPEVKFNWYVDGVEVHNAKTKPREEQYNSTYRVVSVLTVL
HQDWLNGKEYKCKVSNKALPAPIEKTISKAKGQPREPQVYTLPP
SRDELTKNQVSLTCLVKGFYPSDIAVEWESNGQPENNYKTTPPVL
DSDGSFFLYSKLTVDKSRWQQGNVFSCSVMHEALHNHYTQKSLS
LSPGKGGGGSGGGGSGGGGSGGGGSQVQLVQSGAEVVKPGASV

KVSCKASGYSFTGYTMNWVRQAPGQCLEWIGLINPYNAGTSYN
QKFQGKVTLTVDKSTSTAYMELSSLRSEDTAVYYCARSEYRYGG
DYFDYWGQGTTLTVSSGGGGSGGGGSGGGGSGGGGSDIVMTQS
PSSLAVSVGERVTISCKSSQSLLNSSNQKNYLAWYQQKPGQAPKL
LIYFASTRESGVPDRFSGSGSGTDFTLTISSLQAEDVAVYYCQQHY
DTPYTFGCGTKLEIKR (SEQ ID NO: 55)

Unless otherwise specified, in the subsequent experiments and examples, the bispecific antibodies NTLAV2(hG1TM), NTLAV7(hG1TM), NTLAV8(hG1TM), LASEV1(hG1TM), and LASEV2(hG1TM) carrying mutation sites described above are referred to as NTLAV2, NTLAV7, NTLAV8, LASEV1, and LASEV2, respectively.

### 2. Expression and purification of antibodies

The heavy chain cDNA sequences and the light chain cDNA sequences of NTLAV2, NTLAV7, NTLAV8, LASEV1, and LA5EV2 were separately cloned into pUC57simple vectors (provided by GenScript) to obtain plasmids pUC57simple-NTLAV2H/pUC57simple-NTLAV2L, pUC57simple-NTLAV7H/pUC57simple-NTLAV7L, pUC57simple-NTLAV8H/pUC57simple-NTLAV8L, pUC57simple-LA5EV1H/pUC57simple-LA5EV1L, and pUC57simple-LA5EV2H/pUC57simple-LA5EV2H, respectively. Plasmids pUC57simple-NTLAV2H/pUC57simple-NTLAV2L, pUC57simple-NTLAV7H/pUC57simple-NTLAV7L, pUC57simple-NTLAV8H/pUC57simple-NTLAV8L, pUC57simple-LA5EV1H/pUC57simple-LA5EV1L, and pUC57simple-LA5EV2H/pUC57simple-LA5EV2H were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture solution was separated by high-speed centrifugation, and the supernatant was concentrated and loaded onto a HiTrap MabSelect SuRe column. The protein was eluted in one step with an elution buffer. The target sample was isolated and buffer-exchanged into PBS.

### Example 1: Assays for Binding Activity of Anti-LAG3 Antibodies for Antigen by ELISA

An ELISA plate was coated with 2 µg/mL human LAG3-mG1Fc and incubated overnight at 4 °C. Then, the antigen-coated ELISA plate was washed once with PBST and blocked with a PBS solution containing 1% BSA (blocking solution) at 37 °C for 2 h. After blocking, the ELISA plate was washed 3 times with PBST. The antibodies serially diluted with a PBST solution (the dilution gradients for the antibodies are shown in Table 2) were added. The ELISA plate containing the test antibodies was incubated at 37 °C for 30 min and then washed 3 times with PBST. After washing, an HRP-labeled goat anti-human IgG (H+L) (Jackson, Cat. No. 109-035-088) secondary antibody working solution diluted at a ratio of 1:5000 was added, and the plate was then incubated at 37 °C for 30 min. After the incubation, the plate was washed 4 times with PBST. Color development was then performed with TMB (Neogen, 308177) in the dark for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

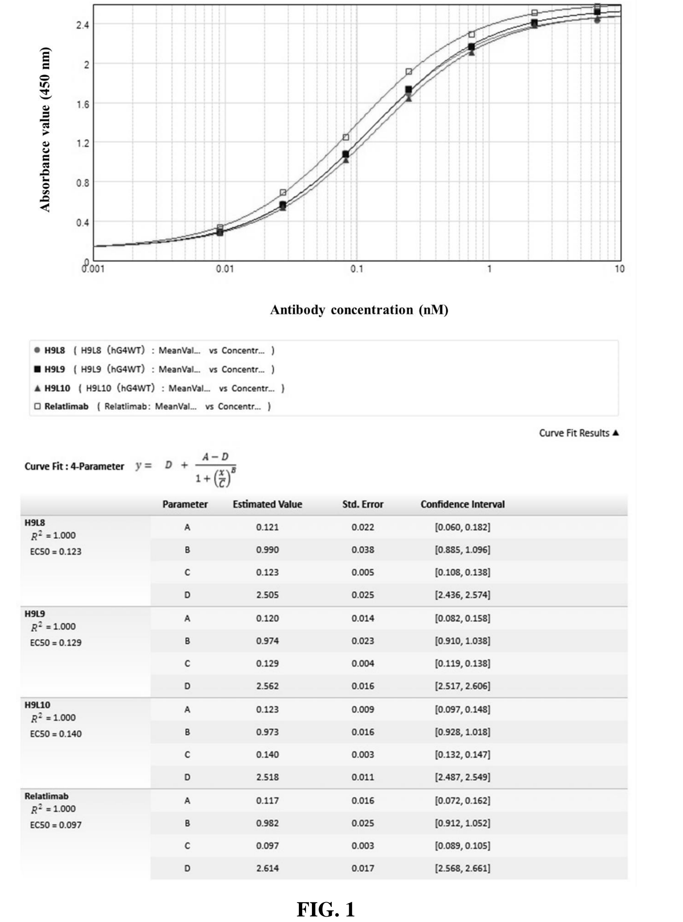

The assay results are shown in Table 2 and FIG. 1.

**Table 2. Results of assays for the binding of H9L8(hG4WT), H9L9(hG4WT), and H9L10(hG4WT) to antigen human LAG3-mG1Fc by ELISA**

| | **Human LAG3-mG1Fc, 2 µg/mL, 50 µL/well** | | | | | | | |
|---|---|---|---|---|---|---|---|---|
| **Antibody dilution (µg/mL)** | **H9L8 (hG4WT)** | | **H9L9 (hG4WT)** | | **H9L10 (hG4WT)** | | **Relatlimab** | |
| **1** | **2.403** | **2.463** | **2.485** | **2.555** | **2.438** | **2.471** | **2.568** | **2.589** |
| **0.3** | **2.414** | **2.399** | **2.420** | **2.412** | **2.345** | **2.419** | **2.468** | **2.557** |
| **0.1** | **2.201** | **2.141** | **2.200** | **2.135** | **2.121** | **2.107** | **2.260** | **2.327** |
| **0.03** | **1.734** | **1.647** | **1.783** | **1.691** | **1.637** | **1.657** | **1.868** | **1.975** |
| **0.01** | **1.104** | **1.065** | **1.089** | **1.054** | **1.019** | **1.018** | **1.220** | **1.288** |
| **0.003** | **0.592** | **0.558** | **0.567** | **0.560** | **0.525** | **0.540** | **0.649** | **0.725** |
| **0.001** | **0.282** | **0.277** | **0.298** | **0.287** | **0.275** | **0.282** | **0.324** | **0.344** |
| **0** | **0.122** | **0.123** | **0.125** | **0.118** | **0.121** | **0.126** | **0.119** | **0.121** |
| **Secondary antibody** | **Goat Anti Human IgG(H+L), HRP(1:5000)** | | | | | | | |
| **EC₅₀(nM)** | **0.1 23** | | **0.1 29** | | **0.1 40** | | **0.0 97** | |

The results show that: the antibodies H9L8(hG4WT), H9L9(hG4WT), and H9L10(hG4WT) all could effectively bind to antigen human LAG3-mG1Fc in a dose-dependent manner, and exhibited a binding activity comparable to that of the positive control antibody Relatlimab.

### Example 2: Assays for Binding Activity of Anti-LAG3 antibodies for Antigen Human LAG3 on Cell Surface by Flow Cytometry

Antibody labeling and flow cytometer detection: The 293T-LAG3 cells expressing antigen human LAG3 were digested with conventional pancreatin, and the number of cells in each collection tube was made to be 3 × 10⁵. LAG3 antibody dilutions prepared using 1% PBSA (PBS containing 1% BSA) at final concentrations of 0.0123 nM, 0.123 nM, 1.23 nM, 3.7 nM, 11.1 nM, 33.3 nM, 100 nM, and 300 nM, respectively, were each incubated with the 293T-LAG3 cells expressing LAG3 on ice for 1 h. After centrifugation and washing several times with 1% PBSA, 100 µL of FITC goat anti-human IgG (purchased from Jackson, Cat. No. 109-095-098) (diluted at a ratio of 1:300) was added to each tube, and the mixture was incubated on ice in the dark for 40 min. After washing once with 1% PBSA, 200 µL of 1% PBSA was added to resuspend the cells. Fluorescence signals were detected with FITC channel on a flow cytometer.

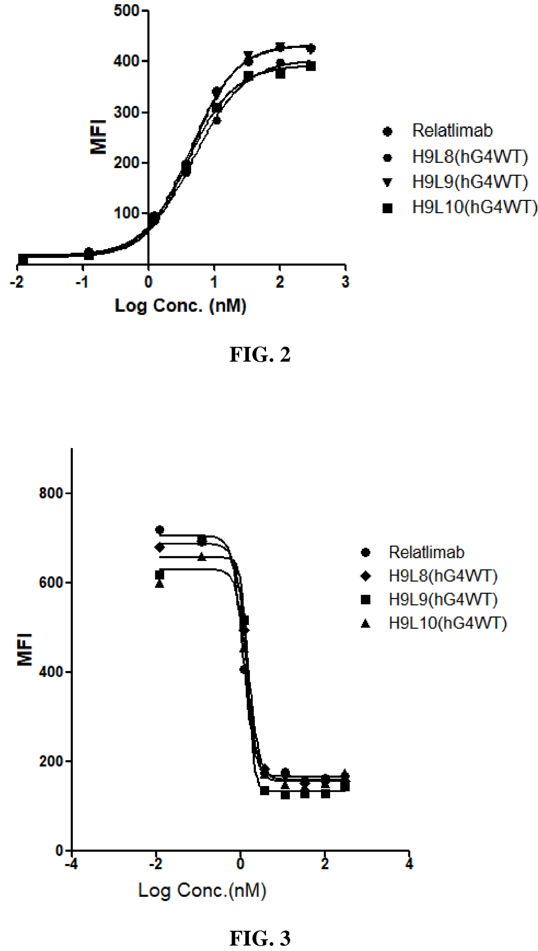

The results of the binding of humanized anti-LAG3 antibodies to 293T-LAG3 cells are shown in FIG. 2. The EC₅₀ values for the binding of the anti-LAG3 antibodies to the antigen on 293T-LAG3 cell surface are shown in Table 3.

**Table 3. Results of assays for the binding activity of anti-LAG3 antibodies for antigen on 293T-LAG3 cell surface by flow cytometer**

| **Antibody** | **EC₅₀ (nM)** |
|---|---|
| **Relatlimab** | **4.289** |
| **H9L8(hG4WT)** | **4.862** |
| **H9L9(hG4WT)** | **4.525** |
| **HIL10(hG4WT)** | **3.925** |

As can be seen from FIG. 2, the anti-LAG3 antibodies could effectively bind to the target LAG3 protein on 293T-LAG3 cell surface, and the binding activity of the anti-LAG3 antibodies H9L8(hG4WT), H9L9(hG4WT), and H9L10(hG4WT) for the antigen on 293T-LAG3 cell surface was comparable to that of the positive control antibody Relatlimab.

### Example 3: Assays for Anti-LAG3 Antibodies Competing with MHC II on Raji Cell Membrane Surface for Binding to Human LAG3-mG1Fc Antigen by Competitive Flow Cytometry

Raji cells (medium: 1640 + 10% FBS) (Cell Resource Center, Shanghai Institutes for Biological Sciences, Chinese Academy of Sciences, Cat. No. TCHu 44) were added to EP tubes at 300,000 cells per sample. 1000 µL of 1% PBSA (PBS containing 1% BSA) was added. The mixture was centrifuged at 600× g for 5 min, and the supernatant was discarded. 100 µL of hIgG1 (prepared by Akeso Biopharma Inc., Batch No. 20190410) at a final concentration of 300 nM was added to each tube, and the mixture was incubated on ice for 1 h; 200 µL of 1% PBSA was added to the Raji cells after incubation, and the mixture was centrifuged at 600× g for 5 min, followed by removal of the supernatant. According to the experimental design, diluted antibodies at corresponding concentrations were added to additional clean EP tubes at 60 µL/tube. 60 µL of human LAG3-mG1Fc (prepared by Akeso Biopharma Inc., Batch No. 20190508) was added to each corresponding antibody tube, and the mixture was mixed well and pre-incubated on ice for 30 min, such that the final concentrations of the antibodies were 300 nM, 100 nM, 33.3 nM, 11.1 nM, 3.7 nM, 1.23 nM, 0.123 nM, and 0.0123 nM. The final concentration of human LAG3-mG1Fc was 3 nM. 100 µL of the pre-incubated mixture of antibody and protein was added to the cells. The resulting mixture was mixed well and incubated on ice in the dark for 1 h; 200 µL of 1% PBSA was added, and the mixture was centrifuged at 600× g for 5 min, followed by removal of the supernatant, and then the pellet was washed twice; 100 µL of an APC anti mouse antibody (purchased from Biolegend, Cat. No. 405308) (diluted at a ratio of 1:400) was added, and the mixture was mixed well and incubated on ice in the dark for 40 min; 200 µL of 1% PBSA was added, and the mixture was centrifuged at 600× g for 5 min, followed by removal of the supernatant; 200 µL of 1% PBSA was added to each tube to resuspend the cells, and then the suspension was transferred to a sample loading tube for assay on a flow cytometer.

The results are shown in FIG. 3 and Table 4. By fluorescence analysis and curve fitting, the EC₅₀ values for the competitive binding of the antibodies Relatlimab, H9L8(hG4WT), H9L9(hG4WT), and H9L10(hG4WT) were calculated to be 1.153 nM, 1.459 nM, 1.482 nM, and 1.435 nM, respectively.

**Table 4. Results of analysis for the fluorescence intensities of Relatlimab, H9L8(hG4WT), H9L9(hG4WT), and H9L10(hG4WT) in competing with MHC II on Raji cell surface for binding to the human LAG3-mG1Fc antigen by FACS**

| **Antibody** | **EC₅₀ (nM)** |
|---|---|
| **Relatlimab** | **1.153** |
| **H9L8(hG4WT)** | **1.459** |
| **H9L9(hG4WT)** | **1.482** |
| **H9L10(hG4WT)** | **1.435** |

The results show that the antibodies H9L8(hG4WT), H9L9(hG4WT), and H9L10(hG4WT) could competitively bind to LAG3 and effectively block the binding of LAG3 to MHC II on Raji cell surface in a dose-dependent manner, and exhibited an activity comparable to that of the positive control antibody Relatlimab.

### Example 4: Assays for Biological Activity of Anti-LAG3 Antibodies in Promoting IFN-γ and IL-2 Secretion by Mixed Lymphocyte Reaction (MLR)

### 1. Assays for biological activity of anti-LAG3 antibodies in promoting IFN-y secretion in Raji-PDL1 mixed lymphocyte reaction system

Raji-PDL1 cells were conventionally subcultured. PBMCs (from healthy donors) were thawed, cultured in 10 mL of a 1640 complete medium, and stimulated with SEB (Staphylococcal enterotoxin B) (Dianotech, Cat. No. S010201) at 0.5 µg/mL for two days. The Raji-PDL1 cells were treated with MMC (Stressmarq, Cat. No. SIH-246-10MG) at a working concentration of 2 µg/mL and incubated at 37 °C in a 5% CO₂ incubator for 1 h; the PBMCs stimulated with SEB for 2 days and the Raji-PDL1 cells treated with MMC for 1 h were collected, washed twice with PBS, then resuspended in a complete medium (i.e., RPMI 1640 + 10% FBS), and counted. The PBMCs and Raji-PDL1 cells were separately added to a U-shaped 96-well plate (Corning, Model No. 3799) at 10 × 10⁴ cells/well and co-cultured. According to the experimental design, the antibodies (the final concentrations of each antibody were 300 nM, 30 nM, and 3 nM when used alone or in combination) were added and co-cultured with the cells in an incubator for 3 days; after 3 days, the cells were centrifuged at 250× g for 5 min, and the cell culture supernatant was collected and assayed for IFN-γ by ELISA.

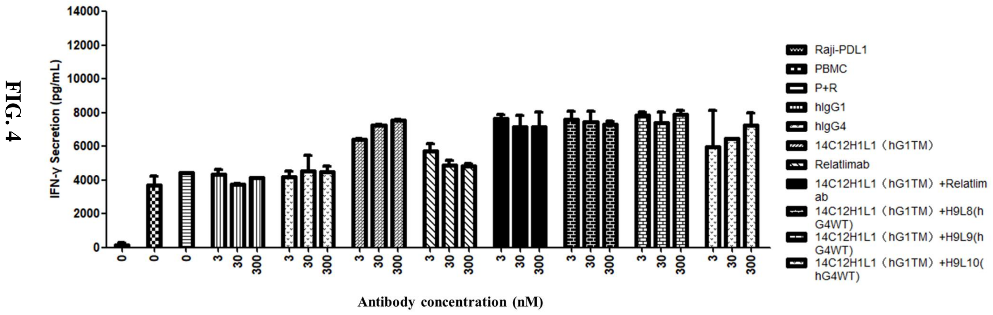

As shown in FIG. 4, the mixed culture of human PBMCs and Raji-PDL1 cells promoted the secretion of IFN-γ in PBMCs, and the addition of the antibodies to the mixed culture system could significantly induce the further secretion of IFN-γ in PBMCs. In terms of the level of activity in promoting IFN-γ secretion, the anti-LAG3 antibodies H9L8(hG4WT), H9L9(hG4WT), and H9L10(hG4WT) each in combination with 14C12H1L1(hG1TM), and the positive control antibody Relatlimab in combination with 14C12H1L1(hG1TM) all could promote IFN-γ secretion, with comparable activities.

### 2. Assays for biological activity of anti-LAG3 antibodies in promoting IL-2 secretion in Raji-PDL1 mixed lymphocyte reaction system

Raji-PDL1 cells were conventionally subcultured. PBMCs were thawed, cultured in 10 mL of a 1640 complete medium, and stimulated with SEB (Staphylococcal enterotoxin B, purchased from Dianotech, Cat. No. S010201) at 0.5 µg/mL for two days. The Raji-PDL1 cells were treated with MMC (Stressmarq, Cat. No. SIH-246-10MG) at a working concentration of 2 µg/mL and incubated at 37 °C in a 5% CO₂ incubator for 1 h. The PBMCs stimulated with SEB for 2 days and the Raji-PDL1 cells treated with MMC for 1 h were collected, washed twice with PBS, then resuspended in a complete medium (i.e., RPMI 1640 + 10% FBS), and counted. The PBMCs and Raji-PDL1 cells were separately added to a U-shaped 96-well plate (Corning, Model No. 3799) at 10x10⁴ cells/well and co-cultured. According to the experimental design, the antibodies (the final concentrations of each antibody were 300 nM, 30 nM, and 3 nM when used alone or in combination) were added and co-cultured with the cells for 3 days; after 3 days, the cells were centrifuged at 250× g for 5 min, and the cell culture supernatant was collected and assayed for IL-2 by ELISA.

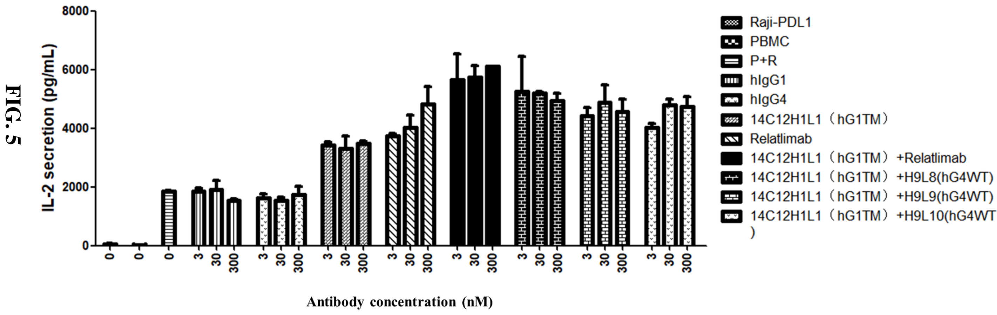

As shown in FIG. 5, the mixed culture of human PBMCs (from healthy donors) and Raji-PDL1 cells promoted the secretion of IL-2 in PBMCs to some extent, and the addition of the antibodies to the mixed culture system could significantly induce the further secretion of IL-2 in PBMCs, exhibiting a significant dose-dependent relationship. In terms of the level of activity in promoting IL-2 secretion, the anti-LAG3 antibodies H9L8(hG4WT), H9L9(hG4WT), and H9L10(hG4WT) each in combination with 14C12H1L1(hG1TM), and the positive control antibody Relatlimab in combination with 14C12H1L1(hG1TM) all could promote IL-2 secretion, with comparable activities.

### Example 5: Assays for Biological Activity Evaluation of Anti-LAG3 Antibodies in Blocking Interaction between LAG3 and MHC-II (Reporter Gene Method)

Jurkat-NFAT-PD1-LAG3 cells and Raji cells were used as a reporter gene system. After a superantigen SEE was added, a TCR-NFAT signaling pathway was activated to induce the expression of luciferase. The LAG3 on the Jurkat cells bound to the MHC-II on the Raji cells, such that the NFAT signaling pathway was inhibited, and the expression of luciferase was down-regulated. The antibody, by specifically binding to LAG3, relieved the inhibition and up-regulated the expression of luciferase.

Jurkat-NFAT-PD1-LAG3 cells and Raji cells (purchased from the Cell Resource Center, Shanghai Institutes for Biological Sciences, Chinese Academy of Sciences, Cat. No. TCHu 44) were collected and centrifuged at 110x g for 5 min, followed by removal of the supernatant. The cells were then resuspended in a 1640 + 10% FBS medium and counted. The Jurkat-NFAT-PD1-LAG3 cells were seeded into a black-bottom 96-well plate (Corning, Model No. 3916) at 10⁵ cells/well (30 µL/well); according to the experimental design, antibodies (at final concentrations of 0.3 nM, 3 nM, and 300 nM) were added at 10 µL/well, and the mixture was pre-incubated at 37 °C in a 5% CO₂ incubator for 30 min. Meanwhile, SEE (Staphylococcal Enterotoxins E, purchased from Toxin Technology, Cat. No. ET404) (at a final concentration of 0.05 ng/mL) was added to the Raji cells, and the mixture was incubated at 37 °C in a 5% CO₂ incubator for 30 min. After 30 min, the SEE-treated Raji cells were added to the 96-well plate containing Jurkat-NFAT-PD1-LAG3 cells described above at 2 × 10⁴ cells/well (40 µL/well), with the final volume of each well being 80 µL. The mixture was mixed well and incubated at 37 °C in a 5% CO₂ incubator for 6 h. After incubation, the culture plate was taken out and allowed to equilibrate to room temperature. Bright-Glo^{™}Luciferase Assay System (purchased from Promega, Cat. No. E2650) was added at 80 µL/well, and the mixture was incubated in the dark for 2 min. Then the RLU values were read. The isotype control hG4WT(hIgG4) was self-made by Akeso Biopharma Inc., with Batch No. 20190910.

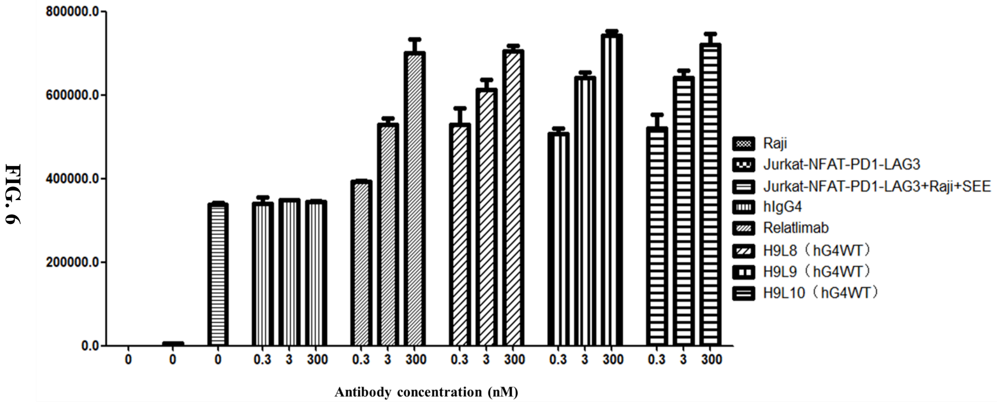

As shown in FIG. 6, the anti-LAG3 antibodies H9L8(hG4WT), H9L9(hG4WT), H9L10(hG4WT), and the positive control antibody Relatlimab all could block the interaction between LAG3 and MHC-II and thereby up-regulate the expression of luciferase, and the activities of the anti-LAG3 antibodies H9L8(hG4WT), H9L9(hG4WT), and H9L10(hG4WT) were all superior to that of the control antibody Relatlimab.

### Example 6: Assays for Binding Activity of Anti-CD73-Anti-LAG3 Bispecific Antibodies for Antigen by ELISA

1. Assays for binding activity of LASEV1, LASEV2, NTLAV2, NTLAV7, and NTLAV8 for antigen NT5E-His by indirect ELISA The specific method was as follows:
   An ELISA plate was coated with 1 µg/mL NT5E-His and incubated overnight at 4 °C. Then, the antigen-coated ELISA plate was washed once with PBST and blocked with a PBS solution containing 1% BSA (blocking solution) at 37 °C for 2 h. After blocking, the ELISA plate was washed 3 times with PBST. The antibodies serially diluted with a PBST solution (the dilution gradients for the antibodies are shown in Tables 5-7) were added. The ELISA plate containing the test antibodies was incubated at 37 °C for 30 min and then washed 3 times with PBST. After washing, an HRP-labeled goat anti-human IgG FC (H+L) (Jackson, Cat. No. 109-035-098) secondary antibody working solution diluted at a ratio of 1:5000 was added, and the plate was then incubated at 37 °C for 30 min. After the incubation, the plate was washed 4 times with PBST. Color development was then performed with TMB (Neogen, 308177) in the dark for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

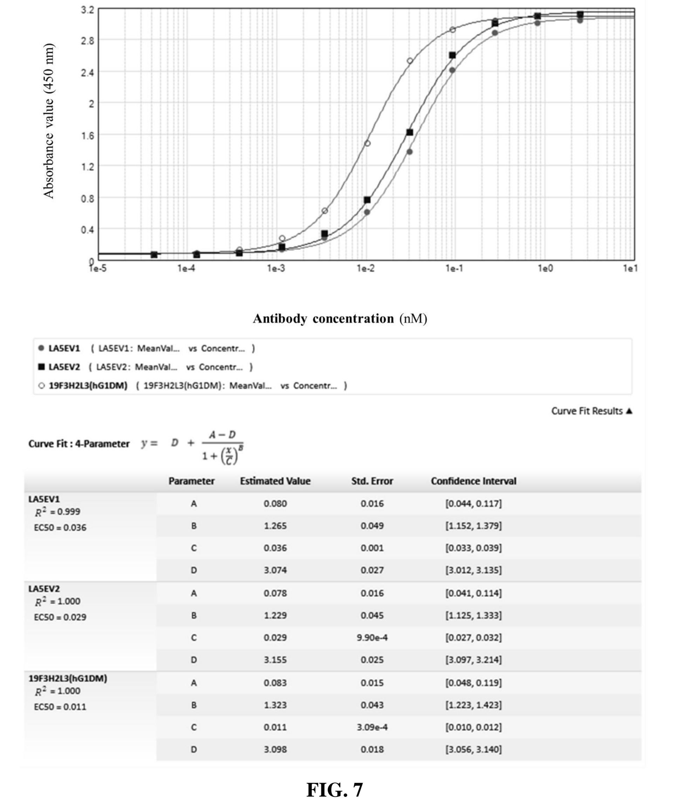

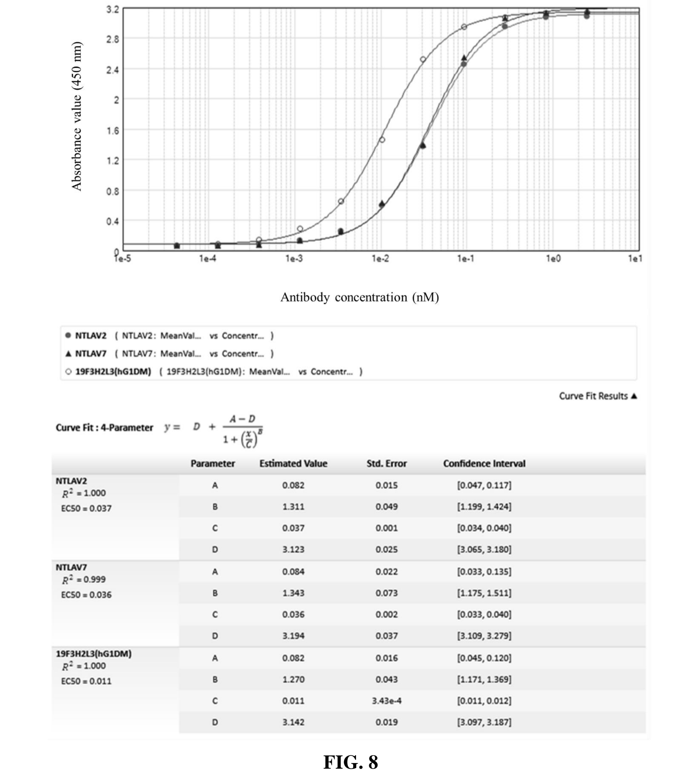

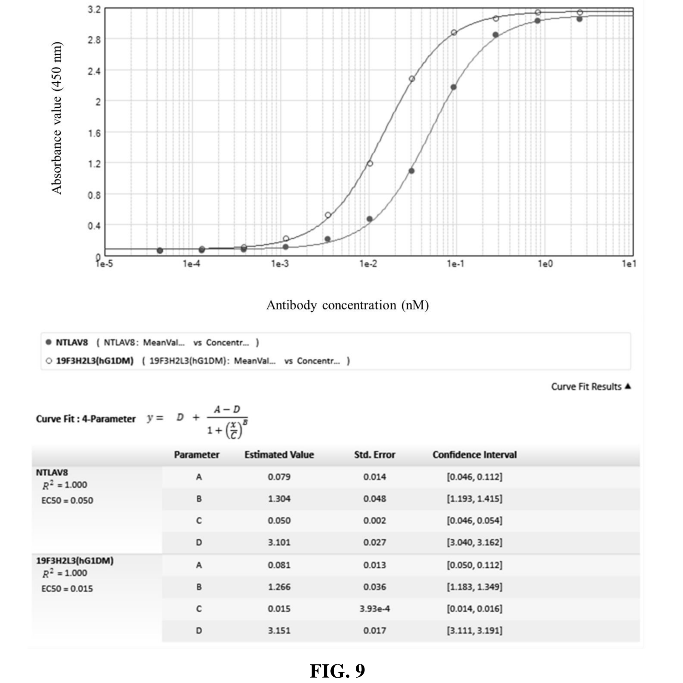

The results are shown in Tables 5-7 and FIGs. 7-9.

The results indicate that under the same experimental conditions, LA5EV1, LASEV2, NTLAV2, NTLAV7, NTLAV8, and 19F3H2L3(hG1DM) all could effectively bind to antigen NT5E-His in a dose-dependent manner.

**Table 5. Results of assays for the binding of LASEV1 and LASEV2 to NT5E-His by ELISA**

| **Antibody dilution concentration (nM)** | **Coating:NT5E-His (1 µg/mL)** | | | | | | | | | | | | **EC₅₀ (nM)** |
|---|---|---|---|---|---|---|---|---|---|---|---|---|---|
| | **2.5** | **0.8333333** | **0.2777778** | **0.0925926** | **0.0308642** | **0.0102881** | **0.0034294** | **0.0011431** | **0.0003810** | **0.0001270** | **0.0000423** | **0** | |
| **LA5EV1** | **3.022** | **3.004** | **2.882** | **2.436** | **1.415** | **0.617** | **0.273** | **0.137** | **0.084** | **0.069** | **0.060** | **0.059** | **0.036** |
| | **3.057** | **3.010** | **2.886** | **2.379** | **1.325** | **0.598** | **0.279** | **0.132** | **0.078** | **0.067** | **0.058** | **0.062** | |
| **LA5EV2** | **3.103** | **3.073** | **2.971** | **2.613** | **1.607** | **0.739** | **0.327** | **0.162** | **0.090** | **0.065** | **0.059** | **0.058** | **0.029** |
| | **3.125** | **3.120** | **3.032** | **2.575** | **1.631** | **0.772** | **0.331** | **0.154** | **0.088** | **0.066** | **0.059** | **0.058** | |
| **19F3H2L3(hG1DM)** | **3.121** | **3.132** | **3.045** | **2.939** | **2.520** | **1.435** | **0.601** | **0.272** | **0.124** | **0.079** | **0.063** | **0.061** | **0.011** |
| | **3.072** | **3.055** | **3.020** | **2.916** | **2.534** | **1.531** | **0.645** | **0.279** | **0.132** | **0.083** | **0.069** | **0.065** | |
| **Secondary antibody** | **Goat Anti Human IgG Fc, HRP (1:5000)** | | | | | | | | | | | | |

**Table 6. Results of assays for the binding of NTLAV2 and NTLAV7 to NT5E-His by ELISA**

| **Antibody dilution concentration (nM)** | **Coating:NT5E-His (1 µg/mL)** | | | | | | | | | | | | **EC₅₀ (nM)** |
|---|---|---|---|---|---|---|---|---|---|---|---|---|---|
| | **2.5** | **0.8333333** | **0.2777778** | **0.0925926** | **0.0308642** | **0.0102881** | **0.0034294** | **0.0011431** | **0.0003810** | **0.0001270** | **0.0000423** | **0** | |
| **NTLAV2** | **3.062** | **3.065** | **2.941** | **2.481** | **1.430** | **0.614** | **0.259** | **0.130** | **0.084** | **0.068** | **0.066** | **0.074** | **0.037** |
| | **3.100** | **3.080** | **2.947** | **2.424** | **1.310** | **0.577** | **0.241** | **0.121** | **0.085** | **0.062** | **0.061** | **0.061** | |
| **NTLAV7** | **3.173** | **3.151** | **3.068** | **2.529** | **1.352** | **0.587** | **0.249** | **0.123** | **0.075** | **0.065** | **0.058** | **0.058** | **0.036** |
| | **3.131** | **3.092** | **3.041** | **2.559** | **1.422** | **0.656** | **0.268** | **0.142** | **0.080** | **0.062** | **0.061** | **0.061** | |
| **19F3H2L3(hG1DM)** | **3.164** | **3.151** | **3.097** | **2.962** | **2.489** | **1.441** | **0.627** | **0.267** | **0.132** | **0.079** | **0.065** | **0.059** | **0.011** |
| | **3.109** | **3.108** | **3.051** | **2.921** | **2.544** | **1.486** | **0.669** | **0.297** | **0.139** | **0.086** | **0.070** | **0.060** | |
| **Secondary antibody** | **Goat Anti Human IgG Fc, HRP (1:5000)** | | | | | | | | | | | | |

**Table 7. Results of assay for the binding of NTLAV8 to NT5E-His by ELISA**

| **Antibody dilution concentration (nM)** | **Coating:NT5E-His (1 µg/mL)** | | | | | | | | | | | | **EC₅₀ (nM)** |
|---|---|---|---|---|---|---|---|---|---|---|---|---|---|
| | **2.5** | **0.8333333** | **0.2777778** | **0.0925926** | **0.0308642** | **0.0102881** | **0.0034294** | **0.0011431** | **0.0003810** | **0.0001270** | **0.0000423** | **0** | |
| **NTLAV8** | **3.036** | **3.006** | **2.830** | **2.166** | **1.091** | **0.466** | **0.219** | **0.113** | **0.077** | **0.067** | **0.066** | **0.065** | **0.05** |
| | **3.067** | **3.051** | **2.870** | **2.178** | **1.094** | **0.467** | **0.197** | **0.102** | **0.075** | **0.061** | **0.053** | **0.063** | |
| **19F3H2L3(hG1DM)** | **3.162** | **3.156** | **3.073** | **2.913** | **2.308** | **1.202** | **0.528** | **0.221** | **0.111** | **0.073** | **0.062** | **0.057** | **0.015** |
| | **3.119** | **3.109** | **3.051** | **2.849** | **2.246** | **1.179** | **0.513** | **0.216** | **0.113** | **0.097** | **0.070** | **0.065** | |
| **Secondary antibody** | **Goat Anti Human IgG Fc, HRP (1:5000)** | | | | | | | | | | | | |

2. Assays for binding activity of LASEV1, LASEV2, NTLAV2, NTLAV7, and NTLAV8 for antigen huLAG3-mG1Fc by indirect ELISA The specific method was as follows:
An ELISA plate was coated with 2 µg/mL huLAG3-mG1Fc and incubated overnight at 4 °C. Then, the antigen-coated ELISA plate was washed once with PBST and blocked with a PBS solution containing 1% BSA (blocking solution) at 37 °C for 2 h. After blocking, the ELISA plate was washed 3 times with PBST. The antibodies serially diluted with a PBST solution (the dilution gradients for the antibodies are shown in Tables 8-10) were added. The ELISA plate containing the test antibodies was incubated at 37 °C for 30 min and then washed 3 times with PBST. After washing, an HRP-labeled goat anti-human IgG FC (H+L) (Jackson, Cat. No. 109-035-098) secondary antibody working solution diluted at a ratio of 1:5000 was added, and the plate was then incubated at 37 °C for 30 min. After the incubation, the plate was washed 4 times with PBST. Color development was then performed with TMB (Neogen, 308177) in the dark for 5 min, and a stop solution was added to stop the color development reaction. The ELISA plate was immediately placed into a microplate reader, and the OD at a wavelength of 450 nm in each well of the ELISA plate was measured. The data were analyzed and processed using SoftMax Pro 6.2.1 software.

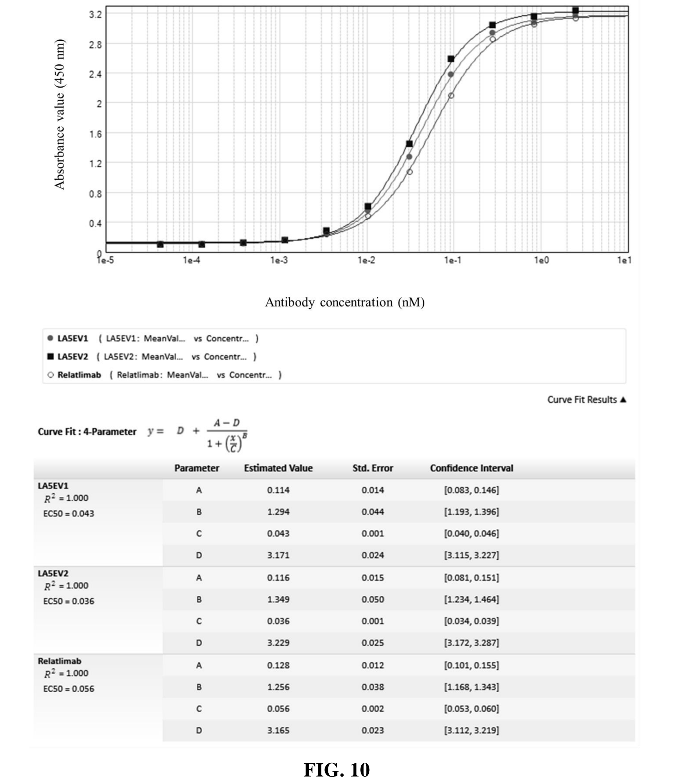

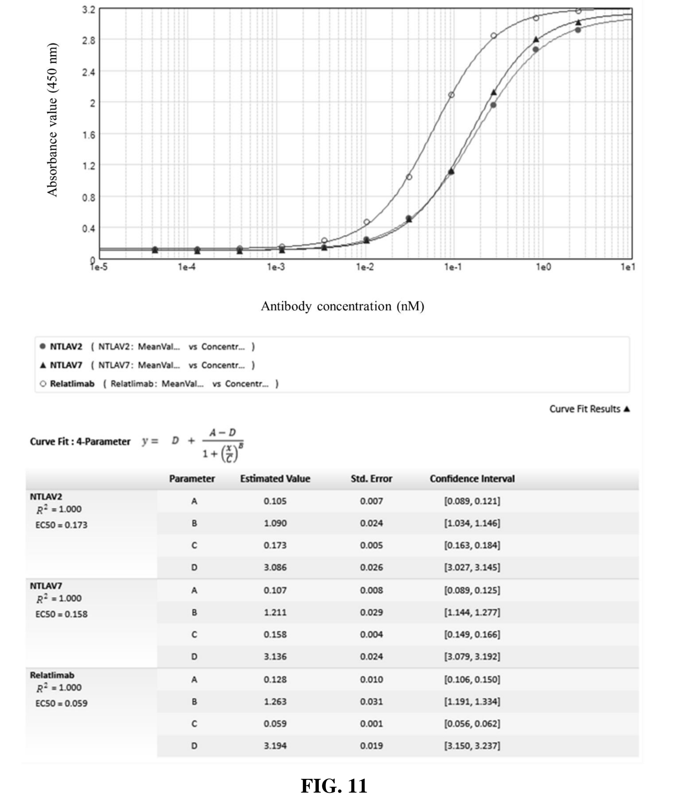

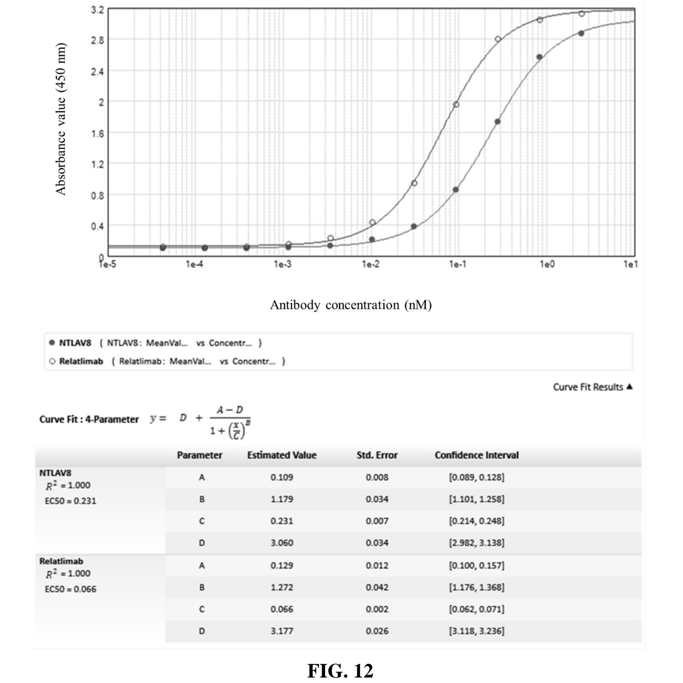

The assay results are shown in Tables 8-10 and FIGs. 10-12.

The results indicate that under the same experimental conditions, LASEV1, LA5EV2, NTLAV2, NTLAV7, NTLAV8, and Relatlimab all could effectively bind to antigen huLAG3-mG1Fc in a dose-dependent manner.

**Table 8. Results of assays for the binding of LASEV1 and LASEV2 to HuLAG3-mG1Fc by ELISA**

| **Antibody dilution concentration (nM)** | **Coating:huLAG3-mG1Fc (2µg/mL)** | | | | | | | | | | | | **EC₅₀ (nM)** |
|---|---|---|---|---|---|---|---|---|---|---|---|---|---|
| | **2.5** | **0.8333333** | **0.2777778** | **0.0925926** | **0.0308642** | **0.0102881** | **0.0034294** | **0.0011431** | **0.0003810** | **0.0001270** | **0.0000423** | **0** | |
| **LA5EV1** | **3.150** | **3.079** | **2.920** | **2.396** | **1.337** | **0.576** | **0.295** | **0.160** | **0.117** | **0.104** | **0.093** | **0.100** | **0.043** |
| | **3.180** | **3.075** | **2.942** | **2.361** | **1.217** | **0.533** | **0.247** | **0.145** | **0.113** | **0.101** | **0.097** | **0.097** | |
| **LA5EV2** | **3.220** | **3.147** | **3.032** | **2.565** | **1.467** | **0.615** | **0.292** | **0.156** | **0.124** | **0.103** | **0.095** | **0.098** | **0.036** |
| | **3.253** | **3.153** | **3.042** | **2.606** | **1.436** | **0.610** | **0.281** | **0.156** | **0.118** | **0.103** | **0.097** | **0.100** | |
| **Relatlimab** | **3.154** | **3.059** | **2.855** | **2.043** | **1.028** | **0.463** | **0.230** | **0.140** | **0.114** | **0.107** | **0.099** | **0.110** | **0.056** |
| | **3.107** | **3.034** | **2.843** | **2.155** | **1.118** | **0.511** | **0.255** | **0.165** | **0.137** | **0.126** | **0.125** | **0.139** | |
| **Secondary antibody** | **Goat Anti Human IgG Fc, HRP(1:5000)** | | | | | | | | | | | | |

**Table 9. Results of assays for the binding of NTLAV2 and NTLAV7 to HuLAG3-mG1Fc by ELISA**

| **Antibody dilution concentration (nM)** | **Coating:huLAG3-mG1Fc (2µg/mL)** | | | | | | | | | | | | **EC₅₀ (nM)** |
|---|---|---|---|---|---|---|---|---|---|---|---|---|---|
| | **2.5** | **0.8333333** | **0.2777778** | **0.0925926** | **0.0308642** | **0.0102881** | **0.0034294** | **0.0011431** | **0.0003810** | **0.0001270** | **0.0000423** | **0** | |
| **NTLAV2** | **2.890** | **2.680** | **1.997** | **1.181** | **0.540** | **0.268** | **0.152** | **0.116** | **0.107** | **0.105** | **0.105** | **0.111** | **0.173** |
| | **2.934** | **2.648** | **1.916** | **1.009** | **0.489** | **0.225** | **0.138** | **0.110** | **0.101** | **0.098** | **0.101** | **0.102** | |
| **NTLAV7** | **3.023** | **2.770** | **2.023** | **1.069** | **0.463** | **0.230** | **0.134** | **0.107** | **0.098** | **0.093** | **0.109** | **0.102** | **0.158** |
| | **3.007** | **2.833** | **2.232** | **1.176** | **0.538** | **0.242** | **0.147** | **0.114** | **0.102** | **0.102** | **0.102** | **0.103** | |
| **Relatlimab** | **3.187** | **3.084** | **2.862** | **2.024** | **0.986** | **0.454** | **0.223** | **0.147** | **0.119** | **0.120** | **0.108** | **0.109** | **0.059** |
| | **3.143** | **3.056** | **2.823** | **2.146** | **1.096** | **0.474** | **0.241** | **0.163** | **0.147** | **0.123** | **0.120** | **0.116** | |
| **Secondary antibody** | **Goat Anti Human IgG Fc, HRP(1:5000)** | | | | | | | | | | | | |

**Table 10. Results of assay for the binding of NTLAV8 to HuLAG3-mG1Fc by ELISA**

| **Antibody dilution concentration (nM)** | **Coating:huLAG3-mG1Fc (2µg/mL)** | | | | | | | | | | | | **EC₅₀ (nM)** |
|---|---|---|---|---|---|---|---|---|---|---|---|---|---|
| | **2.5** | **0.8333333** | **0.2777778** | **0.0925926** | **0.0308642** | **0.0102881** | **0.0034294** | **0.0011431** | **0.0003810** | **0.0001270** | **0.0000423** | **0** | |
| **NTLAV8** | **2.849** | **2.578** | **1.764** | **0.894** | **0.395** | **0.219** | **0.133** | **0.109** | **0.101** | **0.102** | **0.107** | **0.118** | **0.231** |
| | **2.896** | **2.543** | **1.688** | **0.798** | **0.367** | **0.196** | **0.125** | **0.113** | **0.097** | **0.091** | **0.094** | **0.103** | |
| **Relatlimab** | **3.174** | **3.076** | **2.836** | **1.954** | **0.943** | **0.418** | **0.217** | **0.139** | **0.116** | **0.106** | **0.104** | **0.108** | **0.066** |
| | **3.087** | **3.021** | **2.765** | **1.945** | **0.948** | **0.438** | **0.240** | **0.155** | **0.130** | **0.118** | **0.127** | **0.131** | |
| **Secondary antibody** | **Goat Anti Human IgG Fc, HRP (1:5000)** | | | | | | | | | | | | |

### Example 7: Determination of Kinetic Parameters of Humanized Antibodies LA5EV1, LASEV2, NTLAV2, NTLAV7, and NTLAV8

### 1. Determination of kinetic parameters of binding of humanized antibodies LA5EV1, LA5EV2, NTLAV2, NTLAV7, and NTLAV8 to hNTSE(1-552)-His

The sample dilution buffer was PBS containing 0.02% Tween-20 and 0.1% BSA (pH 7.4). 5 µg/mL hNTSE(1-552)-His was immobilized on the HIS1K sensor at an immobilization height of 0.15 nm. The sensor was equilibrated in a buffer for 60 s, and then the binding of the immobilized hNT5E(1-552)-His on the sensor to the antibodies at concentrations of 2.469-200 nM (three-fold dilution) was determined for 120 s. The antibodies were then dissociated in the buffer for 300 s. The sensor was regenerated 4 times in 10 mM glycine at pH 1.7, each for 5 s. The sample plate shaking speed was 1000 rpm, the determination temperature was 37 °C, and the frequency was 5.0 Hz. The data were analyzed by 1:1 model fitting to obtain affinity constants. The data acquisition software was Fortebio Data Acquisition 12.0, and the data analysis software was Fortebio Data Analysis 12.0.

**Table 11. Kinetic parameters of the binding of the humanized antibodies LA5EV1, LASEV2, NTLAV2, NTLAV7, and NTLAV8 to hNT5E(1-552)-His**

| **Antibody** | **Maximum signal height of analyte (nm)** | **KD (M)** | **Kon (1/Ms)** | **S E (kon)** | **Kdis (1/s)** | **S E (kdis)** | **Rmax (nm)** |
|---|---|---|---|---|---|---|---|
| **LA5EV1** | **0.2736** | **7.93E-10** | **5.79E+05** | **1.75E+03** | **4.60E-04** | **2.64E-06** | **0.25-0.36** |
| **LASEV2** | **0.2278** | **1.21E-09** | **4.95E+05** | **1.36E+03** | **5.99E-04** | **2.50E-06** | **0.21-0.36** |
| **NTLAV2** | **0.2014** | **4.08E-10** | **3.35E+05** | **1.23E+03** | **1.37E-04** | **3.22E-06** | **0.18-0.30** |
| **NTLAV7** | **0.2267** | **3.22E-10** | **3.88E+05** | **1.39E+03** | **1.25E-04** | **3.15E-06** | **0.20-0.28** |
| **NTLAV8** | **0.2032** | **3.40E-10** | **3.75E+05** | **1.71E+03** | **1.28E-04** | **4.01E-06** | **0.18-0.25** |
| **19F3H2L3(hG1DM)** | **0.1677** | **5.05E-10** | **5.93E+05** | **2.46E+03** | **3.00E-04** | **3.54E-06** | **0.14-0.17** |

| | | | | | | | |
|---|---|---|---|---|---|---|---|
| K_{D} is affinity constant; kon is binding rate of antigen and antibody; kdis is dissociation rate of antigen and antibody; K_{D} = kdis/kon. | | | | | | | |

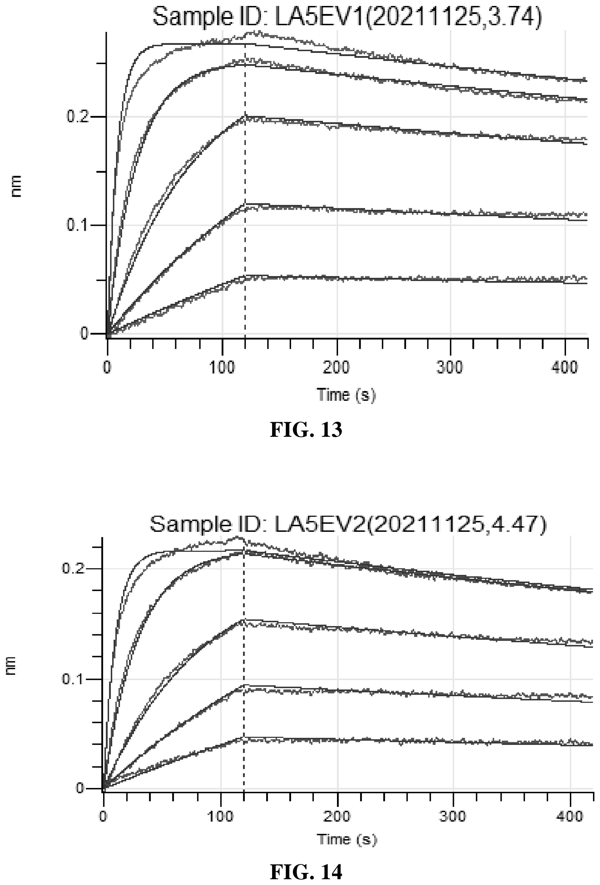

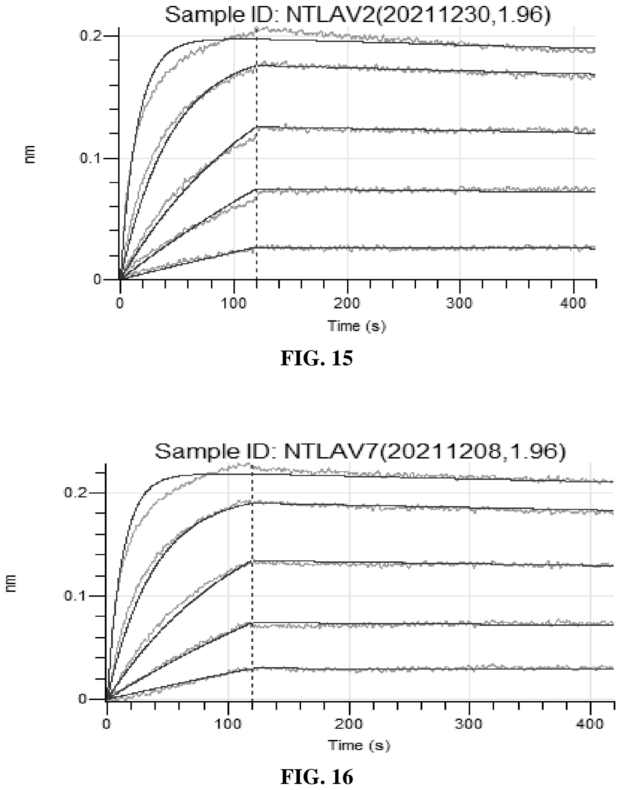

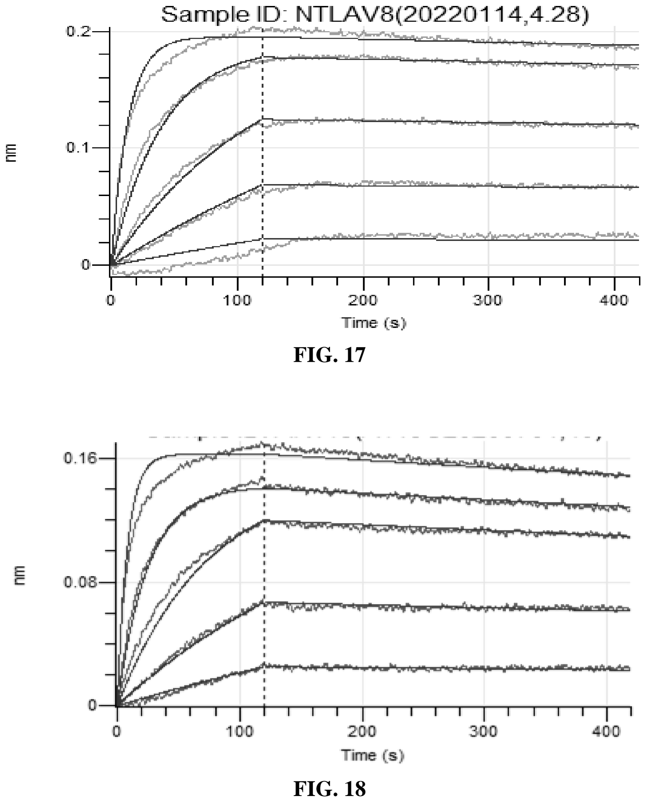

The results are shown in Table 11 and FIGs. 13-18.

The results indicate that the humanized antibodies LASEV1, LASEV2, NTLAV2, NTLAV7, and NTLAV8 all had good affinity for antigen hNTSE(1-552)-His.

### 2. Determination of kinetic parameters of binding of humanized antibodies LA5EV1, LA5EV2, NTLAV2, NTLAV7, and NTLAV8 to huLAG3-mG1Fc

The sample dilution buffer was PBS containing 0.02% Tween-20 and 0.1% BSA (pH 7.4). 10 µg/mL huLAG3-mG1Fc was immobilized on the AMC sensor at an immobilization height of 0.8 nm. The sensor was equilibrated in a buffer for 60 s, and then the binding of the immobilized huLAG3-mG1Fc on the sensor to the antibodies at concentrations of 6.17-500 nM (three-fold dilution) was determined for 100 s. The antibodies were then dissociated in the buffer for 400 s. The sensor was regenerated 4 times in 10 mM glycine at pH 1.7, each for 5 s. The sample plate shaking speed was 1000 rpm, the determination temperature was 30 °C, and the frequency was 5.0 Hz. The data were analyzed by 1:1 model fitting to obtain affinity constants. The data acquisition software was Fortebio Data Acquisition 12.0, and the data analysis software was Fortebio Data Analysis 12.0.

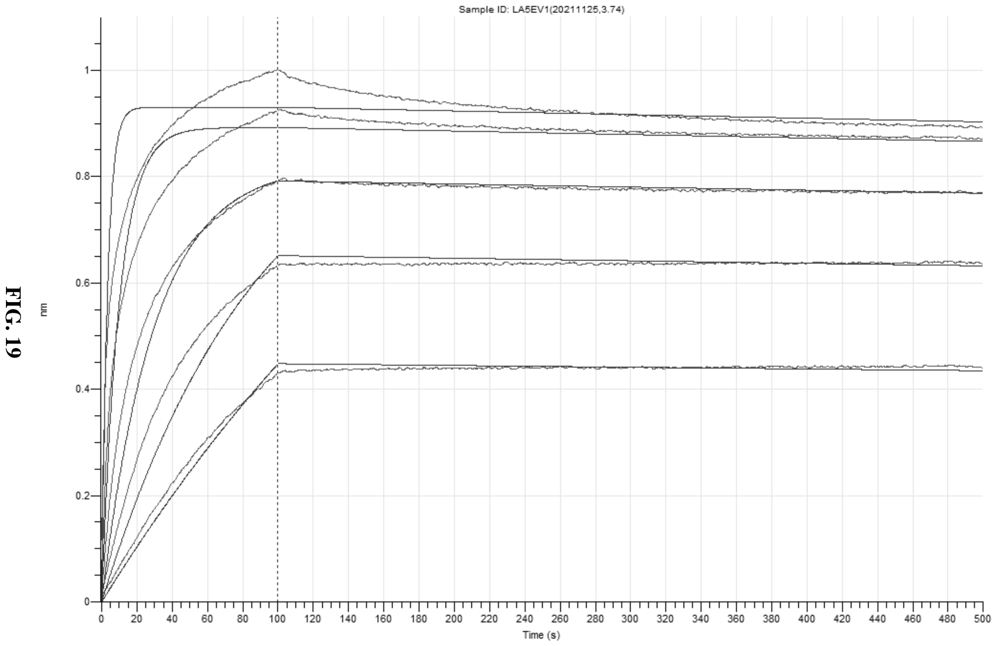

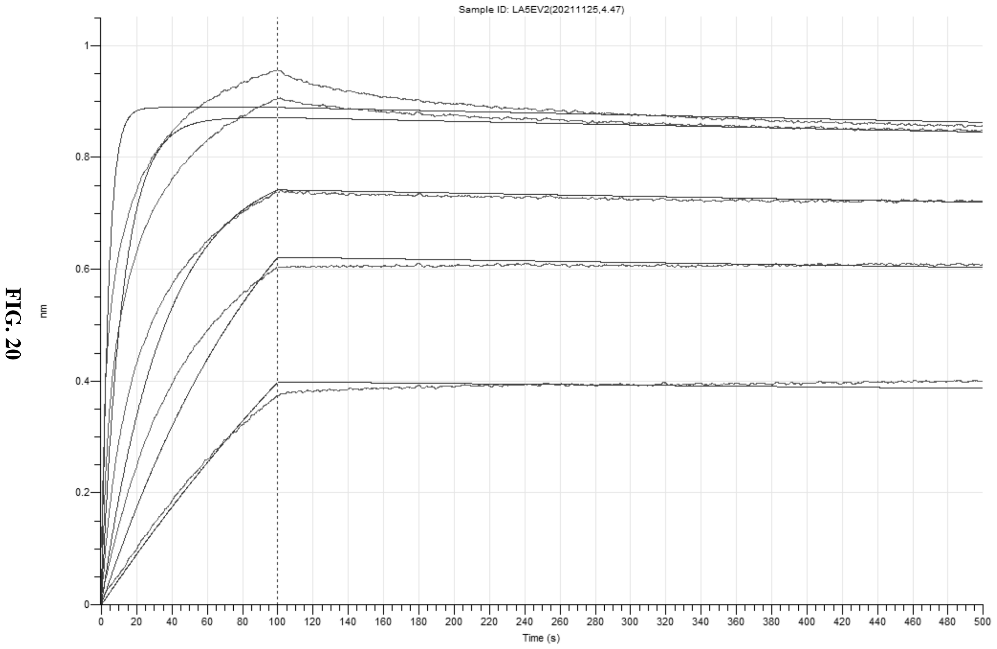

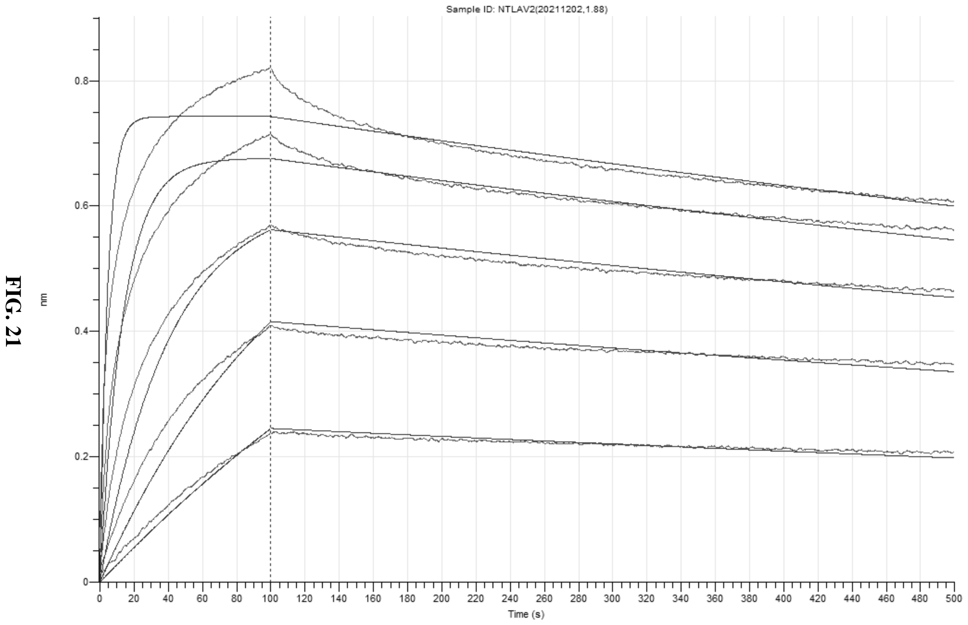

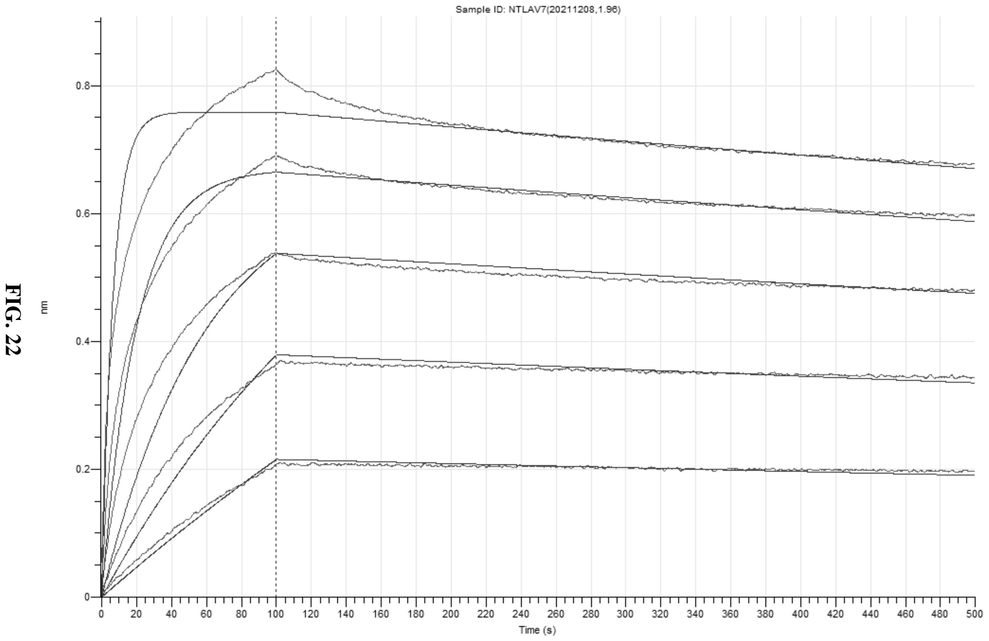

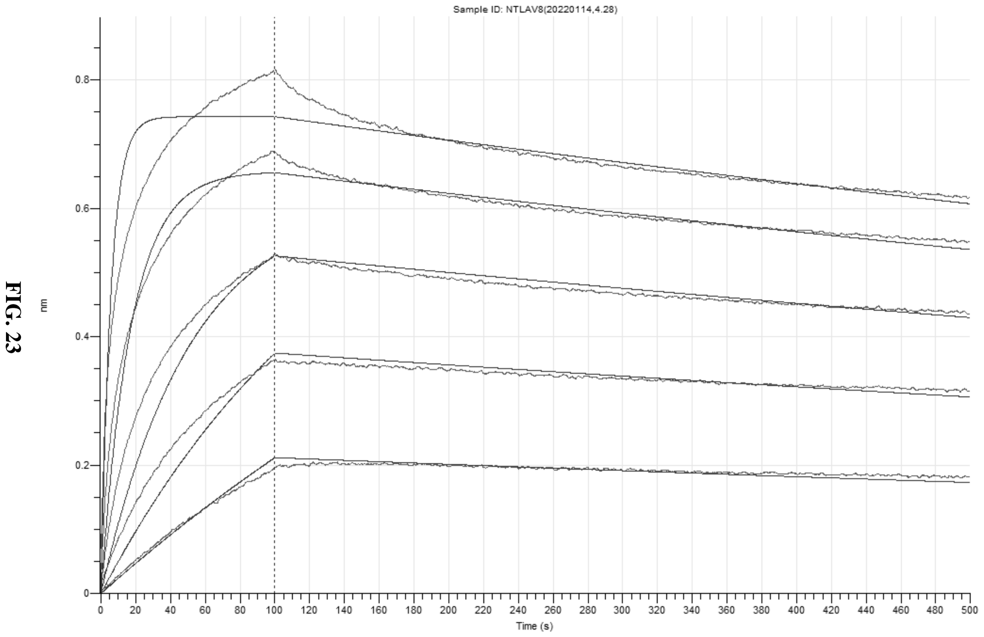

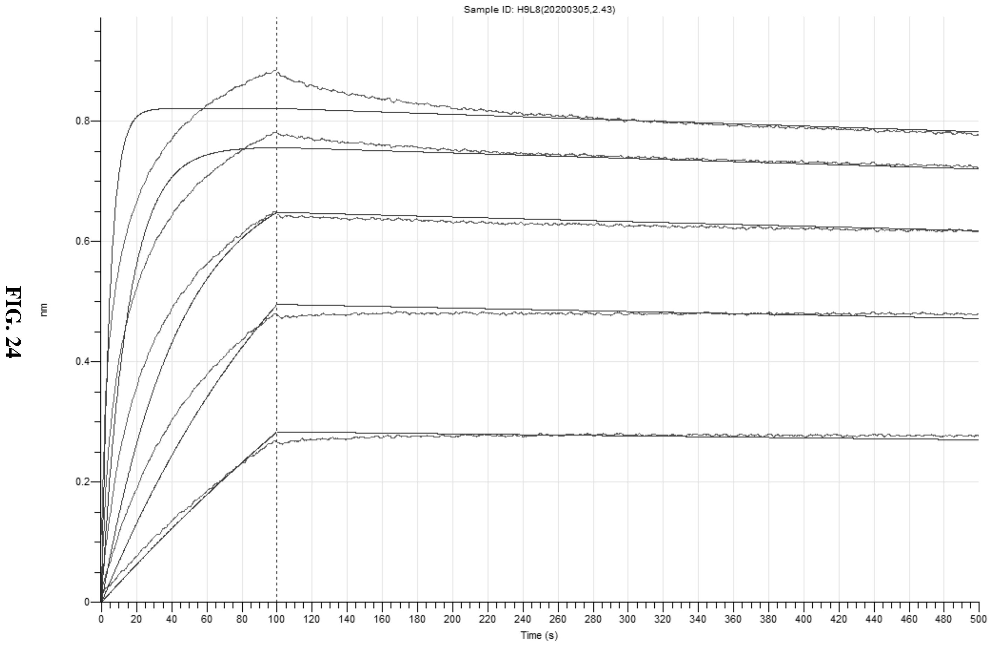

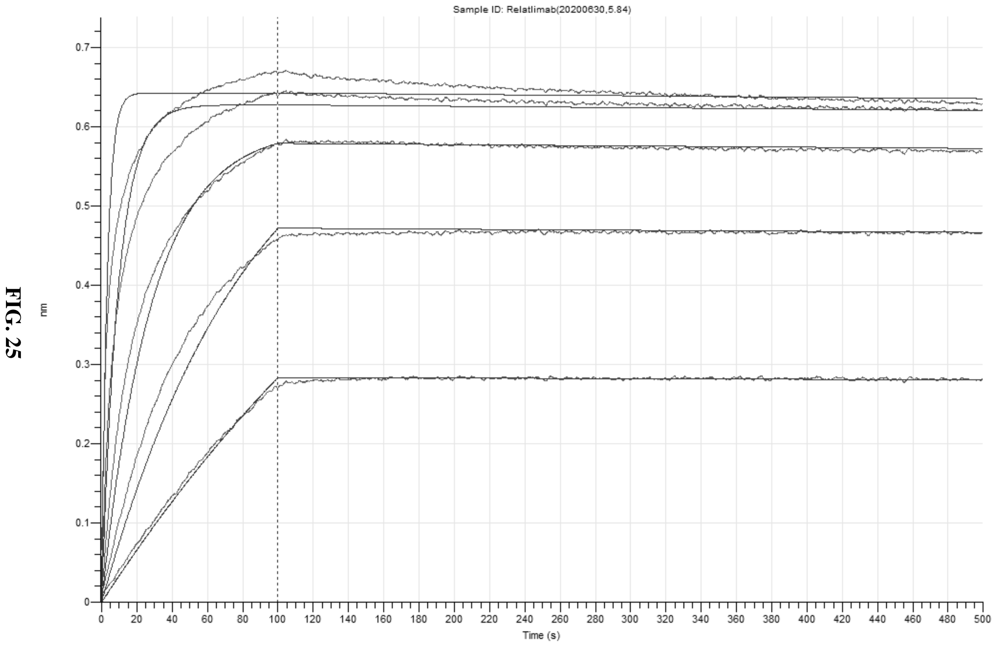

The results are shown in Table 12 and FIGs. 19-25.

The results indicate that the humanized antibodies LASEV1, LASEV2, NTLAV2, NTLAV7, and NTLAV8 all had good affinity for antigen huLAG3-mG1Fc.

**Table 12. Kinetic parameters of the binding of the humanized antibodies LASEV1, LA5EV2, NTLAV2, NTLAV7, and NTLAV8 to huLAG3-mG1Fc**

| **Antibody** | **Maximum signal height of analyte (nm)** | **KD (M)** | **Kon (1/Ms)** | **S E (kon)** | **Kdis (1/s)** | **S E (kdis)** | **Rmax (nm)** |
|---|---|---|---|---|---|---|---|
| **LA5EV1** | **0.9935** | **1.25E-10** | **5.98E+05** | **3.30E+03** | **7.46E-05** | **2.68E-06** | **0.82-1.46** |
| **LASEV2** | **0.9481** | **1.52E-10** | **5.00E+05** | **2.84E+03** | **7.58E-05** | **2.80E-06** | **0.80-1.51** |
| **NTLAV2** | **0.8142** | **1.23E-09** | **4.36E+05** | **2.47E+03** | **5.35E-04** | **3.08E-06** | **0.63-1.07** |
| **NTLAV7** | **0.8167** | **1.04E-09** | **2.98E+05** | **1.68E+03** | **3.09E-04** | **3.11E-06** | **0.67-1.30** |
| **NTLAV8** | **0.8058** | **1.47E-09** | **3.44E+05** | **1.88E+03** | **5.05E-04** | **3.05E-06** | **0.63-1.13** |
| **H9L8(hG4WT)** | **0.8777** | **3.00E-10** | **4.05E+05** | **2.33E+03** | **1.21E-04** | **2.94E-06** | **0.73-1.29** |
| **Relatlimab** | **0.6674** | **4.47E-11** | **6.32E+05** | **2.87E+03** | **2.82E-05** | **2.22E-06** | **0.63-0.88** |

### Example 8: Assays for Binding Activity of Anti-CD73-Anti-LAG3 Bispecific Antibodies for CD73/LAG3 Antigen by FACS

### 1. Assays for binding activity of anti-CD73-anti-LAG3 bispecific antibodies for LAG3 on 293T-LAG3 membrane surface by FACS

293T-LAG3 cells in logarithmic growth phase were collected and transferred to a 96-well flow cytometry plate at 3 × 10⁵ cells/well. 100 µL of 1% PBSA was added, and the mixture was centrifuged at 350× g for 5 min, followed by removal of the supernatant. 100 µL of each antibody diluted with 1% PBSA (at final concentrations of 300 nM, 100 nM, 33.33 nM, 11.11 nM, 3.7 nM, 1.23 nM, 0.41 nM, 0.041 nM, 0.0041 nM, and 0.00041 nM) was added. The mixture was gently mixed well and then incubated on ice for 1 h. 200 µL of 1% PBSA was added, and the mixture was centrifuged at 350× g for 5 min, followed by removal of the supernatant. The plate was washed twice. A 300-fold diluted Alexa Fluor^{®} 647-labeled mouse anti-human IgG secondary antibody (Southern Biotech, Cat. No. 9040-31) was added for resuspension. The mixture was mixed well and incubated on ice in the dark for 0.5 h. 200 µL of 1% PBSA was added, and the mixture was centrifuged at 350× g for 5 min, followed by removal of the supernatant. The plate was washed twice. 200 µL of 1% PBSA was added to resuspend the cell pellets, and the suspension was assayed on a flow cytometer.

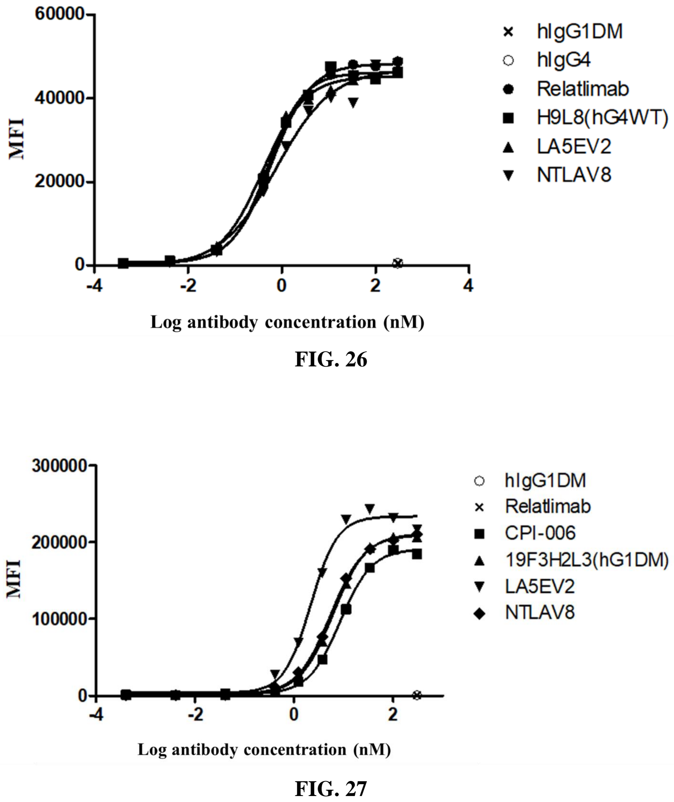

The experimental results are shown in Table 13 and FIG. 26.

The results show that NTLAV8, LASEV2, H9L8(hG4WT), and Relatlimab all could specifically bind to the LAG3 receptor on the 293T-LAG3 cell membrane surface.

**Table 13. Results of assays for the binding activity of NTLAV8, LA5EV2, H9L8(hG4WT), and Relatlimab for LAG3 on 293T-LAG3 cell surface by FACS**

| **Antibody** | **Relatlimab** | **H9L8(hG4WT)** | **LA5EV2** | **NTLAV8** |
|---|---|---|---|---|
| **EC₅₀ (nM)** | **0.6026** | **0.5011** | **0.3989** | **0.7587** |

### 2. Assays for binding activity of anti-CD73-anti-LAG3 bispecific antibodies for CD73 on U87-MG cell membrane surface by FACS

U87-MG cells (ATCC, Cat. No. HTB-14) in logarithmic growth phase were collected and transferred to a 96-well plate at 3 × 10⁵ cells/well. 200 µL of 1% PBSA was added, and the mixture was centrifuged at 750× g for 5 min, followed by removal of the supernatant. 100 µL of each antibody diluted with 1% PBSA (at final concentrations of 300 nM, 100 nM, 33.33 nM, 11.11 nM, 3.7 nM, 1.23 nM, 0.41 nM, 0.041 nM, 0.0041 nM, and 0.00041 nM) was added. The mixture was gently mixed well and then incubated on ice for 1 h. 200 µL of 1% PBSA was added, and the mixture was centrifuged at 750× g for 5 min, followed by removal of the supernatant. The plate was washed twice. A 300-fold diluted Alexa Fluor^{®} 647-labeled mouse anti-human IgG secondary antibody (Southern Biotech, Cat. No. 9040-31) was added for resuspension. The mixture was mixed well and incubated on ice in the dark for 0.5 h. 100 µL of 1% PBSA was added, and the mixture was centrifuged at 750× g for 5 min, followed by removal of the supernatant. The plate was washed twice with 200 µL of 1% PBSA. 200 µL of 1% PBSA was added to resuspend the cell pellets, and the suspension was assayed on a flow cytometer.

The experimental results are shown in Table 14 and FIG. 27.

The results show that NTLAV8, LASEV2, 19F3H2L3(hG1DM), and CPI-006 all could specifically bind to CD73 on the U87-MG cell membrane surface, and the ability of NTLAV8 and LA5EV2 to bind to CD73 on the U87-MG cell membrane surface was stronger than that of the positive control CPI-006.

**Table 14. Results of assays for the binding activity of NTLAV8, LA5EV2, 19F3H2L3(hG1DM), and CPI-006 for CD73 on U87-MG cell surface by FACS**

| **Antibody** | **CPI-006** | **19F3H2L3(hG1DM)** | **LA5EV2** | **NTLAV8** |
|---|---|---|---|---|
| **EC₅₀ (nM)** | **8.472** | **6.059** | **2.162** | **5.456** |

### Example 9: Assays for Anti-CD73-Anti-LAG3 Bispecific Antibodies Competing with MHC II on Raji Cell Membrane Surface for Binding to Human LAG3-mG1Fc Antigen by FACS

According to the experimental design, the antibodies and LAG3-mG1Fc were diluted and mixed well in a ratio of 1:1, such that the final concentration of LAG3-mG1Fc was 3 nM, and the final concentrations of the antibodies were 300 nM, 100 nM, 33.33 nM, 11.11 nM, 3.7 nM, 1.23 nM, 0.41 nM, 0.041 nM, 0.0041 nM, or 0.00041 nM. The mixture was incubated on ice for 30 min. The Raji cells were collected conventionally and seeded into a V-bottom 96-well plate at 3 × 10⁵ cells/well. 1% PBSA was added, and the mixture was centrifuged at 500× g for 5 min, followed by removal of the supernatant. 150 µg/mL mouse IgG Isotype control (Thermofisher, Cat. No. 10400C) was added, and the mixture was incubated on ice for 20 min and then centrifuged at 500× g for 5 min, followed by removal of the supernatant. 100 µL of an antibody-protein pre-incubation solution was added to resuspend the cells, and a blank control, a negative control, and an isotype control were designed. The plate was incubated on ice in the dark for 1 h. 100 µL of 1% PBSA was added, and the mixture was centrifuged at 500× g for 5 min, followed by removal of the supernatant. The plate was washed twice with 200 µL of 1% PBSA. 100 µL of 300-fold diluted APC-labeled goat anti-mouse IgG secondary antibody (Biolegend, Cat. No. 405308) was added to resuspend the cells, and 100 µL of 1% PBSA was added to resuspend the blank control. The mixture was incubated on ice in the dark for 30 min. 100 µL of 1% PBSA was added, and the mixture was centrifuged at 500× g for 5 min, followed by removal of the supernatant. The plate was washed twice with 200 µL of 1% PBSA. 200 µL of 1% PBSA was added to resuspend the cells, and the mixture was assayed on a flow cytometer.

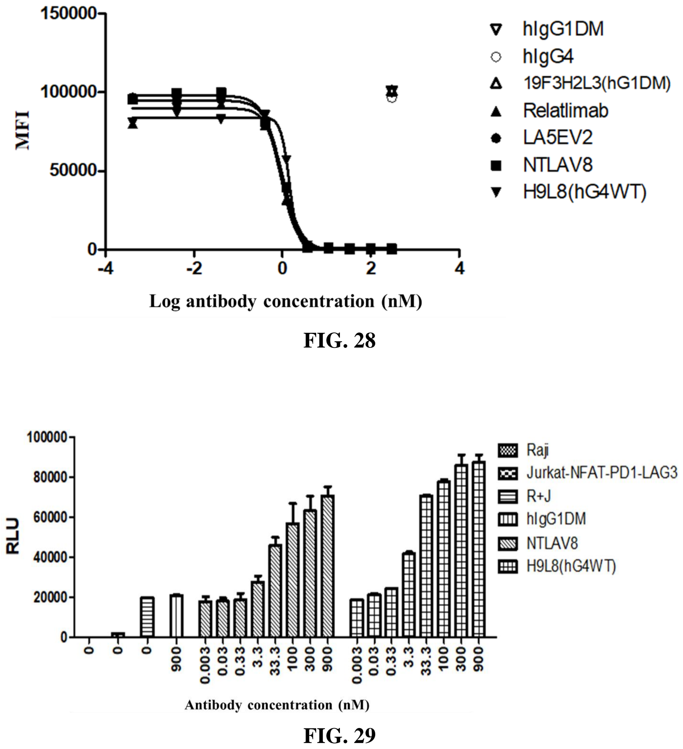

The EC₅₀ values of the samples are shown in Table 15. The results are shown in FIG. 28. The results indicate that the antibodies NTLAV8, LASEV2, Relatlimab (positive control), and H9L8(hG4WT) all could competitively bind to human LAG3-mG1Fc and effectively block the binding of LAG3-mG1Fc to MHC II on the Raji cell surface in a dose-dependent manner.

**Table 15. Results of analysis for the fluorescence intensities of NTLAV8, LASEV2, Relatlimab, and H9L8 in competing with MHC II on Raji cell surface for binding to the human LAG3-mG1Fc antigen by FACS**

| **Antibody** | **LA5EV2** | **NTLAV8** | **Relatlimab** | **H9L8(hG4WT)** |
|---|---|---|---|---|
| **EC₅₀ (nM)** | **0.9989** | **0.9740** | **0.9448** | **1.440** |

### Example 10: Blocking Assays for Anti-CD73-Anti-LAG3 Bispecific Antibodies

Jurkat-NFAT-PD1-LAG3 cells (constructed by Akeso Biopharma Inc.) and Raji cells (Cell Resource Center, Shanghai Institutes for Biological Sciences, Chinese Academy of Sciences, Cat. No. TCHu 44) were collected and centrifuged at 110× g for 5 min, followed by removal of the supernatant. The cells were resuspended in a 1640 medium (containing 10% FBS) and counted. The Jurkat-NFAT-PD1-LAG3 cells were seeded into a black-bottom 96-well plate (Corning, Model No. 3916) at 10 × 10⁴ cells/well. According to the experimental design, the antibodies (at final concentrations of 900 nM, 300 nM, 100 nM, 33.3 nM, 3.3 nM, 0.33 nM, 0.03 nM, and 0.003 nM) were added, and the mixture was pre-incubated at 37 °C in a 5% CO₂ incubator for 30 min. SEE (staphylococcal enterotoxin E) (at a final concentration of 0.05 ng/mL, Toxin Technology, Cat. No. ET404) and Raji cells were incubated at 37 °C in a 5% CO₂ incubator for 30 min. After incubation for 30 min, the Raji cells were added to the 96-well plate at 2 × 10⁴ cells/well (the final volume of the system was 80 µL). The mixture was mixed well and incubated at 37 °C in a 5% CO₂ incubator for 16 h. The culture plate was taken out and allowed to equilibrate to room temperature. Firefly Glo Luciferase Reporter Gene Assay Kit (Yeasen, Cat. No. 11404ES80) was added at 80 µL/well, and the mixture was incubated in the dark for 2 min. Then, the RLU values were read.

The results are shown in FIG. 29.

The results show that both NTLAV8 and H9L8(hG4WT) could effectively block the inhibition of the signaling pathway mediated by the interaction between LAG3 and MHCII.

### Example 11: Assays for Inhibition of Enzyme Activity of CD73 on Cell Membrane Surface by Anti-CD73-Anti-LAG3 Bispecific Antibodies

The groups in the experiment were set as follows:
ATP control group, AMP + ATP control group, negative control group (cells + AMP + ATP), APCP control group (cells + APCP + AMP + ATP), isotype control group (cells + isotype control antibody + AMP + ATP), and antibody group (cells + corresponding antibody + AMP + ATP).

ATP refers to adenosine triphosphate, AMP refers to adenosine monophosphate, and APCP refers to α,β-methylene adenosine-5'-diphosphate, which is a specific CD73 inhibitor.

U87-MG cells (ATCC, Cat. No. HTB-14) in logarithmic phase and good condition were taken, resuspended in an analytic medium (i.e., a serum-free RPMI-1640 culture solution), and counted. The U87-MG cells were seeded into a 96-well plate at 2.5 × 10⁴ cells/60 µL/well. 120 µM APCP and diluted antibodies (initial concentration of 300 nM, diluted in a 3-fold gradient (10-fold dilution of the last two low concentrations)) were prepared in an analytic medium. The APCP or the diluted antibodies described above were added to the 96-well plate at 60 µL/well, and the plate was incubated at 37 °C for 1 h. Then AMP (AMP was purchased from TCI, Cat. No. A0158) diluted with an analytic medium to a concentration of 600 µM was added at 60 µL/well, and the mixture was mixed well and then incubated for 3 h. After 3 h, 100 µL of cell culture supernatant was taken from each well and transferred to a new 96-well plate to which CTG (CellTiter-Glo^{®} One Solution Assay) (promega, Cat. No. G8461) had been added at 40 µL/well. The plate was gently tapped to mix well and left to stand in the dark at room temperature for 5 min. After 5 min of standing, ATP (5'-ATP 2Na Hydrate, purchased from TCI, Cat. No. A0157) at a concentration of 300 µM prepared with a TM buffer (self-prepared by Akeso Biopharma Inc.) was added at 10 µL/well. The plate was gently tapped to mix well and left to stand in the dark at room temperature for 5 min. Finally, the data were read on a multi-label microplate tester (PerkinElmer, Model No. 2140-0020).

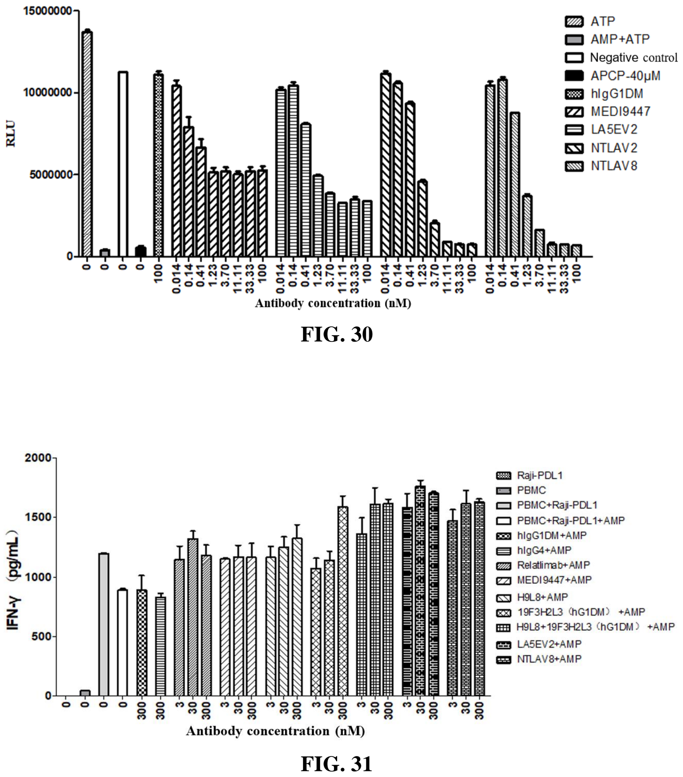

The experimental results are shown in Table 16 and FIG. 30. The results show that the anti-CD73-anti-LAG3 antibodies LASEV2, NTLAV2, and NTLAV8 and the positive control drug MEDI9447 for the CD73 target all could inhibit, in a dose-dependent manner, the enzyme activity of CD73 endogenously expressed by U87-MG to catalyze the conversion of AMP to adenosine, thereby reducing the mean fluorescence intensity RLU value in a dose-dependent manner; LASEV2 and NTLAV8 exhibited a stronger activity in inhibiting the enzyme activity of CD73 than MEDI9447.

**Table 16. Results of the inhibition of the enzyme activity of CD73 endogenously expressed in cells by anti-CD73-anti-LAG3 bispecific antibodies**

| **Antibody** | **MEDI9447** | **LA5EV2** | **NTLAV2** | **NTLAV8** |
|---|---|---|---|---|
| **EC₅₀ (nM)** | **0.1520** | **0.6097** | **0.9399** | **0.8160** |
| **Maximum inhibition rate (%)** | **58.26** | **74.69** | **98.24** | **98.80** |

### Example 12: Assays for Biological Activity of Anti-CD73-Anti-LAG3 Bispecific Antibodies in Promoting IFN-γ Secretion by Mixed Lymphocyte Reaction (MLR)

Raji-PDL1 cells (constructed by Akeso Biopharma Inc.) were conventionally subcultured; meanwhile, PBMCs (from healthy donors) were thawed, cultured in 10 mL of a 1640 complete medium, and stimulated with SEB (Staphylococcal enterotoxin B, Toxin technology, Cat. No. BT202) at a final concentration of 0.5 µg/mL for two days. After two days, the Raji-PDL1 cells were conventionally collected and resuspended in an analytic medium (i.e., RPMI-1640 + 10% FBS), and then MMC (Mito-mycin C, Stressmarq, Cat. No. SIH-246-10MG) at a final concentration of 2 µg/mL was added. The mixture was incubated at 37 °C in a 5% CO₂ incubator for 1 h. The PBMCs stimulated with SEB for two days and the Raji-PDL1 cells treated with MMC for 1 h were conventionally collected and washed twice with an analytic medium. The two types of cells were each resuspended in an analytic medium, counted, and added to a U-shaped 96-well plate (Corning, Model No. 3799) at 1 × 10⁵ cells/well for co-culture. According to the experimental design, AMP (5'-adenylic acid) (at a final concentration of 200 µM, 200TCI, Cat. No. A0158) and antibodies (at final concentrations of 300 nM, 30 nM, and 3 nM) were added, and a negative control group (PBMC + Raji-PDL1 + AMP), an isotype control group, and the like were set. The cells were co-cultured in an incubator for three days. After three days, the cells were centrifuged at 250× g for 5 min, and the cell culture supernatant was collected and assayed for IFN-γ by ELISA.

As shown in FIG. 31, in the mixed culture system of human PBMCs and Raji-PDL1 cells, the addition of the antibodies could significantly induce the secretion of IFN-γ in the system, and the anti-CD73-anti-LAG3 antibodies LASEV2 and NTLAV8 exhibited a superior activity than the antibodies 19F3H2L3(hG1DM) and MEDI9447 targeting only CD73 and the control antibodies H9L8(hG4WT) and Relatlimab targeting only LAG-3.

### Example 13: Potential Cell Phagocytic Activity Mediated by Anti-CD73-Anti-LAG3 Bispecific Antibodies on CHO-K1-LAG3-CD73 Target Cells

Cryopreserved MBMM (induced from C57 mice, purchased from Guangdong GemPharmatech Co., Ltd.) was thawed and cultured overnight in a DMEM + 10% FBS + 100 ng/mL M-CSF (Murine M-CSF, peprotech, Cat. No. 315-02) medium.

The target cells CHO-K1-CD73-LAG3 (constructed by Akeso Biopharma Inc.) were collected, centrifuged at 170× g for 5 min, washed once with PBS, and counted. CFSE (Biolegend, Cat. No. 423801) was diluted with PBS to a final concentration of 2.5 µM. A proper amount of the diluted CFSE was taken to resuspend the cells (staining density: 1 × 10⁷ cells/mL), and the cells were incubated in an incubator for 20 min. 6 mL of a DMEM complete medium (containing 10% FBS) was added to stop the staining. The cells were centrifuged at 170× g for 5 min, followed by removal of the supernatant. 1 mL of a DMEM complete medium was added, and the cells were incubated in an incubator for 10 min. The antibodies were diluted with a DMEM complete medium to final concentrations of 0.01 nM, 0.1 nM, 1 nM, 10 nM, and 100 nM, and negative control and isotype control were set. The target cells were added to a V-bottom 96-well plate at 1.5 × 10⁵ cells/well, and the antibodies were added. The mixture was mixed well and incubated on ice for 40 min. The mixture was centrifuged at 170× g for 5 min and washed twice with a DMEM complete medium. Macrophages (MBMMs) were collected and centrifuged at 750× g for 5 min, followed by removal of the supernatant. The cells were counted, resuspended in a DMEM complete medium, adjusted to a concentration of 5 × 10⁴ cells/100 µL, and added to the V-bottom 96-well plate containing target cells. The cells were resuspended, mixed well, and incubated in an incubator at 37 °C for 2 h. 100 µL of 1% PBSA at room temperature was added to each well, and the mixture was centrifuged at 750× g for 5 min, followed by removal of the supernatant. The plate was washed once with 200 µL of 1% PBSA. An APC anti-mouse/human CD11b antibody (Biolegend, Cat. No. 101212) diluted 500-fold with 1% PBSA was added to the corresponding samples at 100 µL/well, and the mixture was mixed well and incubated on ice for 40 min. 100 µL of 1% PBSA was added to each well, and the mixture was centrifuged at 750× g for 5 min, followed by removal of the supernatant. The plate was washed once with 200 µL of 1% PBSA. The cells were resuspended in 200 µL of 1% PBSA and assayed on a flow cytometer.

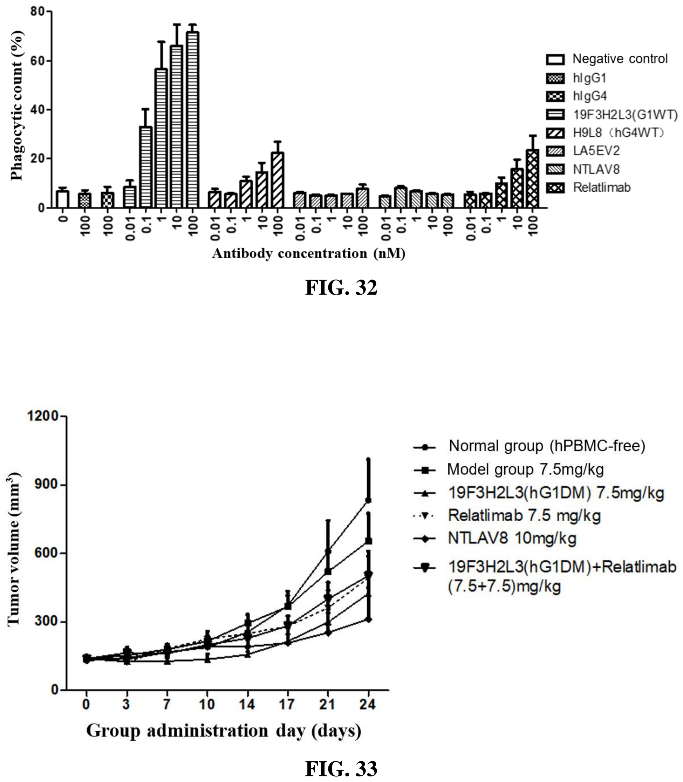

The results are shown in FIG. 32.

The results show that the antibody 19F3H2L2(G1WT) targeting only CD73 could promote phagocytosis of CHO-K1-CD73-LAG3 cells by macrophages (MBMMs) and had an ADCP effect, while the anti-CD73-anti-LAG3 antibodies LASEV2 and NTLAV8 had no ADCP effect.

### Example 14: Pharmacodynamic Evaluation of Anti-CD73-Anti-LAG3 Bispecific Antibody in Mouse Model Subcutaneously Grafted with Tumor Cells

To determine the *in vivo* anti-tumor activity of the anti-CD73-anti-LAG3 bispecific antibody, MIA-PaCa-2 cells (purchased from China Center for Type Culture Collection Cell Bank) were first inoculated subcutaneously into female NCG mice aged 6-7 weeks (purchased from Guangdong GemPharmatech Co., Ltd.). The day of grouping was defined as D0. The route of administration was intraperitoneal injection (IP), once weekly for a total of 4 doses. The modeling and specific dosing regimen are shown in Table 17. After the administration, the length and width of the tumors in each group were measured, and the tumor volume was calculated. Tumor volume was calculated according to the following formula: long diameter of tumor × short diameter of tumor²/2. The data were processed using GraphPad Prism 5 statistical processing software.

**Table 17. Dosing regimen of the anti-CD73-anti-LAG3 bispecific antibody for treating NCG mouse MIA-PaCa-2 cell xenograft tumor model**

| **Group** | **Number of animals** | **Inoculation** | **Administration** |
|---|---|---|---|
| **Normal group** | **6** | **MIA-PaCa-2 cells (5 million), subcutaneously inoculated** | **Normal saline, IP, weekly × 4** |
| **Model group** | **6** | **NCG mice were each subcutaneously inoculated with 5 million MIA-PaCa-2 cells, and each mouse was then intraperitoneally injected with 5 million hPBMCs (unactivated) on day 5 after inoculation. When the mean tumor volume reached about 140 mm³, the mice were grouped for administration (D0).** | **hIgG1, 7.5mg/kg, IP, weekly×4** |
| **19F3H2L3 (hG1DM)** | **6** | | **7.5mg/kg, IP, weekly×4** |
| **Relatlimab** | **6** | | **7.5mg/kg, IP, weekly×4** |
| **NTLAV8** | **6** | | **10mg/kg, IP, weekly×4** |
| **19F3H2L3 (hG1DM)+ Relatlimab** | **6** | | **7.5mg/kg+7.5mg/kg, IP, weekly×4** |

| | | | |
|---|---|---|---|
| Note: The doses of 19F3H2L3(hG1DM) at 7.5 mg/kg, Relatlimab at 7.5 mg/kg, and NTLAV8 at 10 mg/kg were based on an equal molar concentration. | | | |

The results are shown in FIG. 33. The results show that compared with the isotype control antibody, the anti-CD73-anti-LAG3 bispecific antibody NTLAV8 and the control antibodies Relatlimab and 19F3H2L3(hG1DM) all could effectively inhibit the growth of tumors in mice. At the same dose, the anti-tumor effect of the anti-CD73-anti-LAG3 bispecific antibody NTLAV8 was superior to that of the monotherapy with the control antibody Relatlimab or 19F3H2L3(hG1DM) and that of the combination therapy of Relatlimab and 19F3H2L3(hG1DM).

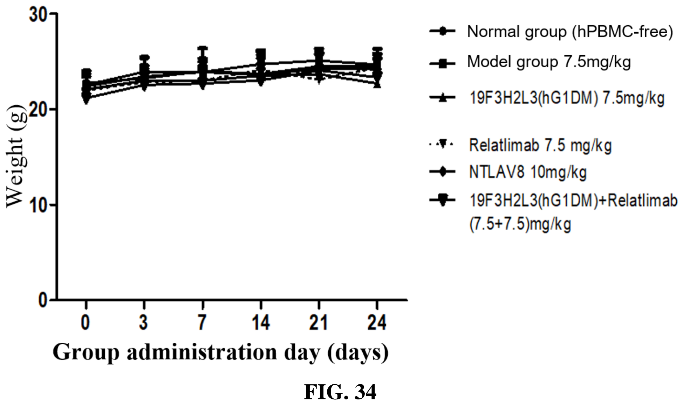

In addition, as shown in FIG. 34, the tumor-bearing mice exhibited good tolerance to all test drugs, and the test drugs in each group had no significant effect on the body weight of the tumor-bearing mice.

Although the specific embodiments of the present disclosure have been described in detail, those skilled in the art will appreciate that various modifications and substitutions can be made to those details according to all the teachings that have been disclosed, and these changes shall all fall within the protection scope of the present disclosure. The full scope of the present disclosure is given by the appended claims and any equivalent thereof.

## Claims

1. A bispecific antibody, comprising a first protein functional region and a second protein functional region, wherein:
the first protein functional region targets LAG3, and
the second protein functional region targets a target other than LAG3 (e.g., CD73 or PD-1),
wherein the first protein functional region is an anti-LAG3 antibody or an antigen-binding fragment thereof, and the anti-LAG3 antibody comprises a heavy chain variable region and a light chain variable region; the heavy chain variable region comprises HCDR1, HCDR2, and HCDR3, and the light chain variable region comprises LCDR1, LCDR2, and LCDR3, wherein
HCDR1 has the amino acid sequence set forth in SEQ ID NO: 9,
HCDR2 has the amino acid sequence set forth in SEQ ID NO: 10, and
HCDR3 has the amino acid sequence set forth in SEQ ID NO: 11;
LCDR1 has the amino acid sequence set forth in SEQ ID NO: 12 or SEQ ID NO: 17,
LCDR2 has the amino acid sequence set forth in SEQ ID NO: 13 or SEQ ID NO: 15, and
LCDR3 has the amino acid sequence set forth in SEQ ID NO: 14 or SEQ ID NO: 16.

2. The bispecific antibody according to claim 1, wherein
LCDR1 has the amino acid sequence set forth in SEQ ID NO: 12,
LCDR2 has the amino acid sequence set forth in SEQ ID NO: 13, and
LCDR3 has the amino acid sequence set forth in SEQ ID NO: 14;
or
LCDR1 has the amino acid sequence set forth in SEQ ID NO: 12,
LCDR2 has the amino acid sequence set forth in SEQ ID NO: 15, and
LCDR3 has the amino acid sequence set forth in SEQ ID NO: 16;
or
LCDR1 has the amino acid sequence set forth in SEQ ID NO: 17,
LCDR2 has the amino acid sequence set forth in SEQ ID NO: 15, and
LCDR3 has the amino acid sequence set forth in SEQ ID NO: 14;
**or**
LCDR1 has the amino acid sequence set forth in SEQ ID NO: 12,
LCDR2 has the amino acid sequence set forth in SEQ ID NO: 13, and
LCDR3 has the amino acid sequence set forth in SEQ ID NO: 16;
**or**
LCDR1 has the amino acid sequence set forth in SEQ ID NO: 12,
LCDR2 has the amino acid sequence set forth in SEQ ID NO: 15, and
LCDR3 has the amino acid sequence set forth in SEQ ID NO: 14;
or
LCDR1 has the amino acid sequence set forth in SEQ ID NO: 17,
LCDR2 has the amino acid sequence set forth in SEQ ID NO: 13, and
LCDR3 has the amino acid sequence set forth in SEQ ID NO: 14;
or
LCDR1 has the amino acid sequence set forth in SEQ ID NO: 17,
LCDR2 has the amino acid sequence set forth in SEQ ID NO: 13, and
LCDR3 has the amino acid sequence set forth in SEQ ID NO: 16;
or
LCDR1 has the amino acid sequence set forth in SEQ ID NO: 17,
LCDR2 has the amino acid sequence set forth in SEQ ID NO: 15, and
LCDR3 has the amino acid sequence set forth in SEQ ID NO: 16.

3. The bispecific antibody according to any one of claims 1 to 2, wherein
the heavy chain variable region of the anti-LAG3 antibody is selected from amino acid sequences set forth in SEQ ID NO: 2 and SEQ ID NO: 44, and the light chain variable region of the anti-LAG3 antibody is selected from amino acid sequences set forth in SEQ ID NO: 4, SEQ ID NO: 6, SEQ ID NO: 8, and SEQ ID NO: 45;
preferably,
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 4;
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 6;
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 8;
the heavy chain variable region of the anti-LAG3 antibody has the amino acid sequence set forth in SEQ ID NO: 2, and the light chain variable region of the anti-LAG3 antibody has the amino acid sequence set forth in SEQ ID NO: 45;
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 4;
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 6;
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 8; or
the heavy chain variable region of the anti-LAG3 antibody has the amino acid sequence set forth in SEQ ID NO: 44, and the light chain variable region of the anti-LAG3 antibody has the amino acid sequence set forth in SEQ ID NO: 45.

4. The bispecific antibody according to any one of claims 1 to 3, wherein the anti-LAG3 antibody or the antigen-binding fragment thereof is selected from Fab, Fab', F(ab')₂, Fd, Fv, dAb, a complementarity determining region fragment, a single chain antibody, a humanized antibody, and a chimeric antibody.

5. The bispecific antibody according to any one of claims 1 to 4, wherein
the anti-LAG3 antibody comprises a non-CDR region derived from a human antibody.

6. The bispecific antibody according to any one of claims 1 to 5, wherein
the second protein functional region is an anti-CD73 antibody or an antigen-binding fragment thereof, and the anti-CD73 antibody comprises a heavy chain variable region and a light chain variable region; the heavy chain variable region comprises HCDR1 having the amino acid sequence set forth in SEQ ID NO: 25, HCDR2 having the amino acid sequence set forth in SEQ ID NO: 26, and HCDR3 having the amino acid sequence set forth in SEQ ID NO: 27; and
the light chain variable region comprises LCDR1 having the amino acid sequence set forth in SEQ ID NO: 28, LCDR2 having the amino acid sequence set forth in SEQ ID NO: 29, and LCDR3 having the amino acid sequence set forth in SEQ ID NO: 30.

7. The bispecific antibody according to claim 6, wherein the heavy chain variable region of the anti-CD73 antibody is selected from amino acid sequences set forth in SEQ ID NO: 31, SEQ ID NO: 33, SEQ ID NO: 35, and SEQ ID NO: 46; the light chain variable region of the anti-CD73 antibody is selected from amino acid sequences set forth in SEQ ID NO: 32, SEQ ID NO: 34, SEQ ID NO: 36, SEQ ID NO: 37, SEQ ID NO: 47, and SEQ ID NO: 62;
preferably,
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 47;
the heavy chain variable region of the anti-CD73 antibody has the amino acid sequence set forth in SEQ ID NO: 31, and the light chain variable region of the anti-CD73 antibody has the amino acid sequence set forth in SEQ ID NO: 62;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 47;
the heavy chain variable region of the anti-CD73 antibody has the amino acid sequence set forth in SEQ ID NO: 33, and the light chain variable region of the anti-CD73 antibody has the amino acid sequence set forth in SEQ ID NO: 62;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 47;
the heavy chain variable region of the anti-CD73 antibody has the amino acid sequence set forth in SEQ ID NO: 35, and the light chain variable region of the anti-CD73 antibody has the amino acid sequence set forth in SEQ ID NO: 62;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 32;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 34;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 36;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 37;
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 47; or
the heavy chain variable region of the anti-CD73 antibody has the amino acid sequence set forth in SEQ ID NO: 46, and the light chain variable region of the anti-CD73 antibody has the amino acid sequence set forth in SEQ ID NO: 62.

8. The bispecific antibody according to any one of claims 1 to 7, wherein
the anti-LAG3 antibody or the anti-CD73 antibody further comprises a constant region derived from a human antibody;
preferably, the constant region of the anti-LAG3 antibody or the anti-CD73 antibody is selected from a constant region of human IgG1, IgG2, IgG3, or IgG4;
preferably, the anti-LAG3 antibody comprises a heavy chain constant region and a light chain constant region, wherein the heavy chain constant region is Ig gamma-1 chain C region (e.g., as set forth in SEQ ID NO: 18) or Ig gamma-4 chain C region (e.g., as set forth in SEQ ID NO: 20), and the light chain constant region is Ig kappa chain C region (e.g., as set forth in SEQ ID NO: 19);
preferably, the anti-CD73 antibody comprises a heavy chain constant region and a light chain constant region, wherein the heavy chain constant region is Ig gamma-1 chain C region (e.g., as set forth in SEQ ID NO: 18) or Ig gamma-4 chain C region (e.g., as set forth in SEQ ID NO: 20), and the light chain constant region is Ig kappa chain C region (e.g., as set forth in SEQ ID NO: 19).

9. The bispecific antibody according to any one of claims 1 to 8, wherein the bispecific antibody is **characterized by** any one of the following items (1) to (4):
(1)
the anti-LAG3 antibody is of human IgG1 subtype,
wherein according to the EU numbering system, the heavy chain constant region of the antibody has the following mutations:
L234A and L235A;
L234A and G237A;
L235A and G237A; or
L234A, L235A, and G237A;
(2)
the anti-LAG3 antibody is of human IgG4 subtype,
wherein according to the EU numbering system, the heavy chain constant region of the antibody has the following mutations:
F234A and L235A;
F234A and G237A;
L235A and G237A; or
F234A, L235A, and G237A;
(3)
the anti-CD73 antibody is of human IgG1 subtype,
wherein according to the EU numbering system, the heavy chain constant region of the antibody has the following mutations:
L234A and L235A;
L234A and G237A;
L235A and G237A; or
L234A, L235A, and G237A;
or
(4)
the anti-CD73 antibody is of human IgG4 subtype,
wherein according to the EU numbering system, the heavy chain constant region of the antibody has the following mutations:
F234A and L235A;
F234A and G237A;
L235A and G237A; or
F234A, L235A, and G237A.

10. The bispecific antibody according to any one of claims 1 to 9, wherein
the bispecific antibody is in an IgG-scFv form;
preferably, the first protein functional region is an immunoglobulin, and the second protein functional region is a single chain antibody; or
preferably, the first protein functional region is a single chain antibody, and the second protein functional region is an immunoglobulin targeting a target other than LAG3 (e.g., CD73 or PD-1).

11. The bispecific antibody according to any one of claims 1 to 10, comprising:
a first protein functional region targeting LAG3, and
a second protein functional region targeting CD73,
wherein
the first protein functional region is an anti-LAG3 antibody, the anti-LAG3 antibody is an immunoglobulin, and the second protein functional region is an anti-CD73 single chain antibody; or
the first protein functional region is an anti-LAG3 single chain antibody, the second protein functional region is an anti-CD73 antibody, and the anti-CD73 antibody is an immunoglobulin.

12. The bispecific antibody according to any one of claims 10 to 11, wherein
a heavy chain variable region of the immunoglobulin comprises HCDR1-HCDR3 having amino acid sequences set forth in SEQ ID NOs: 9-11, respectively, and a light chain variable region of the immunoglobulin comprises LCDR1-LCDR3 having amino acid sequences set forth in SEQ ID NOs: 12-14, respectively; a heavy chain variable region of the single chain antibody comprises HCDR1-HCDR3 having amino acid sequences set forth in SEQ ID NOs: 25-27, respectively, and a light chain variable region of the single chain antibody comprises LCDR1-LCDR3 having amino acid sequences set forth in SEQ ID NOs: 28-30 respectively; or
a heavy chain variable region of the single chain antibody comprises HCDR1-HCDR3 having amino acid sequences set forth in SEQ ID NOs: 9-11, respectively, and a light chain variable region of the single chain antibody comprises LCDR1-LCDR3 having amino acid sequences set forth in SEQ ID NOs: 12-14, respectively; a heavy chain variable region of the immunoglobulin comprises HCDR1-HCDR3 having amino acid sequences set forth in SEQ ID NOs: 25-27, respectively, and a light chain variable region of the immunoglobulin comprises LCDR1-LCDR3 having amino acid sequences set forth in SEQ ID NOs: 28-30, respectively.

13. The bispecific antibody according to any one of claims 10 to 12, wherein
the heavy chain variable region of the immunoglobulin is selected from amino acid sequences set forth in SEQ ID NO: 2 and SEQ ID NO: 44, and the light chain variable region of the immunoglobulin is selected from amino acid sequences set forth in SEQ ID NO: 4, SEQ ID NO: 6, SEQ ID NO: 8, and SEQ ID NO: 45; the heavy chain variable region of the single chain antibody is selected from amino acid sequences set forth in SEQ ID NO: 31, SEQ ID NO: 33, SEQ ID NO: 35, and SEQ ID NO: 46, and the light chain variable region of the single chain antibody is selected from amino acid sequences set forth in SEQ ID NO: 32, SEQ ID NO: 34, SEQ ID NO: 36, SEQ ID NO: 37, SEQ ID NO: 47, and SEQ ID NO: 62; **or**
the heavy chain variable region of the single chain antibody is selected from amino acid sequences set forth in SEQ ID NO: 2 and SEQ ID NO: 44, and the light chain variable region of the single chain antibody is selected from amino acid sequences set forth in SEQ ID NO: 4, SEQ ID NO: 6, SEQ ID NO: 8, and SEQ ID NO: 45; the heavy chain variable region of the immunoglobulin is selected from amino acid sequences set forth in SEQ ID NO: 31, SEQ ID NO: 33, SEQ ID NO: 35, and SEQ ID NO: 46, and the light chain variable region of the immunoglobulin is selected from amino acid sequences set forth in SEQ ID NO: 32, SEQ ID NO: 34, SEQ ID NO: 36, SEQ ID NO: 37, SEQ ID NO: 47, and SEQ ID NO: 62.

14. The bispecific antibody according to any one of claims 10 to 13, wherein the bispecific antibody is selected from any one of the following (1)-(24):
(1)
the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 31, and the light chain variable region of the single chain antibody has the amino acid sequence set forth in SEQ ID NO: 32;
(2)
the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 33, and the light chain variable region of the single chain antibody has the amino acid sequence set forth in SEQ ID NO: 34;
(3)
the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 35, and the light chain variable region of the single chain antibody has the amino acid sequence set forth in SEQ ID NO: 36;
(4)
the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 35, and the light chain variable region of the single chain antibody has the amino acid sequence set forth in SEQ ID NO: 37;
(5)
the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 46, and the light chain variable region of the single chain antibody has the amino acid sequence set forth in SEQ ID NO: 47;
(6)
the heavy chain variable region of the immunoglobulin has the amino acid sequence set forth in SEQ ID NO: 2, and the light chain variable region of the immunoglobulin has the amino acid sequence set forth in SEQ ID NO: 4; the heavy chain variable region of the single chain antibody has the amino acid sequence set forth in SEQ ID NO: 35, and the light chain variable region of the single chain antibody has the amino acid sequence set forth in SEQ ID NO: 62;
(7)
the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 31, and the light chain variable region of the single chain antibody has the amino acid sequence set forth in SEQ ID NO: 32;
(8)
the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 33, and the light chain variable region of the single chain antibody has the amino acid sequence set forth in SEQ ID NO: 34;
(9)
the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 35, and the light chain variable region of the single chain antibody has the amino acid sequence set forth in SEQ ID NO: 36;
(10)
the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 35, and the light chain variable region of the single chain antibody has the amino acid sequence set forth in SEQ ID NO: 37;
(11)
the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 46, and the light chain variable region of the single chain antibody has the amino acid sequence set forth in SEQ ID NO: 47;
(12)
the heavy chain variable region of the immunoglobulin has the amino acid sequence set forth in SEQ ID NO: 44, and the light chain variable region of the immunoglobulin has the amino acid sequence set forth in SEQ ID NO: 45; the heavy chain variable region of the single chain antibody has the amino acid sequence set forth in SEQ ID NO: 35, and the light chain variable region of the single chain antibody has the amino acid sequence set forth in SEQ ID NO: 62;
(13)
the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 31, and the light chain variable region of the immunoglobulin has the amino acid sequence set forth in SEQ ID NO: 32;
(14)
the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 33, and the light chain variable region of the immunoglobulin has the amino acid sequence set forth in SEQ ID NO: 34;
(15)
the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 35, and the light chain variable region of the immunoglobulin has the amino acid sequence set forth in SEQ ID NO: 36;
(16)
the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 35, and the light chain variable region of the immunoglobulin has the amino acid sequence set forth in SEQ ID NO: 37;
(17)
the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 46, and the light chain variable region of the immunoglobulin has the amino acid sequence set forth in SEQ ID NO: 47;
(18)
the heavy chain variable region of the single chain antibody has the amino acid sequence set forth in SEQ ID NO: 2, and the light chain variable region of the single chain antibody has the amino acid sequence set forth in SEQ ID NO: 4; the heavy chain variable region of the immunoglobulin has the amino acid sequence set forth in SEQ ID NO: 35, and the light chain variable region of the immunoglobulin has the amino acid sequence set forth in SEQ ID NO: 62;
(19)
the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 31, and the light chain variable region of the immunoglobulin has the amino acid sequence set forth in SEQ ID NO: 32;
(20)
the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 33, and the light chain variable region of the immunoglobulin has the amino acid sequence set forth in SEQ ID NO: 34;
(21)
the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 35, and the light chain variable region of the immunoglobulin has the amino acid sequence set forth in SEQ ID NO: 36;
(22)
the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 35, and the light chain variable region of the immunoglobulin has the amino acid sequence set forth in SEQ ID NO: 37;
(23)
the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 46, and the light chain variable region of the immunoglobulin has the amino acid sequence set forth in SEQ ID NO: 47; and
(24)
the heavy chain variable region of the single chain antibody has the amino acid sequence set forth in SEQ ID NO: 44, and the light chain variable region of the single chain antibody has the amino acid sequence set forth in SEQ ID NO: 45; the heavy chain variable region of the immunoglobulin has the amino acid sequence set forth in SEQ ID NO: 35, and the light chain variable region of the immunoglobulin has the amino acid sequence set forth in SEQ ID NO: 62.

15. The bispecific antibody according to any one of claims 1 to 14, wherein the first protein functional region and the second protein functional region are each independently 1, 2, or more in number;
preferably, the first protein functional region and the second protein functional region are linked directly or via a linker;
preferably, the heavy chain variable region and the light chain variable region of the anti-LAG3 single chain antibody are linked directly or via a linker;
preferably, the heavy chain variable region and the light chain variable region of the anti-CD73 single chain antibody are linked directly or via a linker;
preferably, the linker is independently the polypeptide set forth in SEQ ID NO: 48 or a polypeptide formed by concatenating a plurality of (e.g., 2, 3, 4, 5, or 6) polypeptides set forth in SEQ ID NO: 48;
preferably, the linker is independently a polypeptide having one or more glycines linked to the C-terminus of the polypeptide set forth in SEQ ID NO: 48 or a polypeptide formed by concatenating a plurality of (e.g., 2, 3, 4, 5, or 6) polypeptides set forth in SEQ ID NO: 48.

16. The bispecific antibody according to any one of claims 10 to 15, wherein each single chain antibody is linked to the C-terminus or the N-terminus of one of the two heavy chains of the immunoglobulin.

17. The bispecific antibody according to any one of claims 1 to 16, comprising:
a first protein functional region targeting LAG3, and
a second protein functional region targeting CD73,
the first protein functional region is 1 in number, and the second protein functional region is 2 in number;
wherein the first protein functional region is an immunoglobulin, and the second protein functional region is a single chain antibody;
a heavy chain of the immunoglobulin has the amino acid sequence set forth in SEQ ID NO: 38, and a light chain of the immunoglobulin has the amino acid sequence set forth in SEQ ID NO: 39;
a heavy chain variable region of the single chain antibody has the amino acid sequence set forth in SEQ ID NO: 35, and a light chain variable region of the single chain antibody has the amino acid sequence set forth in SEQ ID NO: 62; or a heavy chain variable region of the single chain antibody has the amino acid sequence set forth in SEQ ID NO: 46, and a light chain variable region of the single chain antibody has the amino acid sequence set forth in SEQ ID NO: 47;
each single chain antibody is linked to the C-terminus of one of the two heavy chains of the immunoglobulin;
the first protein functional region and the second protein functional region are linked via a first linker; the heavy chain variable region of the single chain antibody and the light chain variable region of the single chain antibody are linked via a second linker; the first linker and the second linker are identical or different;
preferably, the first linker and the second linker each have an amino acid sequence independently selected from SEQ ID NO: 42 and SEQ ID NO: 43;
preferably, the first linker and the second linker both have the amino acid sequence set forth in SEQ ID NO: 43;
preferably, according to the EU numbering system, the constant region of the heavy chain of the immunoglobulin comprises the following mutations:
L234A and L235A;
L234A and G237A;
L235A and G237A; or
L234A, L235A, and G237A.

18. The bispecific antibody according to any one of claims 1 to 16, comprising:
a first protein functional region targeting LAG3, and
a second protein functional region targeting CD73,
the first protein functional region is 2 in number, and the second protein functional region is 1 in number;
wherein the first protein functional region is a single chain antibody, and the second protein functional region is an immunoglobulin;
a heavy chain of the immunoglobulin has the amino acid sequence set forth in SEQ ID NO: 40, and a light chain of the immunoglobulin has the amino acid sequence set forth in SEQ ID NO: 41;
a heavy chain variable region of the single chain antibody has the amino acid sequence set forth in SEQ ID NO: 2, and a light chain variable region of the single chain antibody has the amino acid sequence set forth in SEQ ID NO: 4; or a heavy chain variable region of the single chain antibody has the amino acid sequence set forth in SEQ ID NO: 44, and a light chain variable region of the single chain antibody has the amino acid sequence set forth in SEQ ID NO: 45;
each single chain antibody is linked to the C-terminus of one of the two heavy chains of the immunoglobulin;
the first protein functional region and the second protein functional region are linked via a first linker; the heavy chain variable region of the single chain antibody and the light chain variable region of the single chain antibody are linked via a second linker; the first linker and the second linker are identical or different;
preferably, the first linker and the second linker each have an amino acid sequence independently selected from SEQ ID NO: 42 and SEQ ID NO: 43;
preferably, the first linker and the second linker both have the amino acid sequence set forth in SEQ ID NO: 43;
preferably, according to the EU numbering system, the constant region of the heavy chain of the immunoglobulin comprises the following mutations:
L234A and L235A;
L234A and G237A;
L235A and G237A; or
L234A, L235A, and G237A.

19. An isolated nucleic acid molecule, wherein the isolated nucleic acid molecule encodes the bispecific antibody according to any one of claims 1 to 18.

20. A recombinant vector, comprising the isolated nucleic acid molecule according to claim 19.

21. A host cell, comprising the isolated nucleic acid molecule according to claim 19 or the recombinant vector according to claim 20.

22. A pharmaceutical composition, comprising the bispecific antibody according to any one of claims 1 to 18, wherein optionally, the pharmaceutical composition further comprises a pharmaceutically acceptable auxiliary material.

23. Use of the bispecific antibody according to any one of claims 1 to 18 in the preparation of a medicament for treating or preventing a tumor, wherein
preferably, the tumor is selected from one or more of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

24. The bispecific antibody according to any one of claims 1 to 18 for use in treating or preventing a tumor, wherein
preferably, the tumor is selected from one or more of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

25. A method for treating or preventing a tumor, comprising a step of administering to a subject in need an effective amount of the bispecific antibody according to any one of claims 1 to 18, wherein
preferably, the tumor is selected from one or more of ovarian cancer, esophageal cancer, melanoma, hematological malignancy, glioblastoma, renal cell carcinoma, lung cancer, prostate cancer, bladder cancer, colon cancer, rectal cancer, liver cancer, gastric cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.
